# EUROPEAN PATENT SPECIFICATION

(11) **EP 4 179 111 B1**
(45) Date of publication and mention of the grant of the patent: **03.04.2024**
(21) Application number: 21751692.1
(22) Date of filing: 09.07.2021
(51) Int. Cl.: C12Q 1/6806

(54) **METHODS OF DETECTING GENOMIC REARRANGEMENTS USING CELL FREE NUCLEIC ACIDS**
VERFAHREN ZUM NACHWEIS VON GENOMISCHEN NEUANORDNUNGEN UNTER VERWENDUNG ZELLFREIER NUKLEINSÄUREN
PROCÉDÉS DE DÉTECTION DE RÉARRANGEMENTS GÉNOMIQUES À L'AIDE D'ACIDES NUCLÉIQUES ACELLULAIRES

(30) Priority: 10.07.2020 US 202063050373 P
(43) Date of publication of application: 17.05.2023
(73) Proprietor: Guardant Health, Inc., Palo Alto, CA 94304 (US)
(72) Inventor: HARTWIG, Anna Vilborg, Redwood City, California 94063 (US); BURKE, Jordan Emily Halsig, Redwood City, California 90463 (US)
(74) Representative: Carpmaels & Ransford LLP
(86) International application number: PCT/US2021/041148
(87) International publication number: WO 2023/282916

(56) References cited:
- WO-A1-2018/005983
- WO-A1-2020/121206
- WO-A2-2018/119452
- GAWRONSKI ALEXANDER R ET AL: "Structural variation and fusion detection using targeted sequencing data from circulating cell free DNA", NUCLEIC ACIDS RESEARCH, vol. 47, no. 7, 23 April 2019 (2019-04-23) , pages e38-e38, XP055802947, GB ISSN: 0305-1048, DOI: 10.1093/nar/gkz067 Retrieved from the Internet: URL:https://academic.oup.com/nar/article-p df/47/7/e38/28467885/gkz067.pdf>

## Description

### BACKGROUND

Cancer is responsible for millions of deaths per year worldwide. Selection of effective treatments may result in improved outcomes because many cancer subtypes respond significantly differently to different treatments.

Improperly controlled cell growth is a hallmark of cancer that generally results from an accumulation of genetic and epigenetic changes, such as copy number variations (CNVs), single nucleotide variations (SNVs), gene fusions, insertions and/or deletions (indels), epigenetic variations include 5-methylation of cytosine (5-methylcytosine) and association of DNA with chromatin proteins and transcription factors.

Biopsies represent a traditional approach for detecting or diagnosing cancer in which cells or tissue are extracted from a possible site of cancer and analyzed for relevant phenotypic and/or genotypic features. Biopsies have the drawback of being invasive.

Analysis of DNA from cancer cells, whether performed with samples of tissue or body fluids ("liquid biopsies"), such as blood, can be used to identify treatments to which a cancer is more likely to respond. For example, targeted treatments exist that are particularly effective against cancers with certain genomic rearrangements, such as gene fusions. The presence of certain genomic rearrangements such as gene fusions can also be indicative of the presence of cancer, and therefore may also be relevant to cancer detection. However, it has been challenging to develop rapid, accurate and sensitive methods for analyzing DNA (especially fragmented DNA as occurs in the case of cfDNA and certain types of tissue samples) for the presence of genomic rearrangements such as gene fusions, despite their known involvement in a number of cancers. In particular, for certain sample types including cell-free DNA and DNA from processed tissue samples (e.g., formalin-fixed paraffin-embedded samples), the DNA can be fragmented and/or present in low concentrations. Currently, it is difficult to call gene fusions using libraries generated from such samples, e.g., by a process comprising capture with oligonucleotide baits. Breakpoints may be located anywhere in long introns, which necessitates tiling the introns with probes, which can be challenging and cost prohibitive. Additionally, breakpoints can lead to mismatches between the probe sequence and one of the donor genes, reducing the number of nucleotides that can base-pair to the probe and limiting capture and/or retention of breakpoint-containing fragments, e.g., during post-enrichment washes.

Accordingly, there is a need for improved methods for analyzing DNA for genomic rearrangements, including gene fusions, for use in cancer treatment selection, detecting cancer, and other applications.

Gawronski, Alexander R et al. Nucleic Acids Research, vol. 47, no. 7, 23 April 2019, pages e38-e38 describes "Structural variation and fusion detection using targeted sequencing data from circulating cell free DNA".

### SUMMARY

The present disclosure provides methods of detecting the presence or absence of a genomic rearrangement. The invention is set out in the appended claims.

### BRIEF DESCRIPTION OF THE DRAWINGS

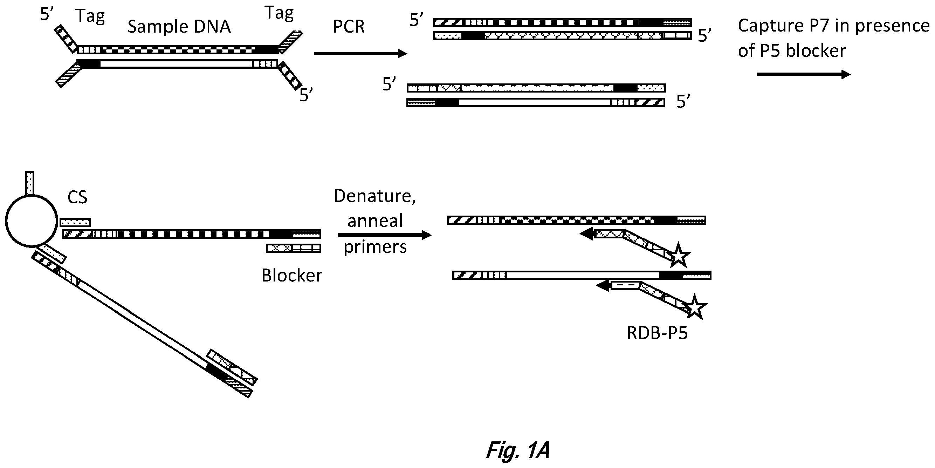

**FIG. 1A**: In an exemplary embodiment, tagged DNA molecules containing a first sequencing adapter (e.g., a P7 sequence) at the 5' end are captured (e.g., using a complementary sequence (CS) attached to a solid support such as a bead) in the presence of oligonucleotides (Blocker) that block a second sequencing adapter (e.g., a P5 sequence) to select a population where substantially all 5' ends have P7 adapters, and substantially all 3' ends have the complement of the second sequencing adapter. DNA of this population is contacted with primers specific for loci of interest that also comprise a rearrangement detection barcode and a sequencing adapter (RDB-P5), and a non-nucleotide binding partner, represented with a star (e.g., biotin) for linear amplification.

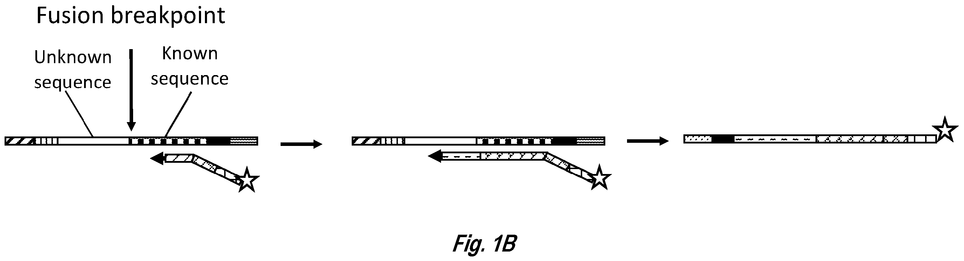

**FIG. 1B**: Linear amplification is performed on isolated tagged DNA. Molecules containing a fusion breakpoint, together with other molecules (not shown), are linearly amplified with primers as described in Fig. 1A, providing extension products comprising the non-nucleotide binding partner, the rearrangement detection barcode, and the fusion breakpoint, as well as sequencing adapters at both ends. The extension products can be captured, e.g., for washing and further processing, using binding to the non-nucleotide binding partner (e.g., binding to biotin with streptavidin-coated beads).

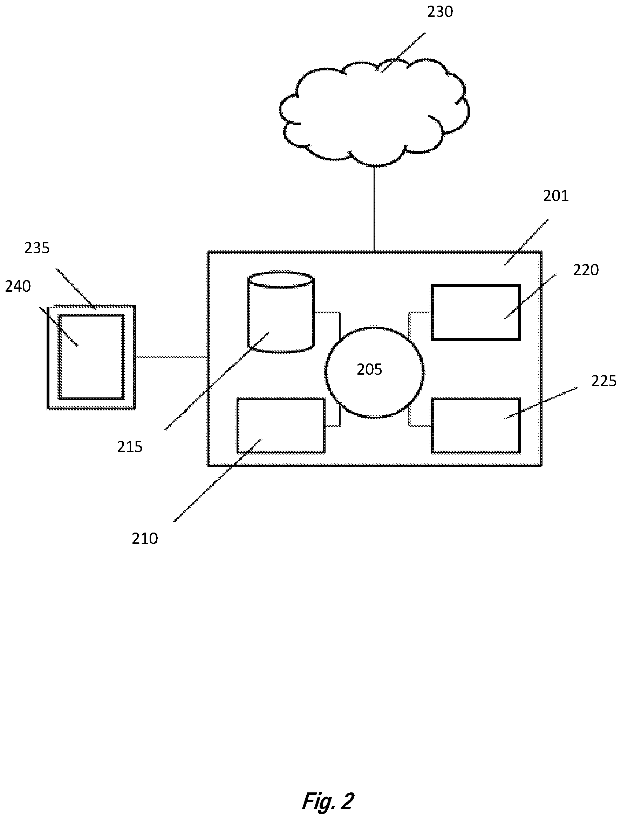

**FIG. 2**: a schematic diagram of an example of a system suitable for use with some embodiments of the disclosure.

### DETAILED DESCRIPTION

Reference will now be made in detail to certain embodiments of the invention. While the invention will be described in conjunction with such embodiments, it will be understood that they are not intended to limit the invention to those embodiments. On the contrary, the invention is intended to cover all alternatives, and modifications, which may be included within the invention as defined by the appended claims.

Before describing the present teachings in detail, it is to be understood that the disclosure is not limited to specific compositions or process steps, as such may vary. It should be noted that, as used in this specification and the appended claims, the singular form "a", "an" and "the" include plural references unless the context clearly dictates otherwise. Thus, for example, reference to "a nucleic acid" includes a plurality of nucleic acids, reference to "a cell" includes a plurality of cells, and the like.

Numeric ranges are inclusive of the numbers defining the range. Measured and measurable values are understood to be approximate, taking into account significant digits and the error associated with the measurement. Also, the use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the teachings.

Unless specifically noted in the above specification, embodiments in the specification that recite "comprising" various components are also contemplated as "consisting of' or "consisting essentially of" the recited components; embodiments in the specification that recite "consisting of" various components are also contemplated as "comprising" or "consisting essentially of" the recited components; and embodiments in the specification that recite "consisting essentially of" various components are also contemplated as "consisting of" or "comprising" the recited components (this interchangeability does not apply to the use of these terms in the claims).

The section headings used herein are for organizational purposes and are not to be construed as limiting the disclosed subject matter in any way.

### I. DEFINITIONS

"Cell-free DNA," "cfDNA molecules," or simply "cfDNA" include DNA molecules that occur in a subject in extracellular form (e.g., in blood, serum, plasma, or other bodily fluids such as lymph, cerebrospinal fluid, urine, or sputum) and includes DNA not contained within or otherwise bound to a cell. While the DNA originally existed in a cell or cells of a large complex biological organism (e.g., a mammal) or other cells, such as bacteria, colonizing the organism, the DNA has undergone release from the cell(s) into a fluid found in the organism. cfDNA includes, but is not limited to, cell-free genomic DNA of the subject (e.g., a human subject's genomic DNA) and cell-free DNA of microbes, such as bacteria, inhabiting the subject (whether pathogenic bacteria or bacteria normally found in commonly colonized locations such as the gut or skin of healthy controls), but does not include the cell-free DNA of microbes that have merely contaminated a sample of bodily fluid. Typically, cfDNA may be obtained by obtaining a sample of the fluid without the need to perform an in vitro cell lysis step and also includes removal of cells present in the fluid (e.g., centrifugation of blood to remove cells).

"Capturing" or "enriching" one or more target nucleic acids refers to preferentially isolating or separating the one or more target nucleic acids from non-target nucleic acids.

A "captured set" of nucleic acids refers to nucleic acids that have undergone capture.

A "target-region set" or "set of target regions" or "target regions" refers to a plurality of genomic loci or a plurality of genomic regions targeted for capture and/or targeted by a set of probes (e.g., through sequence complementarity).

"Corresponding to a target region set" means that a nucleic acid, such as cfDNA, originated from a locus in the target region set or specifically binds one or more probes for the target-region set.

"Specifically binds" in the context of an probe or other oligonucleotide and a target sequence means that under appropriate hybridization conditions, the oligonucleotide or probe hybridizes to its target sequence, or replicates thereof, to form a stable probe:target hybrid, while at the same time formation of stable probe: non-target hybrids is minimized. Thus, a probe hybridizes to a target sequence or replicate thereof to a sufficiently greater extent than to a non-target sequence, to enable capture or detection of the target sequence. Appropriate hybridization conditions are well-known in the art, may be predicted based on sequence composition, or can be determined by using routine testing methods (see, e.g., Sambrook et al., Molecular Cloning, A Laboratory Manual, 2nd ed. (Cold Spring Harbor Laboratory Press, Cold Spring Harbor, NY, 1989) at §§ 1.90-1.91, 7.37-7.57, 9.47-9.51 and 11.47-11.57, particularly §§ 9.50-9.51, 11.12-11.13, 11.45-11.47 and 11.55-11.57).

A circulating tumor DNA or ctDNA is a component of cfDNA that originated from a tumor cell or cancer cell. In some embodiments, cfDNA comprises DNA that originated from normal cells and DNA that originated from tumor cells (i.e., ctDNA). Tumor cells are neoplastic cells that originated from a tumor, regardless of whether they remain in the tumor or become separated from the tumor (as in the cases, e.g., of metastatic cancer cells and circulating tumor cells).

The term "hypermethylation" refers to an increased level or degree of methylation of nucleic acid molecule(s) relative to the other nucleic acid molecules within a population (e.g., sample) of nucleic acid molecules. In some embodiments, hypermethylated DNA can include DNA molecules comprising at least 1 methylated residue, at least 2 methylated residues, at least 3 methylated residues, at least 5 methylated residues, at least 10 methylated residues, at least 20 methylated residues, at least 25 methylated residues, or at least 30 methylated residues.

The term "hypomethylation" refers to a decreased level or degree of methylation of nucleic acid molecule(s) relative to the other nucleic acid molecules within a population (e.g., sample) of nucleic acid molecules. In some embodiments, hypomethylated DNA includes unmethylated DNA molecules. In some embodiments, hypomethylated DNA can include DNA molecules comprising 0 methylated residues, at most 1 methylated residue, at most 2 methylated residues, at most 3 methylated residues, at most 4 methylated residues, or at most 5 methylated residues.

The "capture yield" of a collection of probes for a given target region set refers to the amount (e.g., amount relative to another target region set or an absolute amount) of nucleic acid corresponding to the target region set that the collection captures under typical conditions. Exemplary typical capture conditions are an incubation of the sample nucleic acid and probes at 65°C for 10-18 hours in a small reaction volume (about 20 µL) containing stringent hybridization buffer. The capture yield may be expressed in absolute terms or, for a plurality of collections of probes, relative terms. When capture yields for a plurality of sets of target regions are compared, they are normalized for the footprint size of the target region set (e.g., on a per-kilobase basis). Thus, for example, if the footprint sizes of first and second target regions are 50 kb and 500 kb, respectively (giving a normalization factor of 0.1), then the DNA corresponding to the first target region set is captured with a higher yield than DNA corresponding to the second target region set when the mass per volume concentration of the captured DNA corresponding to the first target region set is more than 0.1 times the mass per volume concentration of the captured DNA corresponding to the second target region set. As a further example, using the same footprint sizes, if the captured DNA corresponding to the first target region set has a mass per volume concentration of 0.2 times the mass per volume concentration of the captured DNA corresponding to the second target region set, then the DNA corresponding to the first target region set was captured with a two-fold greater capture yield than the DNA corresponding to the second target region set.

"Genomic rearrangement" refers to a translocation, insertion, deletion, or other genomic change that results from the breaking and rejoining of strands of genomic DNA.

"Gene fusion" refers to a genomic rearrangement that results in a promoter and optionally part of the coding sequence of a first gene being joined to at least part of the coding sequence of a second gene.

"Oncogenic genomic rearrangement" refers to a genomic rearrangement that contributes to malignant transformation, cell cycle dysregulation, cancer progression, metastasis, or the like.

"Sequence-variable target region set" refers to a set of target regions that may exhibit changes in sequence such as nucleotide substitutions, insertions, deletions, or gene fusions or transpositions in neoplastic cells (e.g., tumor cells and cancer cells).

"Epigenetic target region set" refers to a set of target regions that may manifest non-sequence modifications in neoplastic cells (e.g., tumor cells and cancer cells) and non-tumor cells (e.g., immune cells, cells from tumor microenvironment). These modifications do not change the sequence of the DNA. Examples of non-sequence modifications changes include, but not limited to, changes in methylation (increases or decreases), nucleosome distribution, CTCF binding, transcription start sites, regulatory protein binding regions and any other proteins that may bind to the DNA. For present purposes, loci susceptible to neoplasia-, tumor-, or cancer-associated focal amplifications and/or gene fusions may also be included in an epigenetic target region set because detection of a change in copy number by sequencing or a fused sequence that maps to more than one locus in a reference genome tends to be more similar to detection of exemplary epigenetic changes discussed above than detection of nucleotide substitutions, insertions, or deletions, e.g., in that the focal amplifications and/or gene fusions can be detected at a relatively shallow depth of sequencing because their detection does not depend on the accuracy of base calls at one or a few individual positions. For example, the epigenetic target region set can comprise a set of target regions for analyzing the fragment length or fragment end point location distribution. The terms "epigenetic" and "epigenomic" are used interchangeably herein.

The terms "or a combination thereof' and "or combinations thereof' as used herein refers to any and all permutations and combinations of the listed terms preceding the term. For example, "A, B, C, or combinations thereof' is intended to include at least one of: A, B, C, AB, AC, BC, or ABC, and if order is important in a particular context, also BA, CA, CB, ACB, CBA, BCA, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

"Or" is used in the inclusive sense, i.e., equivalent to "and/or," unless the context requires otherwise.

### II. EXEMPLARY METHODS

### 1. Overview

Disclosed herein are methods and compositions for use in methods detecting the presence or absence of a genomic rearrangement. In some embodiments, the methods comprise dividing a sample of tagged DNA molecules prepared (e.g., amplified) from a sample from a subject into at least first and second aliquots. Performing an amplification step before dividing a sample into aliquots can make it more likely that copies of unique molecules will occur in each of the first and second aliquots. In some embodiments, the method comprises isolating a plurality of tagged DNA molecules from the first aliquot, wherein the plurality of molecules have a common adapter sequence (e.g., a P7 adapter). In some embodiments, the method further comprises linearly amplifying at least a portion of the tagged DNA molecules isolated from the first aliquot with a set of primers that target a first plurality of loci of interest and comprise a rearrangement detection barcode and a sequencing adapter (e.g., a P5 adapter) and a non-nucleotide binding partner (e.g., biotin), thereby producing a first population of processed DNA. Linear amplification requires only one primer and can be performed independently of knowledge of certain details of the rearrangement, such as what sequence has become proximal to a first locus of interest. It also permits generating products that are uniformly labeled with the non-nucleotide binding partner (e.g., biotin). In some embodiments, the rearrangement detection barcode marks the primer as a fusion detection primer. In some embodiments, the method further comprises capturing the first population of processed DNA on a solid support using binding to the non-nucleotide binding partner (e.g., biotin). In some embodiments, the method further comprises amplifying and/or eluting the first population of processed DNA. In some embodiments, the method further comprises enriching the second aliquot for a second plurality of loci of interest, thereby producing a second population of processed DNA. The enrichment of the DNA in the second aliquot may be any enrichment suitable for library preparation. In some embodiments, the method further comprises sequencing at least a portion of the amplified and/or eluted first population of processed DNA and at least a portion of the second population of processed DNA. In some embodiments, the method further comprises detecting the presence or absence of a plurality of genomic rearrangements in the first population of processed DNA. In some embodiments, the tagged DNA comprises tagged cell-free DNA.

In some embodiments, the tagged DNA of the method comprises molecules having a size in the range of 75-100 nucleotides, 100-200 nucleotides, 200-300 nucleotides, 300-400 nucleotides, 400-500 nucleotides, 500-600 nucleotides, or 600-700 nucleotides. In some embodiments, the tagged DNA consists of molecules having a mean size in the range of 100-200 nucleotides, 200-300 nucleotides, 300-400 nucleotides, 400-500 nucleotides, 500-600 nucleotides, or 600-700 nucleotides, wherein "mean size" refers to the average number of nucleotides in each of the tagged DNA molecules.

In some embodiments of the invention, the sample of tagged DNA is a partition of the sample from the subject. In some embodiments, the sample of tagged DNA comprises a hypomethylated partition of the sample from the subject. In some embodiments, the sample of tagged DNA comprises a hypermethylated partition of the sample from the subject. In some embodiments, the sample of tagged DNA comprises at least two partitions of the sample from the subject that have been recombined. In some embodiments, the at least two partitions of the sample from the subject that have been recombined comprise a hypomethylated partition and a hypermethylated partition. In some embodiments, the at least two partitions of the sample from the subject that have been recombined comprise a hypomethylated partition and an intermediately methylated partition (also referred to simply as an intermediate partition). In some embodiments, the at least two partitions of the sample from the subject that have been recombined comprise an intermediately methylated partition and a hypermethylated partition.

In some embodiments, the sample of tagged DNA comprises at least three partitions of the sample from the subject that have been recombined. In some embodiments, the at least three partitions of the sample from the subject that have been recombined comprise a hypomethylated partition, a hypermethylated partition, and an intermediately methylated partition.

In some embodiments, isolating a plurality of tagged DNA molecules from the first aliquot comprises blocking molecules comprising the complement of the common adapter sequence. This blocking step refers to isolating tagged DNA molecules from the first aliquot in a strand-specific manner. For example, if P7 and P5 sequences are used, molecules comprising P7 at the 5' end and the complement of P5 at the 3' end are isolated, but not molecules comprising P5 at the 5' end and the complement of P7 at the 3' end (or vice versa). In some embodiments, this blocking step occurs by capturing down the desired subset of tagged DNA molecules by contacting the tagged molecules with biotinylated complementary sequences of the desired tagged DNA molecules and non-biotinylated complementary sequences of the undesired tagged DNA molecules, and pulling down the desired tagged DNA molecules with streptavidin coated beads. Such a blocking step may be used to improve specificity, e.g., by limiting nonspecific binding interactions and/or avoiding daisy-chaining of products and unwanted molecules.

In some embodiments, the primers that target the first plurality of loci of interest comprise a cleavable linker between the sequencing adapter and the non-nucleotide binding partner. In some embodiments, the cleavable linker comprises at least one ribonucleotide, deoxyuridine, or abasic site. In some embodiments, the method of detecting the presence or absence of a genomic rearrangement further comprises washing the solid support after capturing the first population of processed DNA on the solid support and/or before amplifying and/or eluting the first population of processed DNA. In some embodiments, the first population is combined with DNA of the second aliquot to which labeled capture probes have been hybridized before or at the same time as contacting the first population and the DNA of the second aliquot with the solid support, to facilitate parallel enrichment and washing of the first population and the second aliquot.

Embodiments of the invention may employ one or more reagents for enriching for nucleic acid sequences of interest (e.g., with respect to the second aliquot). Such reagents for enriching are typically polynucleotides (or polynucleotide analogs, such as LNAs, PNAs, phosphorothioates, and the like). Reagents for enriching typically work through sequence specific hybridization. Some reagents for enriching for nucleic acid sequences of interest are commercially available, e.g., SureSelect ^{™} (Agilent Technologies) or from Twist Bioscience. Reagents useful in such capture procedures, such as bait sets, are described in detail elsewhere herein.

A genomic rearrangement detected by methods disclosed herein may be a translocation, insertion, deletion, or other genomic change that results from the breaking and rejoining of DNA strands. In some embodiments, a genomic rearrangements is a gene fusion, a type of genomic rearrangement that results in a promoter and optionally part of the coding sequence of a first gene being joined to at least part of the coding sequence of a second gene. Genomic rearrangements of the methods disclosed also includes oncogenic genomic rearrangements, which contribute to malignant transformation, cell cycle dysregulation, cancer progression, metastasis, or the like.

Genomic rearrangements of the methods disclosed herein may include one or more interchromosomal gene fusions, which are gene fusions involving portions of (at least) two genes originating from different chromosomes. Genomic rearrangements of the methods disclosed herein may also or alternatively include one or more intrachromosomal gene fusions, which are gene fusions involving portions of (at least) two genes originating from the same chromosome.

This skilled in the art are familiar with many genes that may undergo genomic rearrangements, e.g., in connection with the development and/or progression of proliferative diseases, including cancer. In some embodiments of the methods disclosed herein, genes that are involved in or affected by genomic arrangements include one or more of ALK, EML4, BCR, ABL1, MYC, an immunoglobulin gene, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ, or TFE3.

As discussed above, the methods disclosed herein comprise isolating a plurality of tagged DNA molecules from a first aliquot, wherein the plurality of molecules have a common adapter sequence. In some embodiments, the common adapter sequence comprises a sequencing primer sequence or complement thereof.

In some embodiments, the methods disclosed herein comprise linearly amplifying at least a portion of the tagged DNA molecules isolated from a first aliquot with a set of primers that target a first plurality of loci of interest and comprise a rearrangement detection barcode and a sequencing adapter (e.g., a P5 or P7 adapter) and a non-nucleotide binding partner (e.g., biotin). Linear amplification is distinguished from standard PCR amplification in that priming occurs in one direction, rather than in both forward and reverse directions. As a result, one strand of the target DNA is amplified, and the amount of product increases approximately linearly or arithmetically, as opposed to exponentially, with the number of cycles. Because linear amplification only requires one primer, it linearly amplifies any gene fusion involving a complementary sequence to at least one primer from a target gene and does not require advance knowledge of what sequence (e.g., from a fusion partner) is downstream of the primer binding site. In some embodiments, the linear amplifying comprises at least 2, 3, 4, 5, 6, 7, 8, 9, or 10 linear amplification cycles. In some embodiments, one linear amplification cycle is performed.

In some embodiments, the set of primers that target the first plurality of loci of interest tile or comprises primers that tile the loci of interest or at least a portion thereof. In some embodiments, the set of primers tile the loci of interest or a portion thereof at a density of at least 1, 2, 3, 4, 5, 6, 7, 8, 9, or 10 primers per 1000 nt. In some embodiments, the set of primers tile the loci of interest or a portion thereof at a density of at least 1, 2, 3, 4, or 5 primers per 100 nt. The more kb of the loci of interest that are tiled, the more likely the method is to capture and effectively detect the presence or absence of a genomic rearrangement. In some embodiments, at least about 100 nucleotides of a locus of interest are tiled to detect one genomic rearrangement. In some embodiments, the set of primers tile at least 500 nucleotides, 1 kb, or 2 kb of the loci of interest. In some embodiments, the set of primers tile at least 5, 10, 20, or 50 kb of the loci of interest.

In some embodiments, the sample of tagged DNA molecules is prepared by ligating tags to DNA molecules. In some embodiments, the tagged DNA molecules are non-uniquely tagged. In some embodiments, the tagged DNA molecules are amplified tagged DNA molecules. In some embodiments, at least a portion of the tags of the tagged DNA molecules comprise an indicator of epigenetic status. In some embodiments, the epigenetic status is a level of methylation. In some embodiments, the rearrangement detection barcode comprises at least 8, 9, 10, 11, or 12 nucleotides.

Variability in the solid support and the non-nucleotide binding partner of the primers targeting the first plurality of loci of interest is permitted as appropriate. In some embodiments, the solid support is a plurality of beads. In some embodiments, the non-nucleotide binding partner comprises a biotin moiety and the solid support comprises a biotin-binding agent. In some embodiments, the biotin-binding agent comprises an avidin, streptavidin, or anti-biotin antibody. In some other embodiments, the non-nucleotide binding partner comprises a digoxygenin or maltose moiety. In some embodiments, the solid support comprises an anti-digoxygenin antibody or maltose-binding protein.

In some embodiments, the tagged DNA molecules are prepared by a process comprising ligating tags to DNA from the subject. In some embodiments, the tagged DNA molecules are amplified before dividing the sample into first and second aliquots. In some embodiments, the tagged DNA molecules are prepared by a process comprising amplifying DNA from the subject with primers comprising tags.

In some embodiments, the subject is a human. In some embodiments, the subject has or is suspected of having a cancer. In some embodiments, the method of detecting the presence or absence of a genomic rearrangement disclosed herein further comprises detecting the presence or absence of cancer in the subject. In some embodiments, the method further comprises determining the likelihood that a subject has a cancer. In some embodiments, the cancer is or comprises colorectal cancer. In some embodiments, the cancer is or comprises one or more of lung, pancreatic, gastric, prostate, or liver cancer, or a lymphoma or leukemia.

In some embodiments, the first population of processed DNA and the second population of processed DNA are pooled before sequencing. In some embodiments, the pooling occurs after capturing the first population of processed DNA on a solid support. In some embodiments, the pooling occurs during or after enriching the second aliquot for a second plurality of loci of interest. In some embodiments, the first population of processed DNA and the second population of processed DNA are sequenced in the same sequencing cell. Alternatively, in some embodiments, the first population of processed DNA and the second population of processed DNA are sequenced without being pooled.

In some embodiments, enriching the second aliquot for a second plurality of loci of interest comprises capturing DNA obtained from a test subject comprising a plurality of sets of target regions. In some embodiments, the target regions comprise regions that can be used to obtain genetic information. The target regions may comprise epigenetic target regions, which may show differences in methylation levels and/or fragmentation patterns depending on whether they originated from a tumor or from healthy cells. In some embodiments, the epigenetic target region set comprises a hypermethylation variable target region set. In some embodiments, the epigenetic target region set comprises a fragmentation variable target region set. In some embodiments, the fragmentation variable target region set comprises at least one of transcription start site regions or CTCF binding regions. In some embodiments, the target regions comprise sequence-variable target regions, which may show differences in sequence depending on whether they originated from a tumor or from healthy cells. In some embodiments, the method further comprises determining whether DNA molecules corresponding to the sequence-variable target region set comprise cancer-associated mutations. In some embodiments, DNA molecules corresponding to the sequence-variable target region set are captured at a greater capture yield in the captured set of DNA molecules than other DNA molecules, such as DNA molecules corresponding to the epigenetic target region set.

### 2. Exemplary Loci of Interest

In some embodiments, the first plurality of loci of interest comprises a plurality of regions known to undergo genomic rearrangements in cancer. In some embodiments, these genomic rearrangements are oncogenic gene fusions, or genetic events that cause the formation of fusion genes, whereby two previously separate genes are rearranged to form a mutated hybrid gene associated with cancer.

In some embodiments, the first plurality of loci of interest that are linearly amplified in the first aliquot of DNA comprises one or more of ALK, EML4, BCR, ABL1, MYC, an immunoglobulin gene, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ, or TFE3. The tendency of these and other genes to be involved in oncogenic gene fusions has been described in the literature. Parker and Zhang, Chin J Cancer, 32(11): 594-603 (2013).

The following are examples of gene fusions and cancers involving these genes. This set of examples should be considered non-exhaustive and non-limiting. In some non-small cell lung cancers (NSCLC), the anaplastic lymphoma receptor tyrosine kinase (ALK) gene is fused to echinoderm microtubule associated protein-like 4 (EML4) gene. In some instances of chronic myelogenous leukemia, the breakpoint cluster region (BCR) gene is fused to the second exon of the Abelson murine leukemia viral oncogene homolog 1 (ABL1) gene. In some instances of Burkitt lymphoma, immunoglobulin genes are fused to MYC. In some instances of Ewing sarcoma, Ewing sarcoma breakpoint region 1 (EWSR1) gene is fused to members of the E-twenty six (ETS) gene family of transcription factor gene, such as friend leukemia virus integration 1 (FLI1). In some instances of synovial sarcoma, one of the three synovial sarcoma X (SSX) genes (SSX1, SSX2, and SSX4) is fused to the transcriptional coactivator SS18 gene. In some instances of promyelocytic leukemia, the promyelocytic leukemia (PML) gene is fused to the retinoic acid receptor alpha (RARA) gene. Ins some instances of clear-cell sarcoma, the EWSR1 gene is fused to the activating transcription factor 1 (ATF1) gene. In some instances of secretory breast carcinoma, ETS-variant transcription factor 6 (ETV6) gene is fused to the neurotrophic tyrosine receptor kinase type 4 (NTRK3) gene. In some instances of follicular thyroid carcinoma, the paired box 8 (PAX8) gene is fused to the peroxisome proliferator-activated receptor gamma (PPARG) gene. In some instances of mucoepidermoid carcinoma the mucoepidermoid carcinoma translocated 1 (MECT1) gene is fused to notch coactivator mastermind-like protein 2 (MAMI,2) gene. In some instances of prostate cancer, the transmembrane protease, serine 2 (TMPRSS2) gene is fused to ETS-related gene (ERG), ETS variant 1 (ETV1) gene, and ETV4 gene. In some instances of pilocytic astrocytoma, a type of benign brain tumor, the KIAA1549 gene is fused to the BRAF gene. In some instances of adenoid cystic carcinoma, the myb proto-oncogene (MYB) is fused to the nuclear factor 1 B-type (NFIB) gene. In some instances of ovarian cancer, the estrogen receptor-related alpha (ESRRA) gene is fused to the C11orf20 gene. In some instances of lung squamous cell carcinoma, the fibroblast growth factor receptor 3 (FGFR3) gene is fused to the transforming acidic coiled-coil-containing protein 3 (TACC3) gene. In some instances of colorectal cancer, protein tyrosine phosphatase receptor type K (PTPRK) gene is fused to the R-spondin-family protein 3 (RSPO3) gene. In some instances of colorectal cancer, the eukaryotic translation initiation factor 3, subunit E (EIF3E) gene is fused to R-spondin-family protein 2 (RSPO2) gene. In some instances of clear cell renal cell carcinoma (RCC), the splicing factor, proline- and glutamine-rich (SFPQ) gene is fused to transcription factor E3 (TFE3) gene.

### 3. Exemplary method for molecular tag identification of MBD-bead partitioned libraries

An exemplary method for molecular tag identification of MBD-bead partitioned libraries is as follows:
i) Physical partitioning of an extracted DNA sample (e.g., extracted cfDNA from a human sample, which has optionally been subjected to target capture as described herein) using a methyl-binding domain protein-bead purification kit, saving all elutions from process for downstream processing.
ii) Parallel application of differential molecular tags and NGS-enabling adapter sequences to each partition. For example, the hypermethylated, residual methylation ('wash'), and hypomethylated partitions are ligated with NGS- adapters with molecular tags.
iii) Re-combining all molecular tagged partitions, and subsequent amplification using adapter-specific DNA primer sequences.
iv) Division of sample (library) into first and second aliquots.
v) Processing of first aliquot for rearrangement detection, including isolating a plurality of tagged DNA molecules from the first aliquot, wherein the plurality of molecules have a common adapter sequence; linearly amplifying at least a portion of the tagged DNA molecules isolated from the first aliquot with a set of primers that target a first plurality of loci of interest and comprise a rearrangement detection barcode, a sequencing adapter, and a non-nucleotide binding partner , thereby producing a first population of processed DNA; capturing the first population of processed DNA on a solid support using binding to the non-nucleotide binding partner; and amplifying and/or eluting the first population of processed DNA.
vi) Capture/hybridization of second aliquot, targeting genomic regions of interest (e.g., cancer-specific genetic variants and differentially methylated regions), providing a second population of processed DNA.
vii) Re-combining the first and second populations of processed DNA and further amplification, appending a sample tag. Different samples may be pooled and assayed in multiplex on an NGS instrument.
viii) Bioinformatics analysis of NGS data, with the molecular tags being used to identify unique molecules, as well deconvolution of the sample into molecules that were differentially MBD-partitioned or that contain a rearrangement detection barcode. This analysis can yield information on gene fusion events and relative 5-methylcytosine for genomic regions, concurrent with standard genetic sequencing/variant detection.

The exemplary method set forth above may further comprise any compatible feature of methods according to this disclosure set forth elsewhere herein.

### 4. Cancer biomarkers

In some embodiments, a method described herein comprises detecting the presence or absence of one or more cancer biomarkers in a sample from the subject, optionally wherein the sample is a blood sample. For example, such detection can be performed by contacting the sample with one or more affinity agents, such as antibodies, specific for the one or more cancer biomarkers. Detection of such biomarkers in combination with detection/analysis one or both of sequence-variable and epigenetic target regions as described elsewhere herein can provide additional information, e.g., to further improve the specificity and/or sensitivity of cancer detection.

One skilled in the art is familiar with various biomarkers and corresponding antibodies appropriate for use in such methods. For example, US 2012/0040861 A1 and WO 2016/195051 A1 describe pancreatic cancer biomarkers and detection thereof; EP 2620772 A1 describes gastric cancer biomarkers and detection thereof; US 9,995,748 B2 and US 7,981,625 describe prostate cancer biomarkers and detection thereof; WO 2016/003479 A1 describes liver cancer biomarkers and detection thereof; and US 7,883,842, WO 2012/066451 A1, and WO 2009/074276 A2 describe colorectal cancer biomarkers and detection thereof.

In some embodiments, the one or more cancer biomarkers detected in the methods include one or more colorectal, pancreatic, gastric, prostate, or liver cancer biomarkers. In some embodiments, the one or more cancer biomarkers detected in the methods include one or more colorectal cancer biomarkers.

### III. GENERAL FEATURES OF THE METHODS

### 1. Samples

A sample can be any biological sample isolated from a subject. A sample can be a bodily sample. Samples can include body tissues, such as known or suspected solid tumors, whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, including gingival crevicular fluid, bone marrow, pleural effusions, cerebrospinal fluid, saliva, mucous, sputum, semen, sweat, urine. Samples are preferably body fluids, particularly blood and fractions thereof, and urine. A sample can be in the form originally isolated from a subject or can have been subjected to further processing to remove or add components, such as cells, or enrich for one component relative to another. Thus, a preferred body fluid for analysis is plasma or serum containing cell-free nucleic acids. A sample can be isolated or obtained from a subject and transported to a site of sample analysis. The sample may be preserved and shipped at a desirable temperature, e.g., room temperature, 4°C, -20°C, and/or -80°C. A sample can be isolated or obtained from a subject at the site of the sample analysis. The subject can be a human, a mammal, an animal, a companion animal, a service animal, or a pet. The subject may have a cancer. The subject may not have cancer or a detectable cancer symptom. The subject may have been treated with one or more cancer therapy, e.g., any one or more of chemotherapies, antibodies, vaccines or biologies. The subject may be in remission. The subject may or may not be diagnosed of being susceptible to cancer or any cancer-associated genetic mutations/disorders.

The volume of plasma can depend on the desired read depth for sequenced regions. Exemplary volumes are 0.4-40 ml, 5-20 ml, 10-20 ml. For examples, the volume can be 0.5 mL, 1 mL, 5 mL 10 mL, 20 mL, 30 mL, or 40 mL. A volume of sampled plasma may be 5 to 20 mL.

A sample can comprise various amount of nucleic acid that contains genome equivalents. For example, a sample of about 30 ng DNA can contain about 10,000 (10⁴) haploid human genome equivalents and, in the case of cfDNA, about 200 billion (2×10¹¹) individual polynucleotide molecules. Similarly, a sample of about 100 ng of DNA can contain about 30,000 haploid human genome equivalents and, in the case of cfDNA, about 600 billion individual molecules.

A sample can comprise nucleic acids from different sources, e.g., from cells and cell-free of the same subject, from cells and cell-free of different subjects. A sample can comprise nucleic acids carrying mutations. For example, a sample can comprise DNA carrying germline mutations and/or somatic mutations. Germline mutations refer to mutations existing in germline DNA of a subject. Somatic mutations refer to mutations originating in somatic cells of a subject, e.g., cancer cells. A sample can comprise DNA carrying cancer-associated mutations (e.g., cancer-associated somatic mutations). A sample can comprise an epigenetic variant (i.e. a chemical or protein modification), wherein the epigenetic variant associated with the presence of a genetic variant such as a cancer-associated mutation. In some embodiments, the sample comprises an epigenetic variant associated with the presence of a genetic variant, wherein the sample does not comprise the genetic variant.

Exemplary amounts of cell-free nucleic acids in a sample before amplification range from about 1 fg to about 1 µg, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, 200 ng, 250 ng or 300 ng of cell-free nucleic acid molecules. The method can comprise obtaining 1 femtogram (fg) to 200 ng.

Cell-free nucleic acids are nucleic acids not contained within or otherwise bound to a cell or in other words nucleic acids remaining in a sample after removing intact cells. Cell- free nucleic acids include DNA, RNA, and hybrids thereof, including genomic DNA, mitochondrial DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or a hybrid thereof. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA, (ctDNA). Others are released from healthy cells. In some embodiments, cfDNA is cell-free fetal DNA (cffDNA) In some embodiments, cell free nucleic acids are produced by tumor cells. In some embodiments, cell free nucleic acids are produced by a mixture of tumor cells and non-tumor cells.

Cell-free nucleic acids have an exemplary size distribution of about 100-500 nucleotides, with molecules of 110 to about 230 nucleotides representing about 90% of molecules, with a mode of about 168 nucleotides and a second minor peak in a range between 240 to 440 nucleotides.

Cell-free nucleic acids can be isolated from bodily fluids through a fractionation or partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, nucleic acids can be precipitated with an alcohol. Further clean up steps may be used such as silica-based columns to remove contaminants or salts. Non-specific bulk carrier nucleic acids, such as C 1 DNA, DNA or protein for bisulfite sequencing, hybridization, and/or ligation, may be added throughout the reaction to optimize certain aspects of the procedure such as yield.

After such processing, samples can include various forms of nucleic acid including double stranded DNA, single stranded DNA and single stranded RNA. In some embodiments, single stranded DNA and RNA can be converted to double stranded forms so they are included in subsequent processing and analysis steps.

Double-stranded DNA molecules in a sample and single stranded nucleic acid molecules converted to double stranded DNA molecules can be linked to adapters at either one end or both ends. Typically, double stranded molecules are blunt ended by treatment with a polymerase with a 5'-3' polymerase and a 3 '-5' exonuclease (or proof-reading function), in the presence of all four standard nucleotides. Klenow large fragment and T4 polymerase are examples of suitable polymerase. The blunt ended DNA molecules can be ligated with at least partially double stranded adapter (e.g., a Y shaped or bell-shaped adapter). Alternatively, complementary nucleotides can be added to blunt ends of sample nucleic acids and adapters to facilitate ligation. Contemplated herein are both blunt end ligation and sticky end ligation. In blunt end ligation, both the nucleic acid molecules and the adapter tags have blunt ends. In sticky-end ligation, typically, the nucleic acid molecules bear an "A" overhang and the adapters bear a "T" overhang.

### 2. Subjects

In some embodiments, the nucleic acid (e.g., DNA, such as cfDNA or DNA from a tumor tissue sample, such as a formalin-fixed paraffin-embedded (FFPE), fresh frozen, fine needle aspirate (FNA), or core needle biopsy (CNB) sample) is obtained from a subject having a cancer. In some embodiments, the subject is in need of a treatment for cancer, e.g., a new treatment not received previously and/or a different treatment from what the subject has received previously. In some embodiments, the subject or the subject's health care provider has requested, recommended, or determined that a new or different treatment be administered, and/or that a previously received treatment is ineffective or not sufficiently effective. In some embodiments, the nucleic acid (e.g., DNA, such as cfDNA or DNA from a tumor tissue sample, such as an FFPE, fresh frozen, FNA, or CNB sample) is obtained from a subject suspected of having a cancer. In some embodiments, the nucleic acid (e.g., DNA, such as cfDNA or DNA from a tumor tissue sample, such as an FFPE, fresh frozen, FNA, or CNB sample) is obtained from a subject having a tumor. In some embodiments, the nucleic acid (e.g., DNA, such as cfDNA or DNA from a tumor tissue sample, such as an FFPE, fresh frozen, FNA, or CNB sample) is obtained from a subject suspected of having a tumor. In some embodiments, the nucleic acid (e.g., DNA, such as cfDNA or DNA from a tumor tissue sample, such as an FFPE, fresh frozen, FNA, or CNB sample) is obtained from a subject having neoplasia. In some embodiments, the nucleic acid (e.g., DNA, such as cfDNA or DNA from a tumor tissue sample, such as an FFPE, fresh frozen, FNA, or CNB sample) is obtained from a subject suspected of having neoplasia. In some embodiments, the nucleic acid (e.g., DNA, such as cfDNA or DNA from a tumor tissue sample, such as an FFPE, fresh frozen, FNA, or CNB sample) is obtained from a subject in remission from a tumor, cancer, or neoplasia (e.g., following chemotherapy, surgical resection, radiation, or a combination thereof). In any of the foregoing embodiments, the cancer, tumor, or neoplasia or suspected cancer, tumor, or neoplasia may be of the lung, colon, rectum, kidney, breast, prostate, or liver. In some embodiments, the cancer, tumor, or neoplasia or suspected cancer, tumor, or neoplasia is of the lung. In some embodiments, the cancer, tumor, or neoplasia or suspected cancer, tumor, or neoplasia is of the colon or rectum. In some embodiments, the cancer, tumor, or neoplasia or suspected cancer, tumor, or neoplasia is of the breast. In some embodiments, the cancer, tumor, or neoplasia or suspected cancer, tumor, or neoplasia is of the prostate. In any of the foregoing embodiments, the subject may be a human subject.

In some embodiments, the subject was previously diagnosed with a cancer, e.g., any of the cancers noted above or elsewhere herein. Such a subject may have previously received one or more previous cancer treatments, e.g., surgery, chemotherapy, radiation, and/or immunotherapy. In some embodiments, a sample (e.g., cfDNA) is obtained from a previously diagnosed and treated subject at one or more preselected time points following the one or more previous cancer treatments.

The sample (e.g., cfDNA) obtained from the subject may be sequenced to provide a set of sequence information, which may include sequencing captured DNA molecules of the sequence-variable target region set a greater depth of sequencing than captured DNA molecules of the epigenetic target region set, as described in detail elsewhere herein.

### 3. Partitioning; Analysis of epigenetic characteristics

In certain embodiments described herein, a population of different forms of nucleic acids (e.g., hypermethylated and hypomethylated DNA in a sample from the subject, such as tagged DNA or an aliquot thereof) can be physically partitioned based on one or more characteristics of the nucleic acids prior to analysis, e.g., sequencing, or tagging and sequencing. This approach can be used to determine, for example, whether hypermethylation variable epigenetic target regions show hypermethylation characteristic of tumor cells or hypomethylation variable epigenetic target regions show hypomethylation characteristic of tumor cells or otherwise indicative of the presence of disease. Additionally, by partitioning a heterogeneous nucleic acid population, one may increase rare signals, e.g., by enriching rare nucleic acid molecules that are more prevalent in one fraction (or partition) of the population. For example, a genetic variation present in hyper-methylated DNA but less (or not) in hypomethylated DNA can be more easily detected by partitioning a sample into hyper-methylated and hypo-methylated nucleic acid molecules. By analyzing multiple fractions of a sample, a multi-dimensional analysis of a single locus of a genome or species of nucleic acid can be performed and hence, greater sensitivity can be achieved.

In some embodiments, the partitions are differentially tagged and then recombined before dividing the sample into first and second aliquots, followed by subsequent steps of methods described herein. In some embodiments, the sample that is divided into the first and second aliquots is a partition, such as a hypomethylated partition, and the second aliquot is combined with at least one other partition, such as a hypermethylated partition, before undergoing enrichment and/or other steps of the method.

In some instances, a heterogeneous nucleic acid sample is partitioned into two or more partitions (e.g., at least 3, 4, 5, 6 or 7 partitions). In some embodiments, each partition is differentially tagged. Tagged partitions can then be pooled together for collective sample prep and/or sequencing. The partitioning-tagging-pooling steps can occur more than once, with each round of partitioning occurring based on a different characteristics (examples provided herein) and tagged using differential tags that are distinguished from other partitions and partitioning means.

Examples of characteristics that can be used for partitioning include sequence length, methylation level, nucleosome binding, sequence mismatch, immunoprecipitation, and/or proteins that bind to DNA. Resulting partitions can include one or more of the following nucleic acid forms: single-stranded DNA (ssDNA), double-stranded DNA (dsDNA), shorter DNA fragments and longer DNA fragments. In some embodiments, a heterogeneous population of nucleic acids is partitioned into nucleic acids with one or more epigenetic modifications and without the one or more epigenetic modifications. Examples of epigenetic modifications include presence or absence of methylation; level of methylation; type of methylation (e.g., 5-methylcytosine versus other types of methylation, such as adenine methylation and/or cytosine hydroxymethylation); and association and level of association with one or more proteins, such as histones. Alternatively, or additionally, a heterogeneous population of nucleic acids can be partitioned into nucleic acid molecules associated with nucleosomes and nucleic acid molecules devoid of nucleosomes. Alternatively, or additionally, a heterogeneous population of nucleic acids may be partitioned into single-stranded DNA (ssDNA) and double-stranded DNA (dsDNA). Alternatively, or additionally, a heterogeneous population of nucleic acids may be partitioned based on nucleic acid length (e.g., molecules of up to 160 bp and molecules having a length of greater than 160 bp).

In some instances, each partition (representative of a different nucleic acid form) is differentially labelled, and the partitions are pooled together prior to sequencing. In other instances, the different forms are separately sequenced.

Samples can include nucleic acids varying in modifications including post-replication modifications to nucleotides and binding, usually noncovalently, to one or more proteins.

In an embodiment, the population of nucleic acids is one obtained from a serum, plasma or blood sample from a subject suspected of having neoplasia, a tumor, or cancer or previously diagnosed with neoplasia, a tumor, or cancer. The population of nucleic acids includes nucleic acids having varying levels of methylation. Methylation can occur from any one or more post-replication or transcriptional modifications. Post-replication modifications include modifications of the nucleotide cytosine, particularly at the 5-position of the nucleobase, e.g., 5-methylcytosine, 5-hydroxymethylcytosine, 5-formylcytosine and 5-carboxylcytosine.

In some embodiments, the nucleic acids in the original population can be single-stranded and/or double-stranded. Partitioning based on single v. double stranded-ness of the nucleic acids can be accomplished by, e.g. using labelled capture probes to partition ssDNA and using double stranded adapters to partition dsDNA.

The affinity agents can be antibodies with the desired specificity, natural binding partners or variants thereof (Bock et al., Nat Biotech 28: 1106-1114 (2010); Song et al., Nat Biotech 29: 68-72 (2011)), or artificial peptides selected e.g., by phage display to have specificity to a given target.

Examples of capture moieties contemplated herein include methyl binding domain (MBDs) and methyl binding proteins (MBPs) as described herein.

Likewise, partitioning of different forms of nucleic acids can be performed using histone binding proteins which can separate nucleic acids bound to histones from free or unbound nucleic acids. Examples of histone binding proteins that can be used in the methods disclosed herein include RBBP4 (RbAp48) and SANT domain peptides.

Although for some affinity agents and modifications, binding to the agent may occur in an essentially all or none manner depending on whether a nucleic acid bears a modification, the separation may be one of degree. In such instances, nucleic acids overrepresented in a modification bind to the agent at a greater extent that nucleic acids underrepresented in the modification. Alternatively, nucleic acids having modifications may bind in an all or nothing manner. But then, various levels of modifications may be sequentially eluted from the binding agent.

For example, in some embodiments, partitioning can be binary or based on degree/level of modifications. For example, all methylated fragments can be partitioned from unmethylated fragments using methyl-binding domain proteins (e.g., MethylMiner Methylated DNA Enrichment Kit (Thermo Fisher Scientific). Subsequently, additional partitioning may involve eluting fragments having different levels of methylation by adjusting the salt concentration in a solution with the methyl-binding domain and bound fragments. As salt concentration increases, fragments having greater methylation levels are eluted.

In some instances, the final partitions are representatives of nucleic acids having different extents of modifications (overrepresentative or underrepresentative of modifications). Overrepresentation and underrepresentation can be defined by the number of modifications born by a nucleic acid relative to the median number of modifications per strand in a population. For example, if the median number of 5-methylcytosine residues in nucleic acid in a sample is 2, a nucleic acid including more than two 5-methylcytosine residues is overrepresented in this modification and a nucleic acid with 1 or zero 5-methylcytosine residues is underrepresented. The effect of the affinity separation is to enrich for nucleic acids overrepresented in a modification in a bound phase and for nucleic acids underrepresented in a modification in an unbound phase (i.e. in solution). The nucleic acids in the bound phase can be eluted before subsequent processing.

When using MethylMiner Methylated DNA Enrichment Kit (Thermo Fisher Scientific) various levels of methylation can be partitioned using sequential elutions. For example, a hypomethylated partition (e.g., no methylation) can be separated from a methylated partition by contacting the nucleic acid population with the MBD from the kit, which is attached to magnetic beads. The beads are used to separate out the methylated nucleic acids from the non- methylated nucleic acids. Subsequently, one or more elution steps are performed sequentially to elute nucleic acids having different levels of methylation. For example, a first set of methylated nucleic acids can be eluted at a salt concentration of 160 mM or higher, e.g., at least 200 mM, 300 mM, 400 mM, 500 mM, 600 mM, 700 mM, 800 mM, 900 mM, 1000 mM, or 2000 mM. After such methylated nucleic acids are eluted, magnetic separation is once again used to separate higher level of methylated nucleic acids from those with lower level of methylation. The elution and magnetic separation steps can repeat themselves to create various partitions such as a hypomethylated partition (e.g., representative of no methylation), a methylated partition (representative of low level of methylation), and a hyper methylated partition (representative of high level of methylation).

In some methods, nucleic acids bound to an agent used for affinity separation are subjected to a wash step. The wash step washes off nucleic acids weakly bound to the affinity agent. Such nucleic acids can be enriched in nucleic acids having the modification to an extent close to the mean or median (i.e., intermediate between nucleic acids remaining bound to the solid phase and nucleic acids not binding to the solid phase on initial contacting of the sample with the agent).

The affinity separation results in at least two, and sometimes three or more partitions of nucleic acids with different extents of a modification. While the partitions are still separate, the nucleic acids of at least one partition, and usually two or three (or more) partitions are linked to nucleic acid tags, usually provided as components of adapters, with the nucleic acids in different partitions receiving different tags that distinguish members of one partition from another. The tags linked to nucleic acid molecules of the same partition can be the same or different from one another. But if different from one another, the tags may have part of their code in common so as to identify the molecules to which they are attached as being of a particular partition.

For further details regarding portioning nucleic acid samples based on characteristics such as methylation, see WO2018/119452.

In some embodiments, the nucleic acid molecules can be fractionated into different partitions based on the nucleic acid molecules that are bound to a specific protein or a fragment thereof and those that are not bound to that specific protein or fragment thereof.

Nucleic acid molecules can be fractionated based on DNA-protein binding. Protein-DNA complexes can be fractionated based on a specific property of a protein. Examples of such properties include various epitopes, modifications (e.g., histone methylation or acetylation) or enzymatic activity. Examples of proteins which may bind to DNA and serve as a basis for fractionation may include, but are not limited to, protein A and protein G. Any suitable method can be used to fractionate the nucleic acid molecules based on protein bound regions. Examples of methods used to fractionate nucleic acid molecules based on protein bound regions include, but are not limited to, SDS-PAGE, chromatin-immuno-precipitation (ChIP), heparin chromatography, and asymmetrical field flow fractionation (AF4).

In some embodiments, partitioning of the nucleic acids is performed by contacting the nucleic acids with a methylation binding domain ("MBD") of a methylation binding protein ("MBP"). MBD binds to 5-methylcytosine (5mC). MBD is coupled to paramagnetic beads, such as Dynabeads^{®} M-280 Streptavidin via a biotin linker. Partitioning into fractions with different extents of methylation can be performed by eluting fractions by increasing the NaCl concentration.

Examples of MBPs contemplated herein include, but are not limited to:
(a) MeCP2 is a protein preferentially binding to 5-methyl-cytosine over unmodified cytosine.
(b) RPL26, PRP8 and the DNA mismatch repair protein MHS6 preferentially bind to 5-hydroxymethyl-cytosine over unmodified cytosine.
(c) FOXK1, FOXK2, FOXP1, FOXP4 and FOXI3 preferably bind to 5-formyl-cytosine over unmodified cytosine (Iurlaro et al., Genome Biol. 14: R119 (2013)).
(d) Antibodies specific to one or more methylated nucleotide bases.

In general, elution is a function of number of methylated sites per molecule, with molecules having more methylation eluting under increased salt concentrations. To elute the DNA into distinct populations based on the extent of methylation, one can use a series of elution buffers of increasing NaCl concentration. Salt concentration can range from about 100 mM to about 2500 mM NaCl. In one embodiment, the process results in three (3) partitions. Molecules are contacted with a solution at a first salt concentration and comprising a molecule comprising a methyl binding domain, which molecule can be attached to a capture moiety, such as streptavidin. At the first salt concentration a population of molecules will bind to the MBD and a population will remain unbound. The unbound population can be separated as a "hypomethylated" population. For example, a first partition representative of the hypomethylated form of DNA is that which remains unbound at a low salt concentration, e.g., 100 mM or 160 mM. A second partition representative of intermediate methylated DNA is eluted using an intermediate salt concentration, e.g., between 100 mM and 2000 mM concentration. This is also separated from the sample. A third partition representative of hypermethylated form of DNA is eluted using a high salt concentration, e.g., at least about 2000 mM.

In some embodiments, e.g., wherein an epigenetic target region set is captured, sample DNA (for e.g., between 1 and 300 ng) is mixed with an appropriate amount of methyl binding domain (MBD) buffer (the amount of MBD buffer depends on the amount of DNA used) and magnetic beads conjugated with MBD proteins and incubated overnight. Methylated DNA (hypermethylated DNA) binds the MBD protein on the magnetic beads during this incubation. Non-methylated (hypomethylated DNA) or less methylated DNA (intermediately methylated) is washed away from the beads with buffers containing increasing concentrations of salt. For example, one, two, or more fractions containing non-methylated, hypomethylated, and/or intermediately methylated DNA may be obtained from such washes. Finally, a high salt buffer is used to elute the heavily methylated DNA (hypermethylated DNA) from the MBD protein. In some embodiments, these washes result in three partitions (hypomethylated partition, intermediately methylated fraction and hypermethylated partition) of DNA having increasing levels of methylation.

In some embodiments, the three partitions of DNA are desalted and concentrated in preparation for the enzymatic steps of library preparation.

### 4. Tags; adapters; barcodes

In some embodiments, the tagged DNA comprises adapters. Adapters can be added concurrently with an amplification procedure, e.g., by providing the adapters in a 5' portion of a primer, e.g., as described elsewhere herein. Alternatively, adapters can be added by other approaches, such as ligation. Tags comprising barcodes can be incorporated into or otherwise joined to adapters. Tags can be incorporated by ligation or overlap extension PCR among other methods. In some embodiments, tags, which may be or include barcodes, are included in the DNA. Tags can facilitate identification of the origin of a nucleic acid. For example, barcodes can be used to allow the origin (e.g., subject) from which the DNA came to be identified following pooling of a plurality of samples for parallel sequencing. This may be done concurrently with an amplification procedure, e.g., by providing the barcodes in a 5' portion of a primer, e.g., as described above. In some embodiments, adapters and tags/barcodes are provided by the same primer or primer set. For example, the barcode may be located 3' of the adapter and 5' of the target-hybridizing portion of the primer. Alternatively, barcodes can be added by other approaches, such as ligation, optionally together with adapters in the same ligation substrate.

In some embodiments (e.g., after concentrating the DNA in partitions, where applicable), the captured DNA is made ligatable, e.g., by extending the end overhangs of the DNA molecules are extended, and adding adenosine residues to the 3' ends of fragments and phosphorylating the 5' end of each DNA fragment. DNA ligase and adapters are added to ligate each partitioned DNA molecule with an adapter on each end. These adapters can contain partition tags (e.g., non-random, non-unique barcodes) that are distinguishable from the partition tags in the adapters used in the other partitions. After ligation, the three partitions can be pooled together and amplified (e.g., by PCR, such as with primers specific for the adapters).

Following PCR, amplified DNA may be cleaned and concentrated prior to further steps (e.g., division into first and second aliquots).

### a. Molecular tagging strategies

Molecular tagging refers to a tagging practice that allows one to differentiate molecules from which sequence reads originated. Tagging strategies can be divided into unique tagging and non-unique tagging strategies. In unique tagging, all or substantially all the molecules in a sample bear a different tag, so that reads can be assigned to original molecules based on tag information alone. Tags used in such methods are sometimes referred to as "unique tags". In non-unique tagging, different molecules in the same sample can bear the same tag, so that other information in addition to tag information is used to assign a sequence read to an original molecule. Such information may include start and stop coordinate, coordinate to which the molecule maps, start or stop coordinate alone, etc. Tags used in such methods are sometimes referred to as "non-unique tags." Accordingly, it is not necessary to uniquely tag every molecule in a sample. It suffices to uniquely tag molecules falling within an identifiable class within a sample. Thus, molecules in different identifiable families can bear the same tag without loss of information about the identity of the tagged molecule.

In certain embodiments of non-unique tagging, the number of different tags used can be sufficient that there is a very high likelihood (e.g., at least 99%, at least 99.9%, at least 99.99% or at least 99.999%) that all molecules of a particular group bear a different tag. It is to be noted that when barcodes are used as tags, and when barcodes are attached, e.g., randomly, to both ends of a molecule, the combination of barcodes, together, can constitute a tag. This number, in term, is a function of the number of molecules falling into the calls. For example, the class may be all molecules mapping to the same start-stop position on a reference genome. The class may be all molecules mapping across a particular genetic locus, e.g., a particular base or a particular region (e.g., up to 100 bases or a gene or an exon of a gene). In certain embodiments, the number of different tags used to uniquely identify a number of molecules, z, in a class can be between any of 2*z, 3*z, 4*z, 5*z, 6*z, 7*z, 8*z, 9*z, 10*z, 11 *z, 12*z, 13*z, 14*z, 15*z, 16*z, 17*z, 18*z, 19*z, 20*z or 100*z (e.g., lower limit) and any of 100,000*z, 10,000*z, 1000*z or 100*z (e.g., upper limit).

For example, in a sample of about 3 ng to 30 ng of human cell free DNA, one expects around 103-104 molecules to map to a particular nucleotide coordinate, and between about 3 and 10 molecules having any start coordinate to share the same stop coordinate. Accordingly, about 50 to about 50,000 different tags (e.g., between about 6 and 220 barcode combinations) can suffice to uniquely tag all such molecules. To uniquely tag all 103-104 molecules mapping across a nucleotide coordinate, about 1 million to about 20 million different tags would be required.

Generally, assignment of unique or non-unique tags barcodes in reactions follows methods and systems described by US patent applications US2001053519A1, US2003152490A1, US2011160078A1, and U.S. Pat. No. 6,582,908 and U.S. Pat. No. 7,537,898 and US Pat. No. 9,598,731. Tags can be linked to sample nucleic acids randomly or non-randomly.

In some embodiments, the tagged nucleic acids are sequenced after loading into a microwell plate. The microwell plate can have 96, 384, or 1536 microwells. In some cases, they are introduced at an expected ratio of unique tags to microwells. For example, the unique tags may be loaded so that more than about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags are loaded per genome sample. In some cases, the unique tags may be loaded so that less than about 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags are loaded per genome sample. In some cases, the average number of unique tags loaded per sample genome is less than, or greater than, about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags per genome sample.

A preferred format uses 20-50 different tags (e.g., barcodes) ligated to both ends of target nucleic acids. For example, 35 different tags (e.g., barcodes) ligated to both ends of target molecules creating 35 × 35 permutations, which equals 1225 tag combinations for 35 tags. Such numbers of tags are sufficient so that different molecules having the same start and stop points have a high probability (e.g., at least 94%, 99.5%, 99.99%, 99.999%) of receiving different combinations of tags. Other barcode combinations include any number between 10 and 500, e.g., about 15x15, about 35x35, about 75x75, about 100x100, about 250x250, about 500x500.

In some cases, unique tags may be predetermined or random or semi-random sequence oligonucleotides. In other cases, a plurality of barcodes may be used such that barcodes are not necessarily unique to one another in the plurality. In this example, barcodes may be ligated to individual molecules such that the combination of the barcode and the sequence it may be ligated to creates a unique sequence that may be individually tracked. As described herein, detection of non-unique barcodes in combination with sequence data of beginning (start) and end (stop) portions of sequence reads may allow assignment of a unique identity to a particular molecule. The length or number of base pairs, of an individual sequence read may also be used to assign a unique identity to such a molecule. As described herein, fragments from a single strand of nucleic acid having been assigned a unique identity, may thereby permit subsequent identification of fragments from the parent strand.

Where samples undergo partitioning, tags may be applied in a differential manner. For example, tags may include a sample-specific component and a molecule-specific component, and may optionally further include a partition-specific component.

### b. Tagging of partitions

In some embodiments, samples are partitioned as described elsewhere herein. In some embodiments, two or more partitions, e.g., each partition, is/are differentially tagged. Tags can be molecules, such as nucleic acids, containing information that indicates a feature of the molecule with which the tag is associated. For example, molecules can bear a sample tag (which distinguishes molecules in one sample from those in a different sample), a partition tag (which distinguishes molecules in one partition from those in a different partition) or a molecular tag (which distinguishes different molecules from one another (in both unique and non-unique tagging scenarios). In certain embodiments, a tag can comprise one or a combination of barcodes. As used herein, the term "barcode" refers to a nucleic acid molecule having a particular nucleotide sequence, or to the nucleotide sequence, itself, depending on context. A barcode can have, for example, between 10 and 100 nucleotides. A collection of barcodes can have degenerate sequences or can have sequences having a certain Hamming distance, as desired for the specific purpose. So, for example, a sample index, partition index or molecular index can be comprised of one barcode or a combination of two barcodes, each attached to different ends of a molecule.

Tags can be used to label the individual polynucleotide population partitions so as to correlate the tag (or tags) with a specific partition. Alternatively, tags can be used in embodiments of the invention that do not employ a partitioning step. In some embodiments, a single tag can be used to label a specific partition. In some embodiments, multiple different tags can be used to label a specific partition. In embodiments employing multiple different tags to label a specific partition, the set of tags used to label one partition can be readily differentiated for the set of tags used to label other partitions. In some embodiments, the tags may have additional functions, for example the tags can be used to index sample sources or used as unique molecular identifiers (which can be used to improve the quality of sequencing data by differentiating sequencing errors from mutations, for example as in Kinde et al., Proc Nat'l Acad Sci USA 108: 9530-9535 (2011), Kou et al., PLoS ONE,11: e0146638 (2016)) or used as non-unique molecule identifiers, for example as described in US Pat. No. 9,598,731. Similarly, in some embodiments, the tags may have additional functions, for example the tags can be used to index sample sources or used as non-unique molecular identifiers (which can be used to improve the quality of sequencing data by differentiating sequencing errors from mutations).

In one embodiment, partition tagging comprises tagging molecules in each partition with a partition tag. After re-combining partitions and sequencing molecules, the partition tags identify the source partition. In another embodiment, different partitions are tagged with different sets of molecular tags, e.g., comprised of a pair of barcodes. In this way, each molecular barcode indicates the source partition as well as being useful to distinguish molecules within a partition. For example, a first set of 35 barcodes can be used to tag molecules in a first partition, while a second set of 35 barcodes can be used tag molecules in a second partition.

In some embodiments, after partitioning and tagging with partition tags, the molecules may be pooled for sequencing in a single run. In some embodiments, a sample tag is added to the molecules, e.g., in a step subsequent to addition of partition tags and pooling. Sample tags can facilitate pooling material generated from multiple samples for sequencing in a single sequencing run.

Alternatively, in some embodiments, partition tags may be correlated to the sample as well as the partition. As a simple example, a first tag can indicate a first partition of a first sample; a second tag can indicate a second partition of the first sample; a third tag can indicate a first partition of a second sample; and a fourth tag can indicate a second partition of the second sample.

While tags may be attached to molecules already partitioned based on one or more characteristics, the final tagged molecules in the library may no longer possess that characteristic. For example, while single stranded DNA molecules may be partitioned and tagged, the final tagged molecules in the library are likely to be double stranded. Similarly, while DNA may be subject to partition based on different levels of methylation, in the final library, tagged molecules derived from these molecules are likely to be unmethylated. Accordingly, the tag attached to molecule in the library typically indicates the characteristic of the "parent molecule" from which the ultimate tagged molecule is derived, not necessarily to characteristic of the tagged molecule, itself.

As an example, barcodes 1, 2, 3, 4, etc. are used to tag and label molecules in the first partition; barcodes A, B, C, D, etc. are used to tag and label molecules in the second partition; and barcodes a, b, c, d, etc. are used to tag and label molecules in the third partition. Differentially tagged partitions can be pooled prior to sequencing. Differentially tagged partitions can be separately sequenced or sequenced together concurrently, e.g., in the same flow cell of an Illumina sequencer.

After sequencing, analysis of reads to detect genetic variants can be performed on a partition-by-partition level, as well as a whole nucleic acid population level. Tags are used to sort reads from different partitions. Analysis can include in silico analysis to determine genetic and epigenetic variation (one or more of methylation, chromatin structure, etc.) using sequence information, genomic coordinates length, coverage and/or copy number. In some embodiments, higher coverage can correlate with higher nucleosome occupancy in genomic region while lower coverage can correlate with lower nucleosome occupancy or a nucleosome depleted region (NDR).

### 5. Amplification

Sample nucleic acids flanked by adapters can be amplified by PCR and other amplification methods. Amplification is typically primed by primers binding to primer binding sites in adapters flanking a DNA molecule to be amplified. Amplification methods can involve cycles of denaturation, annealing and extension, resulting from thermocycling or can be isothermal as in transcription-mediated amplification. Other amplification methods include the ligase chain reaction, strand displacement amplification, nucleic acid sequence-based amplification, and self-sustained sequence-based replication.

Preferably, the present methods perform dsDNA 'TV A ligations' with T-tailed and C-tailed adapters, which result in amplification of at least 50, 60, 70 or 80% of double stranded nucleic acids before linking to adapters. Preferably the present methods increase the amount or number of amplified molecules relative to control methods performed with T-tailed adapters alone by at least 10, 15 or 20%.

### 6. Enriching the second aliquot of tagged DNA

The following discussion relates to the processing, enriching, and sequencing of DNA from the second aliquot of tagged DNA molecules in the methods of detecting genomic rearrangements disclosed herein. For example, in some embodiments of the methods of detecting the presence or absence of genomic rearrangements, the enriching the second aliquot comprises contacting the DNA of the second aliquot with a plurality of target-binding probes specific for the second plurality of loci of interest.

In some embodiments, methods disclosed herein comprise a step of capturing one or more sets of target regions of DNA, such as cfDNA. Capture may be performed using any suitable approach known in the art.

In some embodiments, capturing comprises contacting the DNA to be captured with a set of target-specific probes. The set of target-specific probes may have any of the features described herein for sets of target-specific probes, including but not limited to in the embodiments set forth above and the sections relating to probes below.

The capturing step may be performed using conditions suitable for specific nucleic acid hybridization, which generally depend to some extent on features of the probes such as length, base composition, etc. Those skilled in the art will be familiar with appropriate conditions given general knowledge in the art regarding nucleic acid hybridization. In some embodiments, complexes of target-specific probes and DNA are formed.

In some embodiments, complexes of target-specific probes and DNA are separated from DNA not bound to target-specific probes. For example, where target-specific probes are bound covalently or noncovalently to a solid support, a washing or aspiration step can be used to separate unbound material. Alternatively, where the complexes have chromatographic properties distinct from unbound material (e.g., where the probes comprise a ligand that binds a chromatographic resin), chromatography can be used.

As discussed in detail elsewhere herein, the set of target-specific probes may comprise a plurality of sets such as probes for a sequence-variable target region set and probes for an epigenetic target region set. In some such embodiments, the capturing step is performed with the probes for the plurality of sets in the same vessel at the same time, e.g., one or both of probes for a sequence-variable target region set and probes for an epigenetic target region set are in the same composition. This approach provides a relatively streamlined workflow.

Alternatively, the capturing step can be performed with the sequence-variable target region set in a first vessel and the epigenetic target region probe set in a second vessel. Alternatively, the contacting step can be performed with one or more probe sets (e.g., the sequence-variable target region set) at a first time and a first vessel and one or more other probe sets (e.g., the epigenetic target region probe set) at a second time before or after the first time. This approach allows for preparation of separate first and second compositions comprising captured DNA corresponding to different target region sets. The compositions can be processed separately as desired (e.g., to fractionate based on methylation as described elsewhere herein) and recombined in appropriate proportions to provide material for further processing and analysis such as sequencing.

In some embodiments, the DNA is amplified. In some embodiments, amplification is performed before the capturing step. In some embodiments, amplification is performed after the capturing step. Methods for nonspecific amplification of DNA are known in the art. See, e.g., Smallwood et al., Nat. Methods 11: 817-820 (2014). For example, random primers having adapter sequences on their 5' ends and random bases on the 3' end can be used. There are usually 6 random bases but can be between 4 and 9 bases long. This approach is amenable for low input/single cell amplification and/or bisulfite sequencing.

In an exemplary workflow for processing of the second aliquot, the amplified DNA can be contacted with a collection of probes described herein (which may be, e.g., biotinylated RNA probes) that target specific regions of interest. The mixture is incubated, e.g., overnight, e.g., in a salt buffer. The probes are captured (e.g., using streptavidin magnetic beads) and separated from the amplified DNA that was not captured, such as by a series of salt washes, thereby providing a captured set of DNA. After capture, the DNA of the captured set is amplified by PCR. In some embodiments, the PCR primers contain a sample tag, thereby incorporating the sample tag into the DNA molecules. In some embodiments, DNA from different samples is pooled together and then multiplex sequenced, e.g., using an Illumina NovaSeq sequencer.

### a. Captured set

Genetic and/or epigenetic information obtained from DNA of the subject can be combined to provide a determination of whether a subject has a cancer or a likelihood that the subject has a cancer. Detailed descriptions of how to analyze cell free human DNA for both genetic and epigenetic variants associated with cancer can be found in US provisional patent application 62/799637. Additional guidance for analyzing cell free DNA for the detecting cancer can be found in, among other places US Patent 9834822, PCT application WO2018064629A1, and PCT application WO2017106768A1.

Various embodiments include the step of sequencing DNA (e.g., cfDNA) for the purpose of detecting genetic variants in genes associated with cancer. Various embodiments also include the step of sequencing DNA (e.g., cfDNA) for the purpose of detecting epigenetic variants in genes associated with cancer, epigenetic variants include (1) DNA sequences that are differentially methylated in cancerous and noncancerous cells and (2) nucleosomal fragmentation patterns such as those described in US published patent application US2017/0211143A1.

In some embodiments, a captured set of nucleic acid, e.g., comprising DNA (such as cfDNA) is provided. With respect to the disclosed methods, the captured set of DNA may be provided, e.g., following capturing, and/or separating steps as described herein. The captured set may comprise DNA corresponding to one or both of a sequence-variable target region set and an epigenetic target region set. In some embodiments, the captured set comprises DNA corresponding to a a sequence-variable target region set, and an epigenetic target region set. In all embodiments described herein involving a sequence-variable target region set and an epigenetic target region set, the sequence-variable target region set comprises regions not present in the epigenetic target region set and vice versa, although in some instances a fraction of the regions may overlap (e.g., a fraction of genomic positions may be represented in both target region sets).

### b. Epigenetic target region set

In some embodiments, an epigenetic target region set is captured. The epigenetic target region set may comprise one or more types of target regions likely to differentiate DNA from neoplastic (e.g., tumor or cancer) cells and from healthy cells, e.g., non-neoplastic circulating cells. The epigenetic target region set can be analyzed in various ways, including methods that do not depend on a high degree of accuracy in sequence determination of specific nucleotides within a target. Exemplary types of such regions are discussed in detail herein. In some embodiments, methods according to the disclosure comprise determining whether cfDNA molecules corresponding to the epigenetic target region set comprise or indicate cancer-associated epigenetic modifications (e.g., hypermethylation in one or more hypermethylation variable target regions; one or more perturbations of CTCF binding; and/or one or more perturbations of transcription start sites) and/or copy number variations (e.g., focal amplifications). Such analyses can be conducted by sequencing and require less data (e.g., number of sequence reads or depth of sequencing coverage) than determining the presence or absence of a sequence mutation such as a base substitution, insertion, or deletion. The epigenetic target region set may also comprise one or more control regions, e.g., as described herein.

In some embodiments, the epigenetic target region set has a footprint of at least 100 kb, e.g., at least 200 kb, at least 300 kb, or at least 400 kb. In some embodiments, the epigenetic target region set has a footprint in the range of 100-1000 kb, e.g., 100-200 kb, 200-300 kb, 300-400 kb, 400-500 kb, 500-600 kb, 600-700 kb, 700-800 kb, 800-900 kb, and 900-1,000 kb.

### i. Hypermethylation variable target regions

In some embodiments, the epigenetic target region set comprises one or more hypermethylation variable target regions. In general, hypermethylation variable target regions refer to regions where an increase in the level of observed methylation indicates an increased likelihood that a sample (e.g., of cfDNA) contains DNA produced by neoplastic cells, such as tumor or cancer cells. For example, hypermethylation of promoters of tumor suppressor genes has been observed repeatedly. See, e.g., Kang et al., Genome Biol. 18:53 (2017) and references cited therein.

An extensive discussion of methylation variable target regions in colorectal cancer is provided in Lam et al., Biochim Biophys Acta. 1866:106-20 (2016). These include VIM, SEPT9, ITGA4, OSM4, GATA4 and NDRG4. An exemplary set of hypermethylation variable target regions comprising the genes or portions thereof based on the colorectal cancer (CRC) studies is provided in Table 1. Many of these genes likely have relevance to cancers beyond colorectal cancer; for example, TP53 is widely recognized as a critically important tumor suppressor and hypermethylation-based inactivation of this gene may be a common oncogenic mechanism.

**Table 1. Exemplary hypermethylation target regions (genes or portions thereof) based on CRC studies.**

| **Gene Name** | **Additional Gene Name** | **Chromosome** |
|---|---|---|
| VIM | | chr10 |
| SEPT9 | | chrl7 |
| CYCD2 | CCND2 | chr12 |
| TFPI2 | | chr7 |
| GATA4 | | chr8 |
| RARB2 | RARB | chr3 |
| p16INK4a | CDKN2A | chr9 |
| MGMT | MGMT | chr10 |
| APC | | chr5 |
| NDRG4 | | chr16 |
| HLTF | | chr3 |
| HPP1 | TMEFF2 | chr2 |
| hMLH1 | MLH1 | chr3 |
| RASSF1A | RASSF1 | chr3 |
| CDH13 | | chr16 |
| IGFBP3 | | chr7 |
| ITGA4 | | chr2 |

In some embodiments, the hypermethylation variable target regions comprise a plurality of genes or portions thereof listed in Table 1, e.g., at least 10%, 20%, 30%, 40%, 50%, 60%, 70%, 80%, 90%, or 100% of the genes or portions thereof listed in Table 1. For example, for each locus included as a target region, there may be one or more probes with a hybridization site that binds between the transcription start site and the stop codon (the last stop codon for genes that are alternatively spliced) of the gene. In some embodiments, the one or more probes bind within 300 bp upstream and/or downstream of the genes or portions thereof listed in Table 1, e.g., within 200 or 100 bp.

Methylation variable target regions in various types of lung cancer are discussed in detail, e.g., in Ooki et al., Clin. Cancer Res. 23:7141-52 (2017); Belinksy, Annu. Rev. Physiol. 77:453-74 (2015); Hulbert et al., Clin. Cancer Res. 23:1998-2005 (2017); Shi et al., BMC Genomics 18:901 (2017); Schneider et al., BMC Cancer. 11:102 (2011); Lissa et al., Transl Lung Cancer Res 5(5):492-504 (2016); Skvortsova et al., Br. J. Cancer. 94(10):1492-1495 (2006); Kim et al., Cancer Res. 61:3419-3424 (2001); Furonaka et al., Pathology International 55:303-309 (2005); Gomes et al., Rev. Port. Pneumol. 20:20-30 (2014); Kim et al., Oncogene. 20:1765-70 (2001); Hopkins-Donaldson et al., Cell Death Differ. 10:356-64 (2003); Kikuchi et al., Clin. Cancer Res. 11:2954-61 (2005); Heller et al., Oncogene 25:959-968 (2006); Licchesi et al., Carcinogenesis. 29:895-904 (2008); Guo et al., Clin. Cancer Res. 10:7917-24 (2004); Palmisano et al., Cancer Res. 63:4620-4625 (2003); and Toyooka et al., Cancer Res. 61:4556-4560, (2001).

An exemplary set of hypermethylation variable target regions comprising genes or portions thereof based on the lung cancer studies is provided in Table 2. Many of these genes likely have relevance to cancers beyond lung cancer; for example, Casp8 (Caspase 8) is a key enzyme in programmed cell death and hypermethylation-based inactivation of this gene may be a common oncogenic mechanism not limited to lung cancer. Additionally, a number of genes appear in both Tables 1 and 2, indicating generality.

**Table 2. Exemplary hypermethylation target regions (genes or portions thereof) based on lung cancer studies**

| **Gene Name** | **Chromosome** |
|---|---|
| MARCH11 | chr5 |
| TAC1 | chr7 |
| TCF21 | chr6 |
| SHOX2 | chr3 |
| p16 | chr3 |
| Casp8 | chr2 |
| CDH13 | chr16 |
| MGMT | chr10 |
| MLH1 | chr3 |
| MSH2 | chr2 |
| TSLC1 | chr11 |
| APC | chr5 |
| DKK1 | chr10 |
| DKK3 | chr11 |
| LKB1 | chr11 |
| WIF1 | chr12 |
| RUNX3 | chr1 |
| GATA4 | chr8 |
| GATA5 | chr20 |
| PAX5 | chr9 |
| E-Cadherin | chr16 |
| H-Cadherin | chr16 |

Any of the foregoing embodiments concerning target regions identified in Table 2 may be combined with any of the embodiments described above concerning target regions identified in Table 1. In some embodiments, the hypermethylation variable target regions comprise a plurality of genes or portions thereof listed in Table 1 or Table 2, e.g., at least 10%, 20%, 30%, 40%, 50%, 60%, 70%, 80%, 90%, or 100% of the genes or portions thereof listed in Table 1 or Table 2.

Additional hypermethylation target regions may be obtained, e.g., from the Cancer Genome Atlas. Kang et al., Genome Biology 18:53 (2017), describe construction of a probabilistic method called Cancer Locator using hypermethylation target regions from breast, colon, kidney, liver, and lung. In some embodiments, the hypermethylation target regions can be specific to one or more types of cancer. Accordingly, in some embodiments, the hypermethylation target regions include one, two, three, four, or five subsets of hypermethylation target regions that collectively show hypermethylation in one, two, three, four, or five of breast, colon, kidney, liver, and lung cancers.

### ii. Hypomethylation variable target regions

Global hypomethylation is a commonly observed phenomenon in various cancers. See, e.g., Hon et al., Genome Res. 22:246-258 (2012) (breast cancer); Ehrlich, Epigenomics 1:239-259 (2009) (review article noting observations of hypomethylation in colon, ovarian, prostate, leukemia, hepatocellular, and cervical cancers). For example, regions such as repeated elements, e.g., LINE1 elements, Alu elements, centromeric tandem repeats, pericentromeric tandem repeats, and satellite DNA, and intergenic regions that are ordinarily methylated in healthy cells may show reduced methylation in tumor cells. Accordingly, in some embodiments, the epigenetic target region set includes hypomethylation variable target regions, where a decrease in the level of observed methylation indicates an increased likelihood that a sample (e.g., of cfDNA) contains DNA produced by neoplastic cells, such as tumor or cancer cells.

In some embodiments, hypomethylation variable target regions include repeated elements and/or intergenic regions. In some embodiments, repeated elements include one, two, three, four, or five of LINE1 elements, Alu elements, centromeric tandem repeats, pericentromeric tandem repeats, and/or satellite DNA.

Exemplary specific genomic regions that show cancer-associated hypomethylation include nucleotides 8403565-8953708 and 151104701-151106035 of human chromosome 1, e.g., according to the hg19 or hg38 human genome construct. In some embodiments, the hypomethylation variable target regions overlap or comprise one or both of these regions.

### iii. CTCF binding regions

CTCF is a DNA-binding protein that contributes to chromatin organization and often colocalizes with cohesin. Perturbation of CTCF binding sites has been reported in a variety of different cancers. *See, e.g.,* Katainen et al., Nature Genetics, doi: 10.1038/ng.3335, published online 8 June 2015; Guo et al., Nat. Commun. 9:1520 (2018). CTCF binding results in recognizable patterns in cfDNA that can be detected by sequencing, e.g., through fragment length analysis. For example, details regarding sequencing-based fragment length analysis are provided in Snyder et al., Cell 164:57-68 (2016); WO 2018/009723; and US20170211143A1.

Thus, perturbations of CTCF binding result in variation in the fragmentation patterns of cfDNA. As such, CTCF binding sites represent a type of fragmentation variable target regions.

There are many known CTCF binding sites. See, e.g., the CTCFBSDB (CTCF Binding Site Database), available on the Internet at insulatordb.uthsc.edu/; Cuddapah et al., Genome Res. 19:24-32 (2009); Martin et al., Nat. Struct. Mol. Biol. 18:708-14 (2011); Rhee et al., Cell. 147:1408-19 (2011). Exemplary CTCF binding sites are at nucleotides 56014955-56016161 on chromosome 8 and nucleotides 95359169-95360473 on chromosome 13, e.g., according to the hg19 or hg38 human genome construct.

Accordingly, in some embodiments, the epigenetic target region set includes CTCF binding regions. In some embodiments, the CTCF binding regions comprise at least 10, 20, 50, 100, 200, or 500 CTCF binding regions, or 10-20, 20-50, 50-100, 100-200, 200-500, or 500-1000 CTCF binding regions, e.g., such as CTCF binding regions described above or in one or more of CTCFBSDB or the Cuddapah et al., Martin et al., or Rhee et al. articles cited above.

In some embodiments, at least some of the CTCF sites can be methylated or unmethylated, wherein the methylation state is correlated with the whether or not the cell is a cancer cell. In some embodiments, the epigenetic target region set comprises at least 100 bp, at least 200 bp, at least 300 bp, at least 400 bp, at least 500 bp, at least 750 bp, at least 1000 bp upstream and/or downstream regions of the CTCF binding sites.

### iv. Transcription start sites

Transcription start sites may also show perturbations in neoplastic cells. For example, nucleosome organization at various transcription start sites in healthy cells of the hematopoietic lineage-which contributes substantially to cfDNA in healthy individuals-may differ from nucleosome organization at those transcription start sites in neoplastic cells. This results in different cfDNA patterns that can be detected by sequencing, for example, as discussed generally in Snyder et al., Cell 164:57-68 (2016); WO 2018/009723; and US20170211143A1.

Thus, perturbations of transcription start sites also result in variation in the fragmentation patterns of cfDNA. As such, transcription start sites also represent a type of fragmentation variable target regions.

Human transcriptional start sites are available from DBTSS (DataBase of Human Transcription Start Sites), available on the Internet at dbtss.hgc.jp and described in Yamashita et al., Nucleic Acids Res. 34(Database issue): D86-D89 (2006).

Accordingly, in some embodiments, the epigenetic target region set includes transcriptional start sites. In some embodiments, the transcriptional start sites comprise at least 10, 20, 50, 100, 200, or 500 transcriptional start sites, or 10-20, 20-50, 50-100, 100-200, 200-500, or 500-1000 transcriptional start sites, e.g., such as transcriptional start sites listed in DBTSS. In some embodiments, at least some of the transcription start sites can be methylated or unmethylated, wherein the methylation state is correlated with the whether or not the cell is a cancer cell. In some embodiments, the epigenetic target region set comprises at least 100 bp, at least 200 bp, at least 300 bp, at least 400 bp, at least 500 bp, at least 750 bp, at least 1000 bp upstream and/or downstream regions of the transcription start sites.

### v. Copy number variations; focal amplifications

Although copy number variations such as focal amplifications are somatic mutations, they can be detected by sequencing based on read frequency in a manner analogous to approaches for detecting certain epigenetic changes such as changes in methylation. As such, regions that may show copy number variations such as focal amplifications in cancer can be included in the epigenetic target region set and may comprise one or more of AR, BRAF, CCND1, CCND2, CCNE1, CDK4, CDK6, EGFR, ERBB2, FGFR1, FGFR2, KIT, KRAS, MET, MYC, PDGFRA, PIK3CA, and RAF1. For example, in some embodiments, the epigenetic target region set comprises at least 2, 3, 4, 5, 6, 7, 8, 9, 10, 11, 12, 13, 14, 15, 16, 17, or 18 of the foregoing targets.

### vi. Methylation control regions

It can be useful to include control regions to facilitate data validation. In some embodiments, the epigenetic target region set includes control regions that are expected to be methylated or unmethylated in essentially all samples, regardless of whether the DNA is derived from a cancer cell or a normal cell. In some embodiments, the epigenetic target region set includes control hypomethylated regions that are expected to be hypomethylated in essentially all samples. In some embodiments, the epigenetic target region set includes control hypermethylated regions that are expected to be hypermethylated in essentially all samples.

### c. Sequence-variable target region set

In some embodiments, the sequence-variable target region set comprises a plurality of regions known to undergo somatic mutations in cancer (referred to herein as cancer-associated mutations). Accordingly, methods may comprise determining whether cfDNA molecules corresponding to the sequence-variable target region set comprise cancer-associated mutations.

In some embodiments, the sequence-variable target region set targets a plurality of different genes or genomic regions ("panel") selected such that a determined proportion of subjects having a cancer exhibits a genetic variant or tumor marker in one or more different genes or genomic regions in the panel. The panel may be selected to limit a region for sequencing to a fixed number of base pairs. The panel may be selected to sequence a desired amount of DNA, e.g., by adjusting the affinity and/or amount of the probes as described elsewhere herein. The panel may be further selected to achieve a desired sequence read depth. The panel may be selected to achieve a desired sequence read depth or sequence read coverage for an amount of sequenced base pairs. The panel may be selected to achieve a theoretical sensitivity, a theoretical specificity, and/or a theoretical accuracy for detecting one or more genetic variants in a sample.

Probes for detecting the panel of regions can include those for detecting genomic regions of interest (hotspot regions) as well as nucleosome-aware probes (e.g., KRAS codons 12 and 13) and may be designed to optimize capture based on analysis of cfDNA coverage and fragment size variation impacted by nucleosome binding patterns and GC sequence composition. Regions used herein can also include non-hotspot regions optimized based on nucleosome positions and GC models.

Examples of listings of genomic locations of interest may be found in Table 3 and Table 4. In some embodiments, a sequence-variable target region set used in the methods of the present disclosure comprises at least a portion of at least 5, at least 10, at least 15, at least 20, at least 25, at least 30, at least 35, at least 40, at least 45, at least 50, at least 55, at least 60, at least 65, or 70 of the genes of Table 3. In some embodiments, a sequence-variable target region set used in the methods of the present disclosure comprises at least 5, at least 10, at least 15, at least 20, at least 25, at least 30, at least 35, at least 40, at least 45, at least 50, at least 55, at least 60, at least 65, or 70 of the SNVs of Table 3. In some embodiments, a sequence-variable target region set used in the methods of the present disclosure comprises at least 1, at least 2, at least 3, at least 4, at least 5, or 6 of the fusions of Table 3. In some embodiments, a sequence-variable target region set used in the methods of the present disclosure comprise at least a portion of at least 1, at least 2, or 3 of the indels of Table 3. In some embodiments, a sequence-variable target region set used in the methods of the present disclosure comprises at least a portion of at least 5, at least 10, at least 15, at least 20, at least 25, at least 30, at least 35, at least 40, at least 45, at least 50, at least 55, at least 60, at least 65, at least 70, or 73 of the genes of Table 4. In some embodiments, a sequence-variable target region set used in the methods of the present disclosure comprises at least 5, at least 10, at least 15, at least 20, at least 25, at least 30, at least 35, at least 40, at least 45, at least 50, at least 55, at least 60, at least 65, at least 70, or 73 of the SNVs of Table 4. In some embodiments, a sequence-variable target region set used in the methods of the present disclosure comprises at least 1, at least 2, at least 3, at least 4, at least 5, or 6 of the fusions of Table 4. In some embodiments, a sequence-variable target region set used in the methods of the present disclosure comprises at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, or 18 of the indels of Table 4. Each of these genomic locations of interest may be identified as a backbone region or hot-spot region for a given panel. An example of a listing of hot-spot genomic locations of interest may be found in Table 5. The coordinates in Table 5 are based on the hg19 assembly of the human genome, but one skilled in the art will be familiar with other assemblies and can identify coordinate sets corresponding to the indicated exons, introns, codons, etc. in an assembly of their choice. In some embodiments, a sequence-variable target region set used in the methods of the present disclosure comprises at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 of the genes of Table 5. Each hot-spot genomic region is listed with several characteristics, including the associated gene, chromosome on which it resides, the start and stop position of the genome representing the gene's locus, the length of the gene's locus in base pairs, the exons covered by the gene, and the critical feature (e.g., type of mutation) that a given genomic region of interest may seek to capture.

**Table 3**

| **Point Mutations (SNVs) and Indels** | | | | | | **Fusions** |
|---|---|---|---|---|---|---|
| AKT1 | ALK | APC | AR | ARAF | ARID1A | ALK |
| ATM | BRAF | BRCA1 | BRCA2 | CCND1 | CCND2 | FGFR2 |
| CCNE1 | CDH1 | CDK4 | CDK6 | CDKN2A | CDKN2B | FGFR3 |
| CTNNB1 | EGFR | ERBB2 | ESR1 | EZH2 | FBXW7 | NTRK1 |
| FGFR1 | FGFR2 | FGFR3 | GATA3 | GNA11 | GNAQ | RET |
| GNAS | HNF1A | HRAS | IDH1 | IDH2 | JAK2 | ROS1 |
| JAK3 | KIT | KRAS | MAP2K1 | MAP2K2 | MET | |
| MLH1 | MPL | MYC | NF1 | NFE2L2 | NOTCH1 | |
| NPM1 | NRAS | NTRK1 | PDGFRA | PIK3CA | PTEN | |
| PTPN11 | RAF1 | RB1 | RET | RHEB | RHOA | |
| RIT1 | ROS1 | SMAD4 | SMO | SRC | STK11 | |
| TERT | TP53 | TSC1 | VHL | | | |

**Table 4**

| **Point Mutations (SNVs) and Indels** | | | | | | **Fusions** |
|---|---|---|---|---|---|---|
| AKT1 | ALK | APC | AR | ARAF | ARID1A | ALK |
| ATM | BRAF | BRCA1 | BRCA2 | CCND1 | CCND2 | FGFR2 |
| CCNE1 | CDH1 | CDK4 | CDK6 | CDKN2A | DDR2 | FGFR3 |
| CTNNB1 | EGFR | ERBB2 | ESR1 | EZH2 | FBXW7 | NTRK1 |
| FGFR1 | FGFR2 | FGFR3 | GATA3 | GNA11 | GNAQ | RET |
| GNAS | HNF1A | HRAS | IDH1 | IDH2 | JAK2 | ROS1 |
| JAK3 | KIT | KRAS | MAP2K1 | MAP2K2 | MET | |
| MLH1 | MPL | MYC | NF1 | NFE2L2 | NOTCH1 | |
| NPM1 | NRAS | NTRK1 | PDGFRA | PIK3CA | PTEN | |
| PTPN11 | RAF1 | RB1 | RET | RHEB | RHOA | |
| RIT1 | ROS1 | SMAD4 | SMO | MAPK1 | STK11 | |
| TERT | TP53 | TSC1 | VHL | MAPK3 | MTOR | |
| NTRK3 | | | | | | |

**Table 5**

| Gene | **Chromosome** | **Start Position** | **Stop Position** | **Length (bp)** | **Exons/ Introns Covered** | **Feature** |
|---|---|---|---|---|---|---|
| ALK | chr2 | 29446405 | 29446655 | 250 | intron 19 | Fusion |
| ALK | chr2 | 29446062 | 29446197 | 135 | intron 20 | Fusion |
| ALK | chr2 | 29446198 | 29446404 | 206 | exon 20 | Fusion |
| ALK | chr2 | 29447353 | 29447473 | 120 | intron 19 | Fusion |
| ALK | chr2 | 29447614 | 29448316 | 702 | intron 19 | Fusion |
| ALK | chr2 | 29448317 | 29448441 | 124 | exon 19 | Fusion |
| ALK | chr2 | 29449366 | 29449777 | 411 | intron 18 | Fusion |
| ALK | chr2 | 29449778 | 29449950 | 172 | exon 18 | Fusion |
| BRAF | chr7 | 140453064 | 140453203 | 139 | exon 15 | BRAF V600 |
| CTNNB1 | chr3 | 41266007 | 41266254 | 247 | exon 3 | S37 |
| EGFR | chr7 | 55240528 | 55240827 | 299 | exons 18 and 19 | G719 and deletions |
| EGFR | chr7 | 55241603 | 55241746 | 143 | exon 20 | Insertions/T790M |
| EGFR | chr7 | 55242404 | 55242523 | 119 | exon 21 | L858R |
| ERBB2 | chr17 | 37880952 | 37881174 | 222 | exon 20 | Insertions |
| ESR1 | chr6 | 152419857 | 152420111 | 254 | exon 10 | V534, P535, L536, Y537, D538 |
| FGFR2 | chr 10 | 123279482 | 123279693 | 211 | exon 6 | S252 |
| GATA3 | chr 10 | 8111426 | 8111571 | 145 | exon 5 | SS / Indels |
| GATA3 | chr 10 | 8115692 | 8116002 | 310 | exon 6 | SS / Indels |
| GNAS | chr20 | 57484395 | 57484488 | 93 | exon 8 | R844 |
| IDH1 | chr2 | 209113083 | 209113394 | 311 | exon 4 | R132 |
| IDH2 | chr15 | 90631809 | 90631989 | 180 | exon 4 | R140, R172 |
| KIT | chr4 | 55524171 | 55524258 | 87 | exon 1 | |
| KIT | chr4 | 55561667 | 55561957 | 290 | exon 2 | |
| KIT | chr4 | 55564439 | 55564741 | 302 | exon 3 | |
| KIT | chr4 | 55565785 | 55565942 | 157 | exon 4 | |
| KIT | chr4 | 55569879 | 55570068 | 189 | exon 5 | |
| KIT | chr4 | 55573253 | 55573463 | 210 | exon 6 | |
| KIT | chr4 | 55575579 | 55575719 | 140 | exon 7 | |
| KIT | chr4 | 55589739 | 55589874 | 135 | exon 8 | |
| KIT | chr4 | 55592012 | 55592226 | 214 | exon 9 | |
| KIT | chr4 | 55593373 | 55593718 | 345 | exons 10 and 11 | 557, 559, 560, 576 |
| KIT | chr4 | 55593978 | 55594297 | 319 | exons 12 and 13 | V654 |
| KIT | chr4 | 55595490 | 55595661 | 171 | exon 14 | T670, S709 |
| KIT | chr4 | 55597483 | 55597595 | 112 | exon 15 | D716 |
| KIT | chr4 | 55598026 | 55598174 | 148 | exon 16 | L783 |
| KIT | chr4 | 55599225 | 55599368 | 143 | exon 17 | C809, R815, D816, L818, D820, S821F, N822, Y823 |
| KIT | chr4 | 55602653 | 55602785 | 132 | exon 18 | A829P |
| KIT | chr4 | 55602876 | 55602996 | 120 | exon 19 | |
| KIT | chr4 | 55603330 | 55603456 | 126 | exon 20 | |
| KIT | chr4 | 55604584 | 55604733 | 149 | exon 21 | |
| KRAS | chr12 | 25378537 | 25378717 | 180 | exon 4 | A146 |
| KRAS | chr12 | 25380157 | 25380356 | 199 | exon 3 | Q61 |
| KRAS | chr12 | 25398197 | 25398328 | 131 | exon 2 | G12/G13 |
| MET | chr7 | 116411535 | 116412255 | 720 | exon 13, exon 14, intron 13, intron 14 | MET exon 14 SS |
| NRAS | chr1 | 115256410 | 115256609 | 199 | exon 3 | Q61 |
| NRAS | chr1 | 115258660 | 115258791 | 131 | exon 2 | G12/G13 |
| PIK3CA | chr3 | 178935987 | 178936132 | 145 | exon 10 | E545K |
| PIK3CA | chr3 | 178951871 | 178952162 | 291 | exon 21 | H1047R |
| PTEN | chr10 | 89692759 | 89693018 | 259 | exon 5 | R130 |
| SMAD4 | chr18 | 48604616 | 48604849 | 233 | exon 12 | D537 |
| TERT | chr5 | 1294841 | 1295512 | 671 | promoter | chr5:1295228 |
| TP53 | chr17 | 7573916 | 7574043 | 127 | exon 11 | Q331, R337, R342 |
| TP53 | chr17 | 7577008 | 7577165 | 157 | exon 8 | R273 |
| TP53 | chr17 | 7577488 | 7577618 | 130 | exon 7 | R248 |
| TP53 | chr17 | 7578127 | 7578299 | 172 | exon 6 | R213/Y220 |
| TP53 | chr17 | 7578360 | 7578564 | 204 | exon 5 | R175 / Deletions |
| TP53 | chr17 | 7579301 | 7579600 | 299 | exon 4 | |
| | | | | 12574 (total target region) | | |
| | | | | 16330 (total probe coverage) | | |

Additionally, or alternatively, suitable target region sets are available from the literature. For example, Gale et al., PLoS One 13: e0194630 (2018) describes a panel of 35 cancer-related gene targets that can be used as part or all of a sequence-variable target region set. These 35 targets are AKT1, ALK, BRAF, CCND1, CDK2A, CTNNB1, EGFR, ERBB2, ESR1, FGFR1, FGFR2, FGFR3, FOXL2, GATA3, GNA11, GNAQ, GNAS, HRAS, IDH1, IDH2, KIT, KRAS, MED12, MET, MYC, NFE2L2, NRAS, PDGFRA, PIK3CA, PPP2R1A, PTEN, RET, STK11, TP53, and U2AF 1.

In some embodiments, the sequence-variable target region set comprises target regions from at least 10, 20, 30, or 35 cancer-related genes, such as the cancer-related genes listed above.

### d. Differential capture yield

In some embodiments (e.g., methods involving both sequence-variable target regions and epigenetic target regions), it can be beneficial to capture cfDNA corresponding to the sequence-variable target region set at a greater capture yield than cfDNA corresponding to the epigenetic target region set. This is because a greater depth of sequencing may be necessary to analyze the sequence-variable target regions with sufficient confidence or accuracy than may be necessary to analyze the epigenetic target regions. The greater depth of sequencing can result in more reads per DNA molecule and can be facilitated by capturing more unique molecules per region. The volume of data needed to determine fragmentation patterns (e.g., to test for perturbation of transcription start sites or CTCF binding sites) or fragment abundance (e.g., in hypermethylated and hypomethylated partitions) is generally less than the volume of data needed to determine the presence or absence of cancer-related sequence mutations. Capturing the target region sets at different yields can facilitate sequencing the target regions to different depths of sequencing in the same sequencing run (e.g., using a pooled mixture and/or in the same sequencing cell). By contrast, isolating an epigenetic target region set and a sequence-variable target region set at the same capture yield would lead to the unnecessary generation of redundant data for the epigenetic target region set and/or provide less accuracy than is desirable in determinations of the genotype of the members of sequence-variable target region set.

Thus, in some embodiments, the methods comprise contacting cfDNA obtained from a test subject with a set of target-specific probes, wherein the set of target-specific probes is configured to capture cfDNA corresponding to the sequence-variable target region set at a greater capture yield than cfDNA corresponding to the epigenetic target region set. The capturing step produces a captured set of cfDNA molecules, and the cfDNA molecules corresponding to the sequence-variable target region set are captured at a greater capture yield in the captured set of cfDNA molecules than cfDNA molecules corresponding to the epigenetic target region set. In some embodiments the quantity of captured sequence-variable target region DNA is greater than the quantity of the captured epigenetic target region DNA, when normalized for the difference in the size of the targeted regions (footprint size). In various embodiments, the methods further comprise sequencing the captured cfDNA, e.g., to different degrees of sequencing depth for the epigenetic and sequence-variable target region sets, consistent with the discussion above.

In some embodiments, probes are used to enrich the second aliquot that are configured to provide higher capture yields for the sequence-variable target region set in various ways, including concentration, different lengths and/or chemistries (e.g., that affect affinity), and combinations thereof. In some embodiments, the target-specific probes specific for the sequence-variable target region set have a higher affinity for their targets than the target-specific probes specific for the epigenetic target region set.

Affinity can be modulated in any way known to those skilled in the art, including by using different probe chemistries, such as by adjusting probe length and/or including nucleotide modifications. For example, certain nucleotide modifications, such as cytosine 5-methylation (in certain sequence contexts), modifications that provide a heteroatom at the 2' sugar position, and LNA nucleotides, can increase stability of double-stranded nucleic acids, indicating that oligonucleotides with such modifications have relatively higher affinity for their complementary sequences. See, e.g., Severin etal., Nucleic Acids Res. 39: 8740-8751 (2011); Freier etal., Nucleic Acids Res. 25: 4429-4443 (1997); US Patent No. 9,738,894. Also, longer sequence lengths will generally provide increased affinity. Other nucleotide modifications, such as the substitution of the nucleobase hypoxanthine for guanine, reduce affinity by reducing the amount of hydrogen bonding between the oligonucleotide and its complementary sequence. In some embodiments, the target-specific probes specific for the sequence-variable target region set have modifications that increase their affinity for their targets. In some embodiments, alternatively or additionally, the target-specific probes specific for the epigenetic target region set have modifications that decrease their affinity for their targets. In some embodiments, the target-specific probes specific for the sequence-variable target region set have longer average lengths and/or higher average melting temperatures than the target-specific probes specific for the epigenetic target region set. These embodiments may be combined with each other and/or with differences in concentration as discussed above to achieve a desired fold difference in capture yield, such as any fold difference or range thereof described above. In some embodiments, the capture yield of the target-binding probes specific for the sequence-variable target region set is higher (e.g., at least 2-fold higher) than the capture yield of the target-binding probes specific for the epigenetic target region set. In some embodiments, the capture yield of the target-binding probes specific for the sequence-variable target region set is at least 10-fold higher than the capture yield of the target-binding probes specific for the epigenetic target region set, e.g., 10- to 20-fold higher than the capture yield of the target-binding probes specific for the epigenetic target region set.

In some embodiments, the collection of target-specific probes is configured to have a capture yield specific for the sequence-variable target region set higher (e.g., at least 2-fold higher) than its capture yield specific for the epigenetic target region set. In some embodiments, the collection of target-specific probes is configured to have a capture yield specific for the sequence-variable target region set at least 10-fold higher than its capture yield for the epigenetic target region set, e.g., 10- to 20-fold higher than its capture yield for the epigenetic target region set.

In some embodiments, the concentration of the target-binding probes specific for the sequence-variable target region set is at least 2-fold higher than the concentration of the target-binding probes specific for the epigenetic target region set. In some embodiments, the concentration of the target-binding probes specific for the sequence-variable target region set is at least 10-fold higher than the concentration of the target-binding probes specific for the epigenetic target region set, e.g., 10- to 20-fold higher than the concentration of the target-binding probes specific for the epigenetic target region set. In such embodiments, concentration may refer to the average mass per volume concentration of individual probes in each set.

In some embodiments, the capture yield of the target-binding probes specific for the sequence-variable target region set is at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than the capture yield of the target-binding probes specific for the epigenetic target region set. In some embodiments, the capture yield of the target-binding probes specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10- to 11-, 11- to 12-, 13- to 14-, or 14- to 15-fold higher than the capture yield of the target-binding probes specific for the epigenetic target region set.

In some embodiments, the collection of target-specific probes is configured to have a capture yield specific for the sequence-variable target region set at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than its capture yield for the epigenetic target region set. In some embodiments, the collection of target-specific probes is configured to have a capture yield specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3-to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10- to 11-, 11- to 12-, 13- to 14-, or 14- to 15-fold higher than its capture yield specific for the epigenetic target region set.

In some embodiments, the target-specific probes specific for the sequence-variable target region set are present at a higher concentration than the target-specific probes specific for the epigenetic target region set. In some embodiments, the concentration of the target-binding probes specific for the sequence-variable target region set is at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than the concentration of the target-binding probes specific for the epigenetic target region set. In some embodiments, the concentration of the target-binding probes specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10- to 11-, 11- to 12-, 13- to 14-, or 14- to 15-fold higher than the concentration of the target-binding probes specific for the epigenetic target region set.

In a captured set comprising DNA corresponding to the sequence-variable target region set and the epigenetic target region set, including a combined captured set containing both, the DNA corresponding to the sequence-variable target region set may be present at a greater concentration than the DNA corresponding to the epigenetic target region set, e.g., a 1.1 to 1.2-fold greater concentration, a 1.2- to 1.4-fold greater concentration, a 1.4- to 1.6-fold greater concentration, a 1.6- to 1.8-fold greater concentration, a 1.8- to 2.0-fold greater concentration, a 2.0- to 2.2-fold greater concentration, a 2.2- to 2.4-fold greater concentration a 2.4- to 2.6-fold greater concentration, a 2.6- to 2.8-fold greater concentration, a 2.8- to 3.0-fold greater concentration, a 3.0- to 3.5-fold greater concentration, a 3.5- to 4.0, a 4.0- to 4.5-fold greater concentration, a 4.5-to 5.0-fold greater concentration, a 5.0- to 5.5-fold greater concentration, a 5.5- to 6.0-fold greater concentration, a 6.0- to 6.5-fold greater concentration, a 6.5- to 7.0-fold greater, a 7.0- to 7.5-fold greater concentration, a 7.5- to 8.0-fold greater concentration, an 8.0- to 8.5-fold greater concentration, an 8.5- to 9.0-fold greater concentration, a 9.0- to 9.5-fold greater concentration, 9.5- to 10.0-fold greater concentration, a 10- to 11-fold greater concentration, an 11- to 12-fold greater concentration a 12- to 13-fold greater concentration, a 13- to 14-fold greater concentration, a 14- to 15-fold greater concentration, a 15- to 16-fold greater concentration, a 16- to 17-fold greater concentration, a 17- to 18-fold greater concentration, an 18- to 19-fold greater concentration, or a 19- to 20-fold greater concentration. The degree of difference in concentrations accounts for normalization for the footprint sizes of the target regions, as discussed in the definition section.

In some embodiments, nucleic acids corresponding to the sequence-variable target region set are sequenced to a greater depth of sequencing than nucleic acids corresponding to the epigenetic target region set. For example, the depth of sequencing for nucleic acids corresponding to the sequence variant target region set may be at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold greater, or 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10- to 11-, 11- to 12-, 13-to 14-, 14- to 15-fold, or 15- to 100-fold greater, than the depth of sequencing for nucleic acids corresponding to the epigenetic target region set. In some embodiments, said depth of sequencing is at least 2-fold greater. In some embodiments, said depth of sequencing is at least 5-fold greater. In some embodiments, said depth of sequencing is at least 10-fold greater. In some embodiments, said depth of sequencing is 4- to 10-fold greater. In some embodiments, said depth of sequencing is 4- to 100-fold greater.

In some embodiments, the captured cfDNA corresponding to the sequence-variable target region set and the captured cfDNA corresponding to the epigenetic target region set are sequenced concurrently, e.g., in the same sequencing cell (such as the flow cell of an Illumina sequencer) and/or in the same composition, which may be a pooled composition resulting from recombining separately captured sets or a composition obtained by capturing the cfDNA corresponding to the sequence-variable target region set and the captured cfDNA corresponding to the epigenetic target region set in the same vessel.

### 7. Bait sets; Capture moieties; Enrichment

As discussed above, nucleic acids in a sample can be subject to a capture step, in which molecules having target sequences are captured for subsequent analysis. Target capture can involve use of a bait set comprising oligonucleotide baits (also referred to as probes) labeled with a capture moiety, such as biotin or the other examples noted below. The probes can have sequences selected to tile across a panel of regions, such as genes. In some embodiments, a bait set can have higher and lower capture yields for sets of target regions such as those of the sequence-variable target region set and the epigenetic target region set, respectively, as discussed elsewhere herein. Such bait sets are combined with a sample under conditions that allow hybridization of the target molecules with the baits. Then, captured molecules are isolated using the capture moiety. For example, a biotin capture moiety by bead-based streptavidin. Such methods are further described in, for example, U.S. patent 9,850,523, issuing December 26, 2017.

Capture moieties include, without limitation, biotin, avidin, streptavidin, a nucleic acid comprising a particular nucleotide sequence, a hapten recognized by an antibody, and magnetically attractable particles. The extraction moiety can be a member of a binding pair, such as biotin/streptavidin or hapten/antibody. In some embodiments, a capture moiety that is attached to an analyte is captured by its binding pair which is attached to an isolatable moiety, such as a magnetically attractable particle or a large particle that can be sedimented through centrifugation. The capture moiety can be any type of molecule that allows affinity separation of nucleic acids bearing the capture moiety from nucleic acids lacking the capture moiety. Exemplary capture moieties are biotin which allows affinity separation by binding to streptavidin linked or linkable to a solid phase or an oligonucleotide, which allows affinity separation through binding to a complementary oligonucleotide linked or linkable to a solid phase.

In some embodiments, a collection of target-specific probes is provided, which comprises target-binding probes specific for at least one of target-binding probes specific for a sequence-variable target region set, and target-binding probes specific for an epigenetic target region set. In some embodiments, the collection of target-specific probes comprises both target-binding probes specific for a sequence-variable target region set and target-binding probes specific for an epigenetic target region set.

In some embodiments, the target-specific probes comprise a capture moiety. The capture moiety may be any of the capture moieties described herein, e.g., biotin. In some embodiments, the target-specific probes are linked to a solid support, e.g., covalently or non-covalently such as through the interaction of a binding pair of capture moieties. In some embodiments, the solid support is a bead, such as a magnetic bead.

In some embodiments, the target-specific probes specific for the sequence-variable target region set, and/or the target-specific probes specific for the epigenetic target region set are a bait set as discussed above, e.g., probes comprising capture moieties and sequences selected to tile across a panel of regions, such as genes.

In some embodiments, the target-specific probes are provided in a single composition. The single composition may be a solution (liquid or frozen). Alternatively, it may be a lyophilizate.

Alternatively, the target-specific probes may be provided as a plurality of compositions, e.g., comprising a first composition comprising probes specific for the epigenetic target region set, and (i) a second composition comprising probes specific for one or both of a sequence-variable target region set and an epigenetic target region set or (ii) a second composition comprising probes specific for a sequence-variable target region set and a third composition comprising probes specific for an epigenetic target region set. These probes may be mixed in appropriate proportions, e.g., to provide a combined probe composition with any of the differences in concentration and/or capture yield described elsewhere herein. Alternatively, they may be used in separate capture procedures (e.g., with aliquots of a sample or sequentially with the same sample) to provide first and second compositions, or first, second, and third compositions, comprising the corresponding captured target regions.

### a. Probes specific for epigenetic target regions

In some embodiments (e.g., collections of probes for capturing target regions including epigenetic target regions, and methods involving epigenetic target regions), the probes for the epigenetic target region set may comprise probes specific for one or more types of target regions likely to differentiate DNA from neoplastic (e.g., tumor or cancer) cells from healthy cells, e.g., non-neoplastic circulating cells. Exemplary types of such regions are discussed in detail herein, e.g., in the sections above concerning captured sets. The probes for the epigenetic target region set may also comprise probes for one or more control regions, e.g., as described herein.

In some embodiments, the probes for the epigenetic target region probe set have a footprint of at least 100 kb, e.g., at least 200 kb, at least 300 kb, or at least 400 kb. In some embodiments, the probes for the epigenetic target region set have a footprint in the range of 100-1000 kb, e.g., 100-200 kb, 200-300 kb, 300-400 kb, 400-500 kb, 500-600 kb, 600-700 kb, 700-800 kb, 800-900 kb, and 900-1,000 kb.

### i. Hypermethylation variable target regions

In some embodiments, the probes for the epigenetic target region set comprise probes specific for one or more hypermethylation variable target regions. The hypermethylation variable target regions may be any of those set forth above. For example, in some embodiments, the probes specific for hypermethylation variable target regions comprise probes specific for a plurality of loci listed in Table 1, e.g., at least 10%, 20%, 30%, 40%, 50%, 60%, 70%, 80%, 90%, or 100% of the loci listed in Table 1. In some embodiments, the probes specific for hypermethylation variable target regions comprise probes specific for a plurality of loci listed in Table 2, e.g., at least 10%, 20%, 30%, 40%, 50%, 60%, 70%, 80%, 90%, or 100% of the loci listed in Table 2. In some embodiments, the probes specific for hypermethylation variable target regions comprise probes specific for a plurality of loci listed in Table 1 or Table 2, e.g., at least 10%, 20%, 30%, 40%, 50%, 60%, 70%, 80%, 90%, or 100% of the loci listed in Table 1 or Table 2. In some embodiments, for each locus included as a target region, there may be one or more probes with a hybridization site that binds between the transcription start site and the stop codon (the last stop codon for genes that are alternatively spliced) of the gene. In some embodiments, the one or more probes bind within 300 bp of the listed position, e.g., within 200 or 100 bp. In some embodiments, a probe has a hybridization site overlapping the position listed above. In some embodiments, the probes specific for the hypermethylation target regions include probes specific for one, two, three, four, or five subsets of hypermethylation target regions that collectively show hypermethylation in one, two, three, four, or five of breast, colon, kidney, liver, and lung cancers.

### ii. Hypomethylation variable target regions

In some embodiments, the probes for the epigenetic target region set comprise probes specific for one or more hypomethylation variable target regions. The hypomethylation variable target regions may be any of those set forth above. For example, the probes specific for one or more hypomethylation variable target regions may include probes for regions such as repeated elements, e.g., LINE1 elements, Alu elements, centromeric tandem repeats, pericentromeric tandem repeats, and satellite DNA, and intergenic regions that are ordinarily methylated in healthy cells may show reduced methylation in tumor cells.

In some embodiments, probes specific for hypomethylation variable target regions include probes specific for repeated elements and/or intergenic regions. In some embodiments, probes specific for repeated elements include probes specific for one, two, three, four, or five of LINE1 elements, Alu elements, centromeric tandem repeats, pericentromeric tandem repeats, and/or satellite DNA.

Exemplary probes specific for genomic regions that show cancer-associated hypomethylation include probes specific for nucleotides 8403565-8953708 and/or 151104701-151106035 of human chromosome 1. In some embodiments, the probes specific for hypomethylation variable target regions include probes specific for regions overlapping or comprising nucleotides 8403565-8953708 and/or 151104701-151106035 of human chromosome 1.

### iii. CTCF binding regions

In some embodiments, the probes for the epigenetic target region set include probes specific for CTCF binding regions. In some embodiments, the probes specific for CTCF binding regions comprise probes specific for at least 10, 20, 50, 100, 200, or 500 CTCF binding regions, or 10-20, 20-50, 50-100, 100-200, 200-500, or 500-1000 CTCF binding regions, e.g., such as CTCF binding regions described above or in one or more of CTCFBSDB or the Cuddapah et al., Martin et al., or Rhee et al. articles cited above. In some embodiments, the probes for the epigenetic target region set comprise at least 100 bp, at least 200 bp at least 300 bp, at least 400 bp, at least 500 bp, at least 750 bp, or at least 1000 bp upstream and downstream regions of the CTCF binding sites.

### iv. Transcription start sites

In some embodiments, the probes for the epigenetic target region set include probes specific for transcriptional start sites. In some embodiments, the probes specific for transcriptional start sites comprise probes specific for at least 10, 20, 50, 100, 200, or 500 transcriptional start sites, or 10-20, 20-50, 50-100, 100-200, 200-500, or 500-1000 transcriptional start sites, e.g., such as transcriptional start sites listed in DBTSS. In some embodiments, the probes for the epigenetic target region set comprise probes for sequences at least 100 bp, at least 200 bp, at least 300 bp, at least 400 bp, at least 500 bp, at least 750 bp, or at least 1000 bp upstream and downstream of the transcriptional start sites.

### v. Focal amplifications

As noted above, although focal amplifications are somatic mutations, they can be detected by sequencing based on read frequency in a manner analogous to approaches for detecting certain epigenetic changes such as changes in methylation. As such, regions that may show focal amplifications in cancer can be included in the epigenetic target region set, as discussed above. In some embodiments, the probes specific for the epigenetic target region set include probes specific for focal amplifications. In some embodiments, the probes specific for focal amplifications include probes specific for one or more of AR, BRAF, CCND1, CCND2, CCNE1, CDK4, CDK6, EGFR, ERBB2, FGFR1, FGFR2, KIT, KRAS, MET, MYC, PDGFRA, PIK3CA, and RAF1. For example, in some embodiments, the probes specific for focal amplifications include probes specific for one or more of at least 2, 3, 4, 5, 6, 7, 8, 9, 10, 11, 12, 13, 14, 15, 16, 17, or 18 of the foregoing targets.

### vi. Control regions

It can be useful to include control regions to facilitate data validation. In some embodiments, the probes specific for the epigenetic target region set include probes specific for control methylated regions that are expected to be methylated in essentially all samples. In some embodiments, the probes specific for the epigenetic target region set include probes specific for control hypomethylated regions that are expected to be hypomethylated in essentially all samples.

### b. Probes specific for sequence-variable target regions

In some embodiments (e.g., collections of probes for capturing target regions including sequence-variable target regions, and methods involving sequence-variable target regions), the probes for the sequence-variable target region set may comprise probes specific for a plurality of regions known to undergo somatic mutations in cancer. The probes may be specific for any sequence-variable target region set described herein. Exemplary sequence-variable target region sets are discussed in detail herein, e.g., in the sections above concerning captured sets.

In some embodiments, the sequence-variable target region probe set has a footprint of at least 10 kb, e.g., at least 20 kb, at least 30 kb, or at least 40 kb. In some embodiments, the epigenetic target region probe set has a footprint in the range of 10-100 kb, e.g., 10-20 kb, 20-30 kb, 30-40 kb, 40-50 kb, 50-60 kb, 60-70 kb, 70-80 kb, 80-90 kb, and 90-100 kb.

In some embodiments, probes specific for the sequence-variable target region set comprise probes specific for at least a portion of at least 5, at least 10, at least 15, at least 20, at least 25, at least 30, at least 35, at least 40, at least 45, at least 50, at least 55, at least 60, at least 65, or 70 of the genes of Table 3. In some embodiments, probes specific for the sequence-variable target region set comprise probes specific for the at least 5, at least 10, at least 15, at least 20, at least 25, at least 30, at least 35, at least 40, at least 45, at least 50, at least 55, at least 60, at least 65, or 70 of the SNVs of Table 3. In some embodiments, probes specific for the sequence-variable target region set comprise probes specific for at least 1, at least 2, at least 3, at least 4, at least 5, or 6 of the fusions of Table 3. In some embodiments, probes specific for the sequence-variable target region set comprise probes specific for at least a portion of at least 1, at least 2, or 3 of the indels of Table 3. In some embodiments, probes specific for the sequence-variable target region set comprise probes specific for at least a portion of at least 5, at least 10, at least 15, at least 20, at least 25, at least 30, at least 35, at least 40, at least 45, at least 50, at least 55, at least 60, at least 65, at least 70, or 73 of the genes of Table 4. In some embodiments, probes specific for the sequence-variable target region set comprise probes specific for at least 5, at least 10, at least 15, at least 20, at least 25, at least 30, at least 35, at least 40, at least 45, at least 50, at least 55, at least 60, at least 65, at least 70, or 73 of the SNVs of Table 4. In some embodiments, probes specific for the sequence-variable target region set comprise probes specific for at least 1, at least 2, at least 3, at least 4, at least 5, or 6 of the fusions of Table 4. In some embodiments, probes specific for the sequence-variable target region set comprise probes specific for at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, or 18 of the indels of Table 4. In some embodiments, probes specific for the sequence-variable target region set comprise probes specific for at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 of the genes of Table 5.

In some embodiments, the probes specific for the sequence-variable target region set comprise probes specific for target regions from at least 10, 20, 30, or 35 cancer-related genes, such as AKT1, ALK, BRAF, CCND1, CDK2A, CTNNB1, EGFR, ERBB2, ESR1, FGFR1, FGFR2, FGFR3, FOXL2, GATA3, GNA11, GNAQ, GNAS, HRAS, IDH1, IDH2, KIT, KRAS, MED12, MET, MYC, NFE2L2, NRAS, PDGFRA, PIK3CA, PPP2R1A, PTEN, RET, STK11, TP53, and U2AF1.

### c. Compositions of probes

In some embodiments, a single composition is provided comprising probes for probes for the sequence-variable target region set and probes for the epigenetic target region set. The probes may be provided in such a composition at any concentration ratio described herein.

In some embodiments, a first composition comprising probes for the epigenetic target region set and a second composition comprising probes for the sequence-variable target region set are provided. The ratio of the concentration of the probes in the first composition to the concentration of the probes in the second composition may be any of the ratios described herein.

### 8. Sequencing

Sample nucleic acids, optionally flanked by adapters, with or without prior amplification are generally subjected to sequencing. Sequencing methods or commercially available formats that are optionally utilized include, for example, Sanger sequencing, high-throughput sequencing, pyrosequencing, sequencing-by-synthesis, single-molecule sequencing, nanopore-based sequencing, semiconductor sequencing, sequencing-by-ligation, sequencing-by-hybridization, RNA-Seq (Illumina), Digital Gene Expression (Helicos), next generation sequencing (NGS), Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, sequencing using PacBio, SOLiD, Ion Torrent, or Nanopore platforms. Sequencing reactions can be performed in a variety of sample processing units, which may include multiple lanes, multiple channels, multiple wells, or other means of processing multiple sample sets substantially simultaneously. Sample processing units can also include multiple sample chambers to enable the processing of multiple runs simultaneously.

The sequencing reactions can be performed on one or more nucleic acid fragment types or regions containing markers of cancer or of other diseases. The sequencing reactions can also be performed on any nucleic acid fragment present in the sample. The sequence reactions may be performed on at least about 5%, 10%, 15%, 20%, 25%, 30%, 40%, 50%, 60%, 70%, 80%, 90%, 95%, 99%, 99.9%, or 100% of the genome. In other cases, sequence reactions may be performed on less than about 5%, 10%, 15%, 20%, 25%, 30%, 40%, 50%, 60%, 70%, 80%, 90%, 95%, 99%, 99.9%, or 100% of the genome.

Simultaneous sequencing reactions may be performed using multiplex sequencing techniques. In some embodiments, cell-free polynucleotides are sequenced with at least about 1000, 2000, 3000, 4000, 5000, 6000, 7000, 8000, 9000, 10000, 50000, or 100,000 sequencing reactions. In other embodiments, cell-free polynucleotides are sequenced with less than about 1000, 2000, 3000, 4000, 5000, 6000, 7000, 8000, 9000, 10000, 50000, or 100,000 sequencing reactions. Sequencing reactions are typically performed sequentially or simultaneously. Subsequent data analysis is generally performed on all or part of the sequencing reactions. In some embodiments, data analysis is performed on at least about 1000, 2000, 3000, 4000, 5000, 6000, 7000, 8000, 9000, 10000, 50000, or 100,000 sequencing reactions. In other embodiments, data analysis may be performed on less than about 1000, 2000, 3000, 4000, 5000, 6000, 7000, 8000, 9000, 10000, 50000, or 100,000 sequencing reactions. An example of a read depth is from about 1000 to about 50000 reads per locus (e.g., base position).

### 9. Exemplary workflows

Exemplary workflows for partitioning and library preparation are provided herein. In some embodiments, some or all features of the partitioning and library preparation workflows may be used in combination. The exemplary workflows set forth above may further comprise any compatible feature of methods according to this disclosure set forth elsewhere herein.

In some embodiments, plasma is isolated from a blood sample. In some embodiments, cfDNA is isolated from a sample, such as plasma.

In some embodiments, sample DNA (e.g., cfDNA) is subjected to end repair. In some embodiments, sample DNA (e.g., end-repaired cfDNA) is tagged with molecular barcodes, e.g., via ligation.

Optionally, a partitioning step as described elsewhere herein can be performed before the end repair and/or tagging step. The tags in such embodiments may comprise barcodes that can be used to identify the partition that contained the molecule. The partitioned molecules can be pooled after tagging.

The steps of dividing a sample of tagged DNA molecules prepared from a sample from a subject into at least first and second aliquots; isolating a plurality of tagged DNA molecules from the first aliquot, wherein the plurality of molecules have a common adapter sequence; linearly amplifying at least a portion of the tagged DNA molecules isolated from the first aliquot with a set of primers that target a first plurality of loci of interest and comprise a rearrangement detection barcode, a sequencing adapter, and a non-nucleotide binding partner, thereby producing a first population of processed DNA; and capturing the first population of processed DNA on a solid support using binding to the non-nucleotide binding partner may be performed, e.g., after a tagging step, e.g., using DNA from a partition, from pooled partitions, or from an unpartitioned sample.

In some embodiments, the methods further comprise an amplification step (e.g., PCR). This may occur after tagging, or after capturing the first population of processed DNA on a solid support using binding to the non-nucleotide binding partner.

Regions of interest (e.g., epigenetic target regions and/or sequence-variable target regions as described elsewhere herein) may be captured or enriched after an amplification step. A further amplification step may be performed after capture or enrichment.

The foregoing steps can be followed by sequencing, e.g., sample pooling and multiplex sequencing.

### 10. Analysis

Sequencing may generate a plurality of sequence reads or reads. Sequence reads or reads may include sequences of nucleotide data less than about 150 bases in length, or less than about 90 bases in length. In some embodiments, reads are between about 80 bases and about 90 bases, e.g., about 85 bases in length. In some embodiments, methods of the present disclosure are applied to very short reads, e.g., less than about 50 bases or about 30 bases in length. Sequence read data can include the sequence data as well as meta information. Sequence read data can be stored in any suitable file format including, for example, VCF files, FASTA files, or FASTQ files.

FASTA may refer to a computer program for searching sequence databases, and the name FASTA may also refer to a standard file format. FASTA is described by, for example, Pearson & Lipman, 1988, Improved tools for biological sequence comparison, PNAS 85:2444-2448. A sequence in FASTA format begins with a single-line description, followed by lines of sequence data. The description line is distinguished from the sequence data by a greater-than (">") symbol in the first column. The word following the ">" symbol is the identifier of the sequence, and the rest of the line is the description (both are optional). There may be no space between the ">" and the first letter of the identifier. It is recommended that all lines of text be shorter than 80 characters. The sequence ends if another line starting with a ">" appears; this indicates the start of another sequence.

The FASTQ format is a text-based format for storing both a biological sequence (usually nucleotide sequence) and its corresponding quality scores. It is similar to the FASTA format but with quality scores following the sequence data. Both the sequence letter and quality score are encoded with a single ASCII character for brevity. The FASTQ format is a de facto standard for storing the output of high throughput sequencing instruments such as the Illumina Genome Analyzer, as described by, for example, Cock et al. ("The Sanger FASTQ file format for sequences with quality scores, and the Solexa/Illumina FASTQ variants," Nucleic Acids Res 38(6):1767-1771, 2009).

For FASTA and FASTQ files, meta information includes the description line and not the lines of sequence data. In some embodiments, for FASTQ files, the meta information includes the quality scores. For FASTA and FASTQ files, the sequence data begins after the description line and is present typically using some subset of IUPAC ambiguity codes optionally with "-". In an embodiment, the sequence data may use the A, T, C, G, and N characters, optionally including "-" or U as-needed (e.g., to represent gaps or uracil).

In some embodiments, the at least one master sequence read file and the output file are stored as plain text files (e.g., using encoding such as ASCII; ISO/IEC 646; EBCDIC; UTF-8; or UTF-16). A computer system provided by the present disclosure may include a text editor program capable of opening the plain text files. A text editor program may refer to a computer program capable of presenting contents of a text file (such as a plain text file) on a computer screen, allowing a human to edit the text (e.g., using a monitor, keyboard, and mouse). Examples of text editors include, without limitation, Microsoft Word, emacs, pico, vi, BBEdit, and TextWrangler. The text editor program may be capable of displaying the plain text files on a computer screen, showing the meta information and the sequence reads in a human-readable format (e.g., not binary encoded but instead using alphanumeric characters as they may be used in print or human writing).

While methods have been discussed with reference to FASTA or FASTQ files, methods and systems of the present disclosure may be used to compress any suitable sequence file format including, for example, files in the Variant Call Format (VCF) format. A typical VCF file may include a header section and a data section. The header contains an arbitrary number of metainformation lines, each starting with characters `##', and a TAB delimited field definition line starting with a single '#' character. The field definition line names eight mandatory columns and the body section contains lines of data populating the columns defined by the field definition line. The VCF format is described by, for example, Danecek et al. ("The variant call format and VCF tools," Bioinformatics 27(15):2156-2158, 2011). The header section may be treated as the meta information to write to the compressed files and the data section may be treated as the lines, each of which can be stored in a master file only if unique.

Some embodiments provide for the assembly of sequence reads. In assembly by alignment, for example, the sequence reads are aligned to each other or aligned to a reference sequence. By aligning each read, in turn to a reference genome, all of the reads are positioned in relationship to each other to create the assembly. In addition, aligning or mapping the sequence read to a reference sequence can also be used to identify variant sequences within the sequence read. Identifying variant sequences can be used in combination with the methods and systems described herein to further aid in the diagnosis or prognosis of a disease or condition, or for guiding treatment decisions.

In some embodiments, any or all of the steps are automated. Alternatively, methods of the present disclosure may be embodied wholly or partially in one or more dedicated programs, for example, each optionally written in a compiled language such as C++, then compiled and distributed as a binary. Methods of the present disclosure may be implemented wholly or in part as modules within, or by invoking functionality within, existing sequence analysis platforms. In some embodiments, methods of the present disclosure include a number of steps that are all invoked automatically responsive to a single starting queue (e.g., one or a combination of triggering events sourced from human activity, another computer program, or a machine). Thus, the present disclosure provides methods in which any or the steps or any combination of the steps can occur automatically responsive to a queue. "Automatically" generally means without intervening human input, influence, or interaction (e.g., responsive only to original or pre-queue human activity).

The methods of the present disclosure may also encompass various forms of output, which includes an accurate and sensitive interpretation of a subject's nucleic acid sample. The output of retrieval can be provided in the format of a computer file. In some embodiments, the output is a FASTA file, a FASTQ file, or a VCF file. The output may be processed to produce a text file, or an XML file containing sequence data such as a sequence of the nucleic acid aligned to a sequence of the reference genome. In other embodiments, processing yields output containing coordinates or a string describing one or more mutations in the subject nucleic acid relative to the reference genome. Alignment strings may include Simple UnGapped Alignment Report (SUGAR), Verbose Useful Labeled Gapped Alignment Report (VULGAR), and Compact Idiosyncratic Gapped Alignment Report (CIGAR) (as described by, for example, Ning et al., Genome Research 11(10): 1725-9, 2001). These strings may be implemented, for example, in the Exonerate sequence alignment software from the European Bioinformatics Institute (Hinxton, UK).

In some embodiments, a sequence alignment is produced-such as, for example, a sequence alignment map (SAM) or binary alignment map (BAM) file-comprising a CIGAR string (the SAM format is described, e.g., by Li et al., "The Sequence Alignment/Map format and SAMtools," Bioinformatics, 25(16):2078-9, 2009). In some embodiments, CIGAR displays or includes gapped alignments one-per-line. CIGAR is a compressed pairwise alignment format reported as a CIGAR string. A CIGAR string may be useful for representing long (e.g., genomic) pairwise alignments. A CIGAR string may be used in SAM format to represent alignments of reads to a reference genome sequence.

A CIGAR string may follow an established motif. Each character is preceded by a number, giving the base counts of the event. Characters used can include M, I, D, N, and S (M=match; I=insertion; D=deletion; N=gap; S=substitution). The CIGAR string defines the sequence of matches and/or mismatches and deletions (or gaps). For example, the CIGAR string 2MD3M2D2M may indicate that the alignment contains 2 matches, 1 deletion (number 1 is omitted in order to save some space), 3 matches, 2 deletions, and 2 matches.

In some embodiments, a nucleic acid population is prepared for sequencing by enzymatically forming blunt-ends on double-stranded nucleic acids with single-stranded overhangs at one or both ends. In these embodiments, the population is typically treated with an enzyme having a 5'-3' DNA polymerase activity and a 3'-5' exonuclease activity in the presence of the nucleotides (e.g., A, C, G, and T or U). Examples of enzymes or catalytic fragments thereof that may be optionally used include Klenow large fragment and T4 polymerase. At 5' overhangs, the enzyme typically extends the recessed 3' end on the opposing strand until it is flush with the 5' end to produce a blunt end. At 3' overhangs, the enzyme generally digests from the 3' end up to and sometimes beyond the 5' end of the opposing strand. If this digestion proceeds beyond the 5' end of the opposing strand, the gap can be filled in by an enzyme having the same polymerase activity that is used for 5' overhangs. The formation of blunt ends on double-stranded nucleic acids facilitates, for example, the attachment of adapters and subsequent amplification.

In some embodiments, nucleic acid populations are subjected to additional processing, such as the conversion of single-stranded nucleic acids to double-stranded nucleic acids and/or conversion of RNA to DNA (e.g., complementary DNA or cDNA). These forms of nucleic acid are also optionally linked to adapters and amplified.

With or without prior amplification, nucleic acids subject to the process of forming blunt-ends described above, and optionally other nucleic acids in a sample, can be sequenced to produce sequenced nucleic acids. A sequenced nucleic acid can refer either to the sequence of a nucleic acid (e.g., sequence information) or a nucleic acid whose sequence has been determined. Sequencing can be performed so as to provide sequence data of individual nucleic acid molecules in a sample either directly or indirectly from a consensus sequence of amplification products of an individual nucleic acid molecule in the sample.

In some embodiments, double-stranded nucleic acids with single-stranded overhangs in a sample after blunt-end formation are linked at both ends to adapters including barcodes, and the sequencing determines nucleic acid sequences as well as in-line barcodes introduced by the adapters. The blunt-end DNA molecules are optionally ligated to a blunt end of an at least partially double-stranded adapter (e.g., a Y-shaped or bell-shaped adapter). Alternatively, blunt ends of sample nucleic acids and adapters can be tailed with complementary nucleotides to facilitate ligation (for e.g., sticky-end ligation).

The nucleic acid sample is typically contacted with a sufficient number of adapters that there is a low probability (e.g., less than about 1 or 0.1 %) that any two copies of the same nucleic acid receive the same combination of adapter barcodes from the adapters linked at both ends. The use of adapters in this manner may permit identification of families of nucleic acid sequences with the same start and stop points on a reference nucleic acid and linked to the same combination of barcodes. Such a family may represent sequences of amplification products of a nucleic acid in the sample before amplification. The sequences of family members can be compiled to derive consensus nucleotide(s) or a complete consensus sequence for a nucleic acid molecule in the original sample, as modified by blunt-end formation and adapter attachment. In other words, the nucleotide occupying a specified position of a nucleic acid in the sample can be determined to be the consensus of nucleotides occupying that corresponding position in family member sequences. Families can include sequences of one or both strands of a double-stranded nucleic acid. If members of a family include sequences of both strands from a double-stranded nucleic acid, sequences of one strand may be converted to their complements for purposes of compiling sequences to derive consensus nucleotide(s) or sequences. Some families include only a single member sequence. In this case, this sequence can be taken as the sequence of a nucleic acid in the sample before amplification. Alternatively, families with only a single member sequence can be eliminated from subsequent analysis.

Nucleotide variations (e.g., SNVs or rearrangements, such as indels) in sequenced nucleic acids can be determined by comparing sequenced nucleic acids with a reference sequence. The reference sequence is often a known sequence, e.g., a known whole or partial genome sequence from a subject (e.g., a whole genome sequence of a human subject). The reference sequence can be, for example, hG19 or hG38. The sequenced nucleic acids can represent sequences determined directly for a nucleic acid in a sample, or a consensus of sequences of amplification products of such a nucleic acid, as described above. A comparison can be performed at one or more designated positions on a reference sequence. A subset of sequenced nucleic acids can be identified including a position corresponding with a designated position of the reference sequence when the respective sequences are maximally aligned. Within such a subset it can be determined which, if any, sequenced nucleic acids include a nucleotide variation at the designated position, and optionally which if any, include a reference nucleotide (e.g., same as in the reference sequence). If the number of sequenced nucleic acids in the subset including a nucleotide variant exceeding a selected threshold, then a variant nucleotide can be called at the designated position. The threshold can be a simple number, such as at least 1, 2, 3, 4, 5, 6, 7, 8, 9, or 10 sequenced nucleic acids within the subset including the nucleotide variant or it can be a ratio, such as at least 0.5, 1, 2, 3, 4, 5, 10, 15, or 20, of sequenced nucleic acids within the subset that include the nucleotide variant, among other possibilities. The comparison can be repeated for any designated position of interest in the reference sequence. Sometimes a comparison can be performed for designated positions occupying at least about 20, 100, 200, or 300 contiguous positions on a reference sequence, e.g., about 20-500, or about 50-300 contiguous positions.

Certain types of rearrangements, such as translocations, duplications, insertions, and deletions, juxtapose sequences that are not normally juxtaposed and can be identified based on the presence of a sequence corresponding to a first genomic location in proximity (e.g., adjacent) to a sequence corresponding to a second genomic location that is not proximal (e.g., adjacent) to the first genomic location in the reference sequence. In some embodiments, a sequence in which a rearrangement is detected comprises at least about 20, 30, 40, or 50 nucleotides from each of the sequence corresponding to the first genomic location and the sequence corresponding to a second genomic location. A sequence corresponding to a genomic location may match the reference sequence at the genomic location with an identity of, e.g., at least about 90%, 95%, 96%, 97%, 98%, or 99%. In some embodiments, the sequence corresponding to the first genomic location contains at least 3, 4, 5, 6, 7, 8, 9, or 10 mismatches and/or gaps when optimally aligned to a portion of the reference sequence comprising the second genomic location. In some embodiments, the sequence corresponding to the second genomic location contains at least 3, 4, 5, 6, 7, 8, 9, or 10 mismatches and/or gaps when optimally aligned to a portion of the reference sequence comprising the first genomic location.

Additional details regarding nucleic acid sequencing, including the formats and applications described herein, are also provided in, for example, Levy et al., Annual Review of Genomics and Human Genetics, 17: 95-115 (2016), Liu et al., J. of Biomedicine and Biotechnology, Volume 2012, Article ID 251364:1-11 (2012), Voelkerding et al., Clinical Chem., 55: 641-658 (2009), MacLean et al., Nature Rev. Microbiol., 7: 287-296 (2009), Astier et al., J Am Chem Soc., 128(5):1705-10 (2006), U.S. Pat. No. 6,210,891, U.S. Pat. No. 6,258,568, U.S. Pat. No. 6,833,246, U.S. Pat. No. 7,115,400, U.S. Pat. No. 6,969,488, U.S. Pat. No. 5,912,148, U.S. Pat. No. 6,130,073, U.S. Pat. No. 7,169,560, U.S. Pat. No. 7,282,337, U.S. Pat. No. 7,482,120, U.S. Pat. No. 7,501,245, U.S. Pat. No. 6,818,395, U.S. Pat. No. 6,911,345, U.S. Pat. No. 7,501,245, U.S. Pat. No. 7,329,492, U.S. Pat. No. 7,170,050, U.S. Pat. No. 7,302,146, U.S. Pat. No. 7,313,308, and U.S. Pat. No. 7,476,503.

### IV. COMPUTER SYSTEMS

Methods of the present disclosure can be implemented using, or with the aid of, computer systems. FIG. 2 shows a computer system 201 that is programmed or otherwise configured to implement the methods of the present disclosure. The computer system 201 can regulate various aspects sample preparation, sequencing, and/or analysis. In some examples, the computer system 201 is configured to perform sample preparation and sample analysis, including (where applicable) nucleic acid sequencing, e.g., according to any of the methods disclosed herein.

The computer system 201 includes a central processing unit (CPU, also "processor" and "computer processor" herein) 205, which can be a single core or multi core processor, or a plurality of processors for parallel processing. The computer system 201 also includes memory or memory location 210 (e.g., random-access memory, read-only memory, flash memory), electronic storage unit 215 (e.g., hard disk), communication interface 220 (e.g., network adapter) for communicating with one or more other systems, and peripheral devices 225, such as cache, other memory, data storage, and/or electronic display adapters. The memory 210, storage unit 215, interface 220, and peripheral devices 225 are in communication with the CPU 205 through a communication network or bus (solid lines), such as a motherboard. The storage unit 215 can be a data storage unit (or data repository) for storing data. The computer system 201 can be operatively coupled to a computer network 230 with the aid of the communication interface 220. The computer network 230 can be the Internet, an internet and/or extranet, or an intranet and/or extranet that is in communication with the Internet. The computer network 230 in some cases is a telecommunication and/or data network. The computer network 230 can include one or more computer servers, which can enable distributed computing, such as cloud computing. The computer network 230, in some cases with the aid of the computer system 201, can implement a peer-to-peer network, which may enable devices coupled to the computer system 201 to behave as a client or a server.

The CPU 205 can execute a sequence of machine-readable instructions, which can be embodied in a program or software. The instructions may be stored in a memory location, such as the memory 210. Examples of operations performed by the CPU 205 can include fetch, decode, execute, and writeback.

The storage unit 215 can store files, such as drivers, libraries, and saved programs. The storage unit 215 can store programs generated by users and recorded sessions, as well as output(s) associated with the programs. The storage unit 215 can store user data, e.g., user preferences and user programs. The computer system 201 in some cases can include one or more additional data storage units that are external to the computer system 201, such as located on a remote server that is in communication with the computer system 201 through an intranet or the Internet. Data may be transferred from one location to another using, for example, a communication network or physical data transfer (e.g., using a hard drive, thumb drive, or other data storage mechanism).

The computer system 201 can communicate with one or more remote computer systems through the network 230. For embodiment, the computer system 201 can communicate with a remote computer system of a user (e.g., operator). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 201 via the network 230.

Methods as described herein can be implemented by way of machine (e.g., computer processor) executable code stored on an electronic storage location of the computer system 201, such as, for example, on the memory 210 or electronic storage unit 215. The machine executable or machine-readable code can be provided in the form of software. During use, the code can be executed by the processor 205. In some cases, the code can be retrieved from the storage unit 215 and stored on the memory 210 for ready access by the processor 205. In some situations, the electronic storage unit 215 can be precluded, and machine-executable instructions are stored on memory 210.

In an aspect, the present disclosure provides a non-transitory computer-readable medium comprising computer-executable instructions which, when executed by at least one electronic processor, perform at least a portion of a method described herein. For example, such methods may comprise: collecting cfDNA from a test subject; capturing a plurality of sets of target regions from the cfDNA, wherein the plurality of target region sets comprises one or both of a sequence-variable target region set and an epigenetic target region set, whereby a captured set of cfDNA molecules is produced; sequencing the captured cfDNA molecules; obtaining a plurality of sequence reads generated by a nucleic acid sequencer from sequencing the captured cfDNA molecules; mapping the plurality of sequence reads to one or more reference sequences to generate mapped sequence reads; and processing the mapped sequence reads corresponding to the sequence-variable target region set and to the epigenetic target region set to determine the likelihood that the subject has cancer.

In an aspect, the present disclosure provides a non-transitory computer-readable medium comprising computer-executable instructions which, when executed by at least one electronic processor, perform at least a portion of a method comprising: collecting cfDNA from a test subject; capturing a plurality of sets of target regions from the cfDNA, wherein the plurality of target region sets comprises one or both of a sequence-variable target region set and an epigenetic target region set, whereby a captured set of cfDNA molecules is produced; sequencing the captured cfDNA molecules; obtaining a plurality of sequence reads generated by a nucleic acid sequencer from sequencing the captured cfDNA molecules; mapping the plurality of sequence reads to one or more reference sequences to generate mapped sequence reads; and processing the mapped sequence reads corresponding to the sequence-variable target region set and to the epigenetic target region set to determine the likelihood that the subject has cancer.

The code can be pre-compiled and configured for use with a machine have a processer adapted to execute the code or can be compiled during runtime. The code can be supplied in a programming language that can be selected to enable the code to execute in a pre-compiled or ascompiled fashion.

Aspects of the systems and methods provided herein, such as the computer system 201, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming.

All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and electromagnetic waves, such as those used across physical interfaces between local devices, through wired and optical landline networks, and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

Hence, a machine-readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards, paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 201 can include or be in communication with an electronic display that comprises a user interface (UI) for providing, for example, one or more results of sample analysis. Examples of UIs include, without limitation, a graphical user interface (GUI) and webbased user interface.

Additional details relating to computer systems and networks, databases, and computer program products are also provided in, for example, Peterson, Computer Networks: A Systems Approach, Morgan Kaufmann, 5th Ed. (2011), Kurose, Computer Networking: A Top-Down Approach, Pearson, 7th Ed. (2016), Elmasri, Fundamentals of Database Systems, Addison Wesley, 6th Ed. (2010), Coronel, Database Systems: Design, Implementation, & Management, Cengage Learning, 11th Ed. (2014), Tucker, Programming Languages, McGraw-Hill Science/Engineering/Math, 2nd Ed. (2006), and Rhoton, Cloud Computing Architected: Solution Design Handbook, Recursive Press (2011).

### V. APPLICATIONS

### 1. Cancer and Other Diseases

The present methods can be used to diagnose presence of conditions, particularly cancer, in a subject, to characterize conditions (e.g., staging cancer or determining heterogeneity of a cancer), monitor response to treatment of a condition, effect prognosis risk of developing a condition or subsequent course of a condition. The present disclosure can also be useful in determining the efficacy of a particular treatment option. Successful treatment options may increase the amount of copy number variation or rare mutations detected in subject's blood if the treatment is successful as more cancers may die and shed DNA. In other examples, this may not occur. In another example, certain treatment options may be correlated with genetic profiles of cancers over time, e.g., including the presence or level of one or more genomic rearrangements such as gene fusions. This correlation may be useful in selecting a therapy.

Additionally, if a cancer is observed to be in remission after treatment, the present methods can be used to monitor residual disease or recurrence of disease.

In some embodiments, the methods and systems disclosed herein may be used to identify customized or targeted therapies to treat a given disease or condition. For example, the presence of a particular type of genomic rearrangement (e.g., gene fusion) may indicate that the disease or condition is more likely to respond to a certain treatment or class of treatments. Typically, the disease under consideration is a type of cancer. Non-limiting examples of such cancers include biliary tract cancer, bladder cancer, transitional cell carcinoma, urothelial carcinoma, brain cancer, gliomas, astrocytomas, breast carcinoma, metaplastic carcinoma, cervical cancer, cervical squamous cell carcinoma, rectal cancer, colorectal carcinoma, colon cancer, hereditary nonpolyposis colorectal cancer, colorectal adenocarcinomas, gastrointestinal stromal tumors (GISTs), endometrial carcinoma, endometrial stromal sarcomas, esophageal cancer, esophageal squamous cell carcinoma, esophageal adenocarcinoma, ocular melanoma, uveal melanoma, gallbladder carcinomas, gallbladder adenocarcinoma, renal cell carcinoma, clear cell renal cell carcinoma, transitional cell carcinoma, urothelial carcinomas, Wilms tumor, leukemia, acute lymphocytic leukemia (ALL), acute myeloid leukemia (AML), chronic lymphocytic leukemia (CLL), chronic myeloid leukemia (CML), chronic myelomonocytic leukemia (CMML), liver cancer, liver carcinoma, hepatoma, hepatocellular carcinoma, cholangiocarcinoma, hepatoblastoma, Lung cancer, non-small cell lung cancer (NSCLC), mesothelioma, B-cell lymphomas, non-Hodgkin lymphoma, diffuse large B-cell lymphoma, Mantle cell lymphoma, T cell lymphomas, non-Hodgkin lymphoma, precursor T-lymphoblastic lymphoma/leukemia, peripheral T cell lymphomas, multiple myeloma, nasopharyngeal carcinoma (NPC), neuroblastoma, oropharyngeal cancer, oral cavity squamous cell carcinomas, osteosarcoma, ovarian carcinoma, pancreatic cancer, pancreatic ductal adenocarcinoma, pseudopapillary neoplasms, acinar cell carcinomas. Prostate cancer, prostate adenocarcinoma, skin cancer, melanoma, malignant melanoma, cutaneous melanoma, small intestine carcinomas, stomach cancer, gastric carcinoma, gastrointestinal stromal tumor (GIST), uterine cancer, or uterine sarcoma. Type and/or stage of cancer can be detected from genetic variations including mutations, rare mutations, indels, copy number variations, transversions, translocations, inversion, deletions, aneuploidy, partial aneuploidy, polyploidy, chromosomal instability, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, and abnormal changes in nucleic acid 5-methylcytosine.

For example, regarding treatment selection, fusions involving TMPRSS2, RET, FGFR3, or ALK may be "druggable" with targeted therapies. See, e.g., Gao et al., Cell Rep. 2018 April 03; 23(1): 227-238.e3. As specific examples, prostate cancers with a TMPRSS2 fusion may be responsive to androgen deprivation therapy; cancers with a RET fusion may be responsive to a kinase inhibitor such as selpercatinib; cancers with FGFR fusions may be responsive to an FGFR inhibitor such as BGJ398 or JNJ-42756493; and cancers with ALK fusions may be responsive to an ALK inhibitor such as crizotinib. See, e.g., Costa et al., Oncotarget 2016 July 7; 7(34): 55924-38; Graff et al., Prostate. 2015 June 15; 75(9): 897-906; Arbour et al., Hematol Oncol Clin North Am. 2017 February, 31(1): 101-111; Cabanillas et al., J. Endocrine Soc. 2020; 4(Abstract Suppl.):A1121. Additionally, as described in Sun et al., Bioinformatics, 34(24), 2018, 4315-4317, a database of curated genomic variants found in cancers is available that have clinically supported drug therapies.

In some embodiments, the subject is in need of a new or different treatment for the cancer. A treatment is new if the subject has not previously received it. In some embodiments, the subject has received a treatment for the cancer. A treatment is different if the subject has previously received a treatment and needs a new treatment other than what has been previously administered. Disease detection, diagnosis, or progression, and/or failure to respond adequately to a previous treatment, are examples of reasons that a subject may need a new or different treatment. In some embodiments, a previous treatment did not result in a complete response, i.e., a detectable level of cancer remained following the previous treatment. In some embodiments, the previous treatment did not result in remission. In some embodiments, wherein the cancer is a solid tumor and the treatment did not result in arrested growth of the tumor. For example, the tumor may show a nonzero increase in any appropriate measure of size at a time after administration of the treatment relative to before the treatment. In some embodiments, the cancer is a hematological cancer and the treatment did not result in a reduction of circulating cancer cells, or did not result in a reduction of cancer cells in bone marrow. In some embodiments, the subject experienced a relapse subsequent to the treatment (e.g., where a treatment initially resulted in remission, but the cancer subsequently regrew, reappeared, or became detectable again).

In some embodiments, at least one genomic rearrangement of the cancer is detected, e.g., wherein the genomic rearrangement is a gene fusion, such as an oncogenic gene fusion. In some embodiments, the genomic rearrangement or gene fusion is indicative of an increased likelihood of efficacy for a new or different treatment. Exemplary rearrangements that can be indicative of an increased likelihood of efficacy for certain treatments are discussed above. In some embodiments, the subject receives the new or different treatment after detection of the genomic rearrangement or gene fusion.

Genetic data can also be used for characterizing a specific form of cancer. Cancers are often heterogeneous in both composition and staging. Genetic profile data may allow characterization of specific sub-types of cancer that may be important in the diagnosis or treatment of that specific sub-type. This information may also provide a subject or practitioner clues regarding the prognosis of a specific type of cancer and allow either a subject or practitioner to adapt treatment options in accord with the progress of the disease. Some cancers can progress to become more aggressive and genetically unstable. Other cancers may remain benign, inactive or dormant. The system and methods of this disclosure may be useful in determining disease progression.

Further, the methods of the disclosure may be used to characterize the heterogeneity of an abnormal condition in a subject. Such methods can include, e.g., generating a genetic profile of extracellular polynucleotides derived from the subject, wherein the genetic profile comprises a plurality of data resulting from copy number variation and rare mutation analyses. In some embodiments, an abnormal condition is cancer. In some embodiments, the abnormal condition may be one resulting in a heterogeneous genomic population. In the example of cancer, some tumors are known to comprise tumor cells in different stages of the cancer. In other examples, heterogeneity may comprise multiple foci of disease. Again, in the example of cancer, there may be multiple tumor foci, perhaps where one or more foci are the result of metastases that have spread from a primary site.

The present methods can be used to generate or profile, fingerprint or set of data that is a summation of genetic information derived from different cells in a heterogeneous disease. This set of data may comprise copy number variation, epigenetic variation, and mutation analyses alone or in combination.

The present methods can be used to diagnose, prognose, monitor or observe cancers, or other diseases. In some embodiments, the methods herein do not involve the diagnosing, prognosing or monitoring a fetus and as such are not directed to non-invasive prenatal testing. In other embodiments, these methodologies may be employed in a pregnant subject to diagnose, prognose, monitor or observe cancers or other diseases in an unborn subject whose DNA and other polynucleotides may co-circulate with maternal molecules.

### 2. Therapies and Related Administration

In certain embodiments, the methods disclosed herein relate to identifying and administering customized therapies to patients given the status of a nucleic acid variant as being of somatic or germline origin. In some embodiments, essentially any cancer therapy (e.g., surgical therapy, radiation therapy, chemotherapy, and/or the like) may be included as part of these methods. In some embodiments, customized therapies include at least one immunotherapy (or an immunotherapeutic agent). Immunotherapy refers generally to methods of enhancing an immune response against a given cancer type. In certain embodiments, immunotherapy refers to methods of enhancing a T cell response against a tumor or cancer.

In certain embodiments, the status of a nucleic acid variant from a sample from a subject as being of somatic or germline origin may be compared with a database of comparator results from a reference population to identify customized or targeted therapies for that subject. In some embodiments, the reference population includes patients with the same cancer or disease type as the test subject and/or patients who are receiving, or who have received, the same therapy as the test subject. A customized or targeted therapy (or therapies) may be identified when the nucleic variant and the comparator results satisfy certain classification criteria (e.g., are a substantial or an approximate match).

In certain embodiments, the customized therapies described herein are typically administered parenterally (e.g., intravenously or subcutaneously). Pharmaceutical compositions containing an immunotherapeutic agent are typically administered intravenously. Certain therapeutic agents are administered orally. However, customized therapies (e.g., immunotherapeutic agents, etc.) may also be administered by methods such as, for example, buccal, sublingual, rectal, vaginal, intraurethral, topical, intraocular, intranasal, and/or intraauricular, which administration may include tablets, capsules, granules, aqueous suspensions, gels, sprays, suppositories, salves, ointments, or the like.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the disclosure described herein may be employed in practicing the invention. It is therefore contemplated that the disclosure shall also cover any such alternatives, modifications or variations. It is intended that the following claims define the scope of the invention and that methods and structures within the scope of these claims be covered thereby.

While the foregoing disclosure has been described in some detail by way of illustration and example for purposes of clarity and understanding, it will be clear to one of ordinary skill in the art from a reading of this disclosure that various changes in form and detail can be made without departing from the true scope of the disclosure and may be practiced within the scope of the appended claims. For example, all the methods, systems, computer readable media, and/or component features, steps, elements, or other aspects thereof can be used in various combinations.

If different versions of a sequence are associated with an accession number at different times, the version associated with the accession number at the effective filing date of this application is meant. The effective filing date means the earlier of the actual filing date or filing date of a priority application referring to the accession number, if applicable. Likewise, if different versions of a publication, website or the like are published at different times, the version most recently published at the effective filing date of the application is meant, unless otherwise indicated.

### VI. EXAMPLES

### I) DETECTION OF CANCER USING METHOD OF DETECTION GENOMIC REARRANGEMENT ALONE OR IN COMBINATION WITH EPIGENETIC AND SEQUENCE-VARIABLE TARGET REGION SETS

Cohorts of cell-free nucleic acid samples from cancer patients, such as colorectal cancer (CRC) patients with different stages of cancer, e.g., from I to IVA may be analyzed in accordance with the methods of the invention.

### 1. Detection of genomic rearrangement

A DNA (e.g., cfDNA) sample from a subject having a cancer (e.g., extracted from a liquid biopsy) is processed through standard library preparation (e.g., end repair, adapter ligation at both ends) and amplification through PCR. The resulting sample of tagged cfDNA is divided into first and second aliquots. The first aliquot is taken through a fusion detection workflow, as described herein, while the second aliquot undergoes enrichment for loci of interest, e.g., for detection of variants such as point mutations. A plurality of tagged cfDNA molecules from the first aliquot is isolated, such that the plurality of molecules have a common first adapter sequence, such as a P7 adapter sequence. Specifically, molecules containing the first adapter at their 5' end are captured in the presence of blockers that prevent capture of molecules containing a second adapter (e.g., a P5 sequence) at their 5' end to select a molecule population where substantially all molecule 5' ends have the first adapter sequence, and all 3' ends have the complement of the second adapter sequence. See FIG. 1A. Next, at least a portion of said cfDNA molecules from the first aliquot is linearly amplified with a set of primers that target a first plurality of loci of interest tiling regions known to be at or near gene fusion breakpoints. See FIG. 1(b). The primers of this set comprise, 5' to the target-binding portion, a rearrangement detection barcode for identification of products as being for rearrangement detection, the second sequencing adapter (e.g., a P5 sequence), and a biotin moiety. The plurality of loci of interest may include any or all of ALK, EML4, BCR, ABL1, MYC, an immunoglobulin gene, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ, or TFE3. The products of the linear amplification constitute a first population of processed DNA.

As described above, the second aliquot of cfDNA may undergo standard enrichment. Specifically, the second aliquot is enriched for a second plurality of loci of interest, thereby producing a second population of processed DNA. The first population of processed DNA is reintroduced to the second aliquot of cfDNA at the stage of post-enrichment washes. During post-enrichment washes, the first population of processed DNA is captured on a solid support, such as streptavidin beads, using binding to the biotin moiety. The solid support may then be washed. After post-enrichment washes, either PCR is performed on the solid support, in which case no elution of the processed DNA is needed, or, if elution is desired, fusion detection primers are modified to contain RNA, uridine, and/or abasic nucleotides at their 5' end, which can be degraded through appropriate enzymatic treatment. Products from the fusion detection workflow are identified by their unique ends based on the P7 end and the rearrangement detection barcode added by adapter ligation. The rearrangement detection barcode is sufficiently long (e.g., 8, 9, 10, 11, or 12 nucleotides) to facilitate error correction in the fusion detection population.

At least a portion of the amplified and/or eluted first population of processed DNA and at least a portion of the second population of processed DNA may then be sequenced. Captured cell-free nucleic acid isolated in this way is then prepared for sequencing and sequenced using a high-throughput (e.g., Illumina NextSeq, HiSeq or NovaSeq) sequencer. Results may be analyzed to determine the presence or absence of genomic rearrangements, such as oncogenic genomic rearrangements or gene fusions, of interest in the first population of processed DNA relative to a reference sequence. Further, results are analyzed to determine the presence or absence of one or more alterations (e.g., mutations and/or copy number alterations) from the second population of processed DNA relative to a reference sequence or internal control. The results of the analysis can be used to select a new or different treatment for the subject based at least in part on what genomic rearrangement(s) or gene fusion(s) are detected.

### 2. Detection of genomic rearrangements and one or both a sequence-variable target region set and an epigenetic target region set

As described in Example 1, DNA (e.g., cfDNA) is processed through standard library preparation and PCR, and the resulting tagged DNA is divided into first and second aliquots. The first aliquot is taken through the fusion detection workflow described above to produce a first population of processed DNA. The second aliquot is enriched for a second plurality of loci of interest, thereby producing a second population of processed DNA. The second plurality of loci of interest comprises a sequence-variable target region set and/or an epigenetic target region set.

To enrich the second aliquot for a second plurality of loci of interest, the second aliquot is contacted with target region probes comprising probes for one or both of a sequence-variable target region set and an epigenetic target region set. If probes for an epigenetic target region set are used, the DNA is processed prior to being contacted with a target region probe set (e.g., before any amplification is performed, and before division into first and second aliquots) by performing partitioning based on methylation status, end repair, ligation with adapters, and amplified by PCR (e.g., using primers targeted to the adapters).

The probes for the sequence-variable target region set may have a footprint of about 10-500 kb, while the probes for the epigenetic target region set may have a target region footprint of about 100-1000 kb. probes for the sequence-variable target region set may comprise oligonucleotides targeting a selection of regions identified in Tables 3-5 and the probes for the epigenetic target region set may comprise oligonucleotides targeting a selection of hypermethylation variable target regions, hypomethylation variable target regions, CTCF binding target regions, transcription start site target regions, focal amplification target regions, and methylation control regions.

Captured DNA (e.g., cfDNA) isolated in this way may then be sequenced using a high-throughput (e.g., Illumina NextSeq, HiSeq or NovaSeq) sequencer, and results will be analyzed. The sequence-variable target region sequences are analyzed by detecting genomic alterations such as SNVs, insertions, deletions, and fusions that can be called with enough support to discriminate real tumor variants from technical errors. The epigenetic target region sequences are analyzed independently to detect methylated fragments in regions that have been shown to be differentially methylated in cancer compared to blood cells.

As described in Example 1 above, at least a portion of the first population of processed DNA is sequenced to detect the presence of absence of a plurality of genomic rearrangements. The results of the analysis of the sequence-variable target region sequences and/or the epigenetic target region sequences are further combined with the rearrangement detection results. The results of the analysis can be used to select a new or different treatment for the subject based at least in part on what genomic rearrangement(s) or gene fusion(s) are detected.

## Claims

1. A method of detecting the presence or absence of a genomic rearrangement, the method comprising:
dividing a sample of tagged DNA molecules prepared from a sample from a subject into at least first and second aliquots;
isolating a plurality of tagged DNA molecules from the first aliquot, wherein the plurality of molecules have a common adapter sequence;
linearly amplifying at least a portion of the tagged DNA molecules isolated from the first aliquot with a set of primers that target a first plurality of loci of interest and comprise a rearrangement detection barcode, a sequencing adapter, and a non-nucleotide binding partner, thereby producing a first population of processed DNA;
capturing the first population of processed DNA on a solid support using binding to the non-nucleotide binding partner;
amplifying and/or eluting the first population of processed DNA;
enriching the second aliquot for a second plurality of loci of interest, thereby producing a second population of processed DNA;
sequencing at least a portion of the amplified and/or eluted first population of processed DNA and at least a portion of the second population of processed DNA; and
detecting the presence or absence of a plurality of genomic rearrangements in the first population of processed DNA.

2. The method of claim 1, further comprising detecting the presence or absence of one or more mutations relative to a reference sequence using sequence data from the second population of processed DNA.

3. The method of claim 1 or claim 2, wherein the sample of tagged DNA:
(i) comprises tagged cell-free DNA;
(ii) comprises molecules having a size in the range of 100-200 nucleotides, 200-300 nucleotides, 300-400 nucleotides, 400-500 nucleotides, 500-600 nucleotides, or 600-700 nucleotides;
(iii) consists of molecules having a mean size in the range of 100-200 nucleotides, 200-300 nucleotides, 300-400 nucleotides, 400-500 nucleotides, 500-600 nucleotides, or 600-700 nucleotides;
(iv) is a partition of the sample from the subject;
(v) comprises a hypomethylated partition of the sample from the subject; and/or
(vi) comprises at least two partitions of the sample from the subject that have been recombined, optionally wherein the at least two partitions of the sample from the subject that have been recombined comprise a hypomethylated partition and a hypermethylated partition.

4. The method of any one of the preceding claims, wherein:
(i) isolating a plurality of tagged DNA molecules from the first aliquot comprises blocking molecules comprising the complement of the common adapter sequence;
(ii) the primers that target the first plurality of loci of interest comprise a cleavable linker between the sequencing adapter and the non-nucleotide binding partner, optionally wherein the cleavable linker comprises at least one ribonucleotide, deoxyuridine, or abasic site; and/or
(iii) the method further comprises washing the solid support after capturing the first population of processed DNA on the solid support and/or before amplifying and/or eluting the first population of processed DNA.

5. The method of any one of the preceding claims, wherein:
(i) the plurality of genomic rearrangements comprises a plurality of gene fusions;
(ii) the plurality of genomic rearrangements comprises a plurality of oncogenic genomic rearrangements or gene fusions;
(iii) the plurality of genomic rearrangements comprises one or more interchromosomal gene fusions;
(iv) the plurality of genomic rearrangements comprises one or more intrachromosomal gene fusions;
(v) the plurality of genomic rearrangements comprises rearrangements affecting one or more of ALK, EML4, BCR, ABL1, MYC, an immunoglobulin gene, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ, or TFE3; and/or
(vi) the first plurality of loci of interest comprises one or more of ALK, EML4, BCR, ABL1, MYC, an immunoglobulin gene, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ, or TFE3.

6. The method of any one of the preceding claims, wherein the set of primers that target the first plurality of loci of interest comprises:
(i) primers that tile the loci of interest or at least a portion thereof;
(ii) primers that tile the loci of interest or at least a portion thereof at a density of at least 1, 2, 3, 4, 5, 6, 7, 8, 9, or 10 primers per 1000 nt; and/or
(iii) primers that tile the loci of interest or at least a portion thereof at a density of at least 1, 2, 3, 4, or 5 primers per 100 nt;
optionally wherein the set of primers that target the first plurality of loci of interest comprises:
(a) primers that tile at least 100 nucleotides of the loci of interest;
(b) primers that tile at least 5 kb of the loci of interest;
(c) primers that tile at least 10 kb of the loci of interest;
(d) primers that tile at least 20 kb of the loci of interest; or
(e) primers that tile at least 50 kb of the loci of interest.

7. The method of any one of the preceding claims, wherein:
(i) the common adapter sequence comprises a sequencing primer sequence or complement thereof;
(ii) the linear amplifying comprises at least 2, 3, 4, 5, 6, 7, 8, 9, or 10 linear amplification cycles;
(iii) the sample of tagged DNA molecules is prepared by ligating tags to DNA molecules;
(iv) the tagged DNA molecules are non-uniquely tagged;
(v) the tagged DNA molecules are amplified tagged DNA molecules;
(vi) at least a portion of the tags of the tagged DNA molecules comprise an indicator of epigenetic status, optionally wherein the epigenetic status is a level of methylation;
(vii) the rearrangement detection barcode comprises at least 8, 9, 10, 11, or 12 nucleotides; and/or
(viii) the solid support is a plurality of beads.

8. The method of any one of the preceding claims, wherein:
(i) the non-nucleotide binding partner comprises a biotin moiety and the solid support comprises a biotin-binding agent, optionally wherein the biotin-binding agent comprises an avidin, streptavidin, or anti-biotin antibody; or
(ii) the non-nucleotide binding partner comprises one of a digoxygenin, anti-dig antibody, maltose, or maltose-binding protein and the solid support comprises a binding agent of digoxygenin, anti-dig antibody, maltose, or maltose-binding protein, respectively.

9. The method of any one of the preceding claims, wherein:
(i) the tagged DNA molecules are prepared by a process comprising ligating tags to DNA from the subject, optionally wherein the method further comprises amplifying the tagged DNA molecules before dividing the sample into first and second aliquots; and/or
(ii) the tagged DNA molecules are prepared by a process comprising amplifying DNA from the subject with primers comprising tags.

10. The method of any one of the preceding claims, wherein the subject is a human.

11. The method of any one of the preceding claims, wherein:
I. the subject is suspected of having a cancer;
II. the method further comprises detecting the presence or absence of a cancer in the subj ect;
III. the method further comprises determining a likelihood that the subject has a cancer; and/or
IV. the subject has a cancer, optionally wherein:
(a) the subject is in need of a new or different treatment for the cancer;
(b) the subject has received a treatment for the cancer, such as wherein:
(i) the treatment did not result in a complete response;
(ii) the treatment did not result in remission;
(iii) the cancer is a solid tumor and the treatment did not result in arrested growth of the tumor;
(iv) the cancer is a hematological cancer and the treatment did not result in a reduction of circulating cancer cells, or did not result in a reduction of cancer cells in bone marrow; or
(v) the subject experienced a relapse subsequent to the treatment;
and/or
(c) at least one genomic rearrangement of the cancer is detected, optionally wherein:
(i) the genomic rearrangement is a gene fusion, such as an oncogenic gene fusion; and/or
(ii) the genomic rearrangement or gene fusion is indicative of an increased likelihood of efficacy for a new or different treatment, such as wherein the subject receives the new or different treatment after detection of the genomic rearrangement or gene fusion;
optionally wherein the cancer is or comprises colorectal cancer or one or more of lung, pancreatic, gastric, prostate, or liver cancer, or a lymphoma or leukemia.

12. The method of any one of the preceding claims, wherein the sequencing comprises high-throughput sequencing, pyrosequencing, sequencing-by-synthesis, single-molecule sequencing, nanopore-based sequencing, semiconductor sequencing, sequencing-by-ligation, sequencing-by-hybridization, RNA-Seq (Illumina), Digital Gene Expression (Helicos), next generation sequencing (NGS), Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, sequencing using PacBio, SOLiD, Ion Torrent, or a Nanopore platform.

13. The method of any one of the preceding claims, wherein the first population of processed DNA and the second population of processed DNA are:
(a) pooled before sequencing, optionally wherein:
(i) pooling occurs after capturing the first population of processed DNA on a solid support; or
(ii) pooling occurs during or after enriching the second aliquot for a second plurality of loci of interest;
and/or
(b) sequenced in the same sequencing cell;
or
(c) sequenced without being pooled.

14. The method of any one of the preceding claims, wherein:
(a) the second plurality of loci of interest comprises a sequence-variable target region set, optionally further comprising determining whether DNA molecules corresponding to the sequence-variable target region set comprise cancer-associated mutations; and/or
(b) the second plurality of loci of interest comprises an epigenetic target region set, optionally wherein:
(i) the second plurality of loci of interest comprises an epigenetic target region set and a sequence-variable target region set, and the sequence-variable target regions are sequenced at a greater depth of sequencing than the epigenetic target regions;
(ii) the epigenetic target region set comprises a hypermethylation variable target region set; and/or
(iii) the epigenetic target region set comprises a fragmentation variable target region set, such as wherein the fragmentation variable target region set comprises at least one of transcription start site regions or CTCF binding regions.

15. The method of any one of the preceding claims, wherein:
(a) enriching the second aliquot comprises contacting the DNA of the second aliquot with a plurality of target-binding probes specific for the second plurality of loci of interest; and/or
(b) the method further comprises detecting the presence or absence of one or more cancer biomarkers in a sample from the subject, optionally wherein the sample is a blood sample, further optionally wherein detecting the presence or absence of one or more cancer biomarkers in the sample comprises contacting the sample with one or more affinity agents, such as antibodies, specific for the one or more cancer biomarkers, such as wherein:
(i) the cancer biomarkers comprise one or more lung, pancreatic, gastric, prostate, or liver cancer biomarkers, or lymphoma or leukemia biomarkers; or
(ii) the cancer biomarkers comprise one or more colorectal cancer biomarkers.

## Patentansprüche

1. Verfahren zum Nachweis des Vorliegens oder des Nichtvorliegens einer genomischen Neuanordnung, wobei das Verfahren Folgendes umfasst:
Unterteilen einer Probe von getaggten DNA-Molekülen, hergestellt aus einer Probe aus einem Subjekt, in mindestens erste und zweite Aliquots;
Isolieren einer Vielzahl von getaggten DNA-Molekülen aus dem ersten Aliquot, wobei die Vielzahl von Molekülen eine gemeinsame Adaptersequenz aufweist;
lineares Amplifizieren von mindestens einem Teil der getaggten DNA-Moleküle, die aus dem ersten Aliquot isoliert wurden, mit einem Satz von Primern, die auf eine erste Vielzahl interessierender Loci abzielen und einen Rearrangement-Detektions-Barcode, einen Sequenzierungsadapter und einen Nichtnukleotid-Bindungspartner umfassen, wodurch eine erste Population von verarbeiteter DNA hergestellt wird;
Einfangen der ersten Population von verarbeiteter DNA auf einem festen Träger mittels Bindung an den Nichtnukleotid-Bindungspartner;
Amplifizieren und/oder Eluieren der ersten Population von verarbeiteter DNA;
Anreichern des zweiten Aliquots hinsichtlich einer zweiten Vielzahl interessierender Loci, wodurch eine zweite Population von verarbeiteter DNA hergestellt wird;
Sequenzieren mindestens eines Teils der amplifizierten und/oder eluierten ersten Population von verarbeiteter DNA und mindestens eines Teils der zweiten Population von verarbeiteter DNA; und
Nachweisen des Vorliegens oder des Nichtvorliegens einer Vielzahl von genomischen Neuanordnungen (Rearrangements) in der ersten Population von verarbeiteter DNA.

2. Verfahren nach Anspruch 1, ferner umfassend das Nachweisen des Vorliegens oder des Nichtvorliegens einer oder mehrerer Mutationen relativ zu einer Referenzsequenz unter Verwendung von Sequenzdaten aus der zweiten Population von verarbeiteter DNA.

3. Verfahren nach Anspruch 1 oder Anspruch 2, wobei die Probe von getaggter DNA:
(i) getaggte zellfreie DNA umfasst;
(ii) Moleküle mit einer Größe im Bereich von 100-200 Nukleotiden, 200-300 Nukleotiden, 300-400 Nukleotiden, 400-500 Nukleotiden, 500-600 Nukleotiden oder 600-700 Nukleotiden umfasst;
(iii) aus Molekülen mit einer mittleren Größe im Bereich von 100-200 Nukleotiden, 200-300 Nukleotiden, 300-400 Nukleotiden, 400-500 Nukleotiden, 500-600 Nukleotiden oder 600-700 Nukleotiden besteht;
(iv) eine Partition (Teilfraktion) der Probe aus dem Subjekt ist;
(v) eine hypomethylierte Partition der Probe aus dem Subjekt umfasst; und/oder
(vi) mindestens zwei Partitionen der Probe aus dem Subjekt, die rekombiniert wurden, umfasst, optional wobei die mindestens zwei Partitionen der Probe aus dem Subjekt, die rekombiniert wurden, eine hypomethylierte Partition und eine hypermethylierte Partition umfassen.

4. Verfahren nach einem der vorhergehenden Ansprüche, wobei:
(i) das Isolieren einer Vielzahl von getaggten DNA-Molekülen aus dem ersten Aliquot das Blockieren von Molekülen umfasst, die das Komplement der gemeinsamen Adaptersequenz umfassen;
(ii) die Primer, die auf die erste Vielzahl interessierender Loci abzielen, einen spaltbaren Linker zwischen dem Sequenzierungsadapter und dem Nichtnukleotid-Bindungspartner umfassen, optional wobei der spaltbare Linker mindestens ein(e) Ribonukleotid, Desoxyuridin oder abasische Stelle umfasst; und/oder
(iii) das Verfahren ferner das Waschen des festen Trägers nach dem Einfangen der ersten Population von verarbeiteter DNA auf dem festen Träger und/oder vor dem Amplifizieren und/oder Eluieren der ersten Population von verarbeiteter DNA umfasst.

5. Verfahren nach einem der vorhergehenden Ansprüche, wobei:
(i) die Vielzahl von genomischen Neuanordnungen eine Vielzahl von Genfusionen umfasst;
(ii) die Vielzahl von genomischen Neuanordnungen eine Vielzahl von onkogenen genomischen Neuanordnungen oder Genfusionen umfasst;
(iii) die Vielzahl von genomischen Neuanordnungen eine oder mehrere interchromosomale Genfusionen umfasst;
(iv) die Vielzahl von genomischen Neuanordnungen eine oder mehrere intrachromosomale Genfusionen umfasst;
(v) die Vielzahl von genomischen Neuanordnungen Neuanordnungen umfasst, die eines oder mehrere von ALK, EML4, BCR, ABL1, MYC, einem Immunglobulingen, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ oder TFE3 betreffen; und/oder
(vi) die erste Vielzahl interessierender Loci eines oder mehrere von ALK, EML4, BCR, ABL1, MYC, einem Immunglobulingen, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ oder TFE3 umfasst.

6. Verfahren nach einem der vorhergehenden Ansprüche, wobei der Satz von Primern, die auf die erste Vielzahl interessierender Loci abzielen, umfasst:
(i) Primer, die die interessierenden Loci oder mindestens einen Teil davon kachelartig abdecken;
(ii) Primer, die die interessierenden Loci oder mindestens einen Teil davon kachelartig bei einer Dichte von mindestens 1, 2, 3, 4, 5, 6, 7, 8, 9 oder 10 Primern pro 1000 nt abdecken; und/oder
(iii) Primer, die die interessierenden Loci oder mindestens einen Teil davon kachelartig bei einer Dichte von mindestens 1, 2, 3, 4 oder 5 Primern pro 100 nt abdecken;
optional wobei der Satz von Primern, die auf die erste Vielzahl interessierender Loci abzielen, umfasst:
(a) Primer, die mindestens 100 Nukleotide der interessierenden Loci kachelartig abdecken;
(b) Primer, die mindestens 5 kb der interessierenden Loci kachelartig abdecken;
(c) Primer, die mindestens 10 kb der interessierenden Loci kachelartig abdecken;
(d) Primer, die mindestens 20 kb der interessierenden Loci kachelartig abdecken; oder
(e) Primer, die mindestens 50 kb der interessierenden Loci kachelartig abdecken.

7. Verfahren nach einem der vorhergehenden Ansprüche, wobei:
(i) die gemeinsame Adaptersequenz eine Sequenzierungsprimer-Sequenz oder das Komplement davon umfasst;
(ii) das lineare Amplifizieren mindestens 2, 3, 4, 5, 6, 7, 8, 9, oder 10 Zyklen der linearen Amplifikation umfasst;
(iii) die Probe von getaggten DNA-Molekülen durch Ligieren von Tags an DNA-Moleküle hergestellt wird;
(iv) die getaggten DNA-Moleküle nicht-eindeutig getaggt sind;
(v) die getaggten DNA-Moleküle amplifizierte getaggte DNA-Moleküle sind;
(vi) mindestens ein Teil der Tags der getaggten DNA-Moleküle einen Indikator des epigenetischen Status umfasst, wobei es sich bei dem epigenetischen Status optional um ein Niveau der Methylierung handelt;
(vii) der Rearrangement-Detektions-Barcode mindestens 8, 9, 10, 11 oder 12 Nukleotide umfasst; und/oder
(viii) es sich bei dem festen Träger um eine Vielzahl von Kügelchen handelt.

8. Verfahren nach einem der vorhergehenden Ansprüche, wobei:
(i) der Nichtnukleotid-Bindungspartner eine Biotin-Einheit umfasst, und der feste Träger ein Biotinbindendes Mittel umfasst, optional wobei das Biotinbindende Mittel ein Avidin, ein Streptavidin oder einen Anti-Biotin-Antikörper umfasst; oder
(ii) der Nichtnukleotid-Bindungspartner eines von einem Digoxygenin, Anti-Dig-Antikörper, Maltose oder Maltosebindungsprotein umfasst, und der feste Träger ein bindendes Mittel für Digoxygenin, Anti-Dig-Antikörper, Maltose bzw. Maltosebindungsprotein umfasst.

9. Verfahren nach einem der vorhergehenden Ansprüche, wobei:
(i) die getaggten DNA-Moleküle durch ein Verfahren hergestellt werden, umfassend das Ligieren von Tags an DNA aus dem Subjekt, optional wobei das Verfahren ferner das Amplifizieren der getaggten DNA-Moleküle vor dem Unterteilen der Probe in erste und zweite Aliquots umfasst; und/oder
(ii) die getaggten DNA-Moleküle durch ein Verfahren hergestellt werden, umfassend das Amplifizieren von DNA aus dem Subjekt mit Primern, die Tags umfassen.

10. Verfahren nach einem der vorhergehenden Ansprüche, wobei das Subjekt ein Mensch ist.

11. Verfahren nach einem der vorhergehenden Ansprüche, wobei:
I. bei dem Subjekt der Verdacht besteht, dass es einen Krebs hat;
II. das Verfahren ferner das Nachweisen des Vorliegens oder Nichtvorliegens eines Krebses in dem Subjekt umfasst;
III. das Verfahren ferner das Bestimmen einer Wahrscheinlichkeit, dass das Subjekt einen Krebs hat, umfasst; und/oder
IV. das Subjekt einen Krebs hat, optional wobei:
(a) das Subjekt einer neuen oder andersartigen Behandlung für den Krebs bedarf;
(b) das Subjekt eine Behandlung für den Krebs erhalten hat, beispielsweise wobei:
(i) die Behandlung nicht zu einem vollständigen Ansprechen führte;
(ii) die Behandlung nicht zu einer Remission (Krankheitsrückgang) führte;
(iii) der Krebs ein solider Tumor ist und die Behandlung nicht zum Aufhalten des Wachstums des Tumors führte;
(iv) der Krebs ein hämatologischer Krebs ist und die Behandlung nicht zu einer Verringerung von zirkulierenden Krebszellen führte oder nicht zu einer Verringerung von Krebszellen im Knochenmark führte; oder
(v) das Subjekt im Anschluss an die Behandlung einen Rückfall erlitten hat;
und/oder
(c) mindestens eine genomische Neuanordnung des Krebses nachgewiesen wird, optional wobei:
(i) die genomische Neuanordnung eine Genfusion, wie eine onkogene Genfusion, ist; und/oder
(ii) die genomische Neuanordnung oder Genfusion auf eine erhöhte Wahrscheinlichkeit für die Wirksamkeit einer neuen oder andersartigen Behandlung hindeutet, beispielsweise wobei das Subjekt die neue oder andersartige Behandlung nach dem Nachweis der genomischen Neuanordnung oder Genfusion erhält;
optional wobei der Krebs Kolorektalkrebs oder eines oder mehrere von Lungen-, Pankreas-, Magen-, Prostata- oder Leberkrebs oder ein Lymphom oder eine Leukämie ist oder umfasst.

12. Verfahren nach einem der vorhergehenden Ansprüche, wobei das Sequenzieren Hochdurchsatz-Sequenzierung, Pyrosequenzierung, Sequenzierung-durch-Synthese, Einzelmolekül-Sequenzierung, Nanoporen-basierte Sequenzierung, Halbleiter-Sequenzieren, Sequenzierung-durch-Ligation, Sequenzierung-durch-Hybridisierung, RNA-Seq (Illumina), Digitale-Gen-Expression (Helicos), Next-Generation-Sequenzierung (NGS), Single-Molecule-Sequenzierung durch Synthese (SMSS) (Helicos), massivparallele Sequenzierung, Klonales-Einzelmolekül-Array (Solexa), Shotgun-Sequenzierung, Ion Torrent, Oxford Nanopore, Roche Genia, Maxim-Gilbert-Sequenzierung, Primer-Walking, Sequenzieren mithilfe von PacBio, SOLiD, Ion Torrent oder einer Nanopore-Plattform umfasst.

13. Verfahren nach einem der vorhergehenden Ansprüche, wobei die erste Population von verarbeiteter DNA und die zweite Population von verarbeiteter DNA:
(a) vor dem Sequenzieren vereinigt werden, optional wobei:
(i) das Vereinigen nach dem Einfangen der ersten Population von verarbeiteter DNA auf einem festen Träger stattfindet; oder
(ii) das Vereinigen während oder nach dem Anreichern des zweiten Aliquots hinsichtlich einer zweiten Vielzahl interessierender Loci stattfindet;
und/oder
(b) in derselben Sequenzierungszelle sequenziert werden;
oder
(c) sequenziert werden ohne vereinigt zu werden.

14. Verfahren nach einem der vorhergehenden Ansprüche, wobei:
(a) die zweite Vielzahl interessierender Loci einen sequenzvariable-Zielregion-Satz umfasst, optional ferner umfassend das Bestimmen, ob dem sequenzvariable-Zielregion-Satz entsprechende DNA-Moleküle Krebsassoziierte Mutationen umfassen; und/oder
(b) die zweite Vielzahl interessierender Loci einen epigenetische-Zielregion-Satz umfasst, optional wobei:
(i) die zweite Vielzahl interessierender Loci einen epigenetische-Zielregion-Satz und einen sequenzvariable-Zielregion-Satz umfasst, und die sequenzvariablen Zielregionen zu einer größeren Sequenzierungstiefe als die epigenetischen Zielregionen sequenziert werden;
(ii) der epigenetische-Zielregion-Satz einen Hypermethylierungs-variable-Zielregion-Satz umfasst; und/oder
(iii) der epigenetische-Zielregion-Satz einen Fragmentierungs-variable-Zielregion-Satz umfasst, wobei beispielsweise der Fragmentierungs-variable-Zielregion-Satz mindestens eines von Transkriptionsstartstellen-Regionen oder CTCF-Bindungsregionen umfasst.

15. Verfahren nach einem der vorhergehenden Ansprüche, wobei:
(a) das Anreichern des zweiten Aliquots das Inberührungbringen der DNA des zweiten Aliquots mit einer Vielzahl zielbindender Sonden, die spezifisch für die zweite Vielzahl interessierender Loci sind, umfasst; und/oder
(b) das Verfahren ferner das Nachweisen des Vorliegens oder des Nichtvorliegens von einem oder mehreren Krebsbiomarkern in einer Probe aus dem Subjekt umfasst, optional wobei die Probe eine Blutprobe ist, ferner optional wobei das Nachweisen des Vorliegens oder des Nichtvorliegens von einem oder mehreren Krebsbiomarkern in der Probe das Inberührungbringen der Probe mit einem oder mehreren Affinitätsmittel(n), wie Antikörpern, die für den einen oder die mehreren Krebsbiomarker spezifisch sind, umfasst, beispielsweise wobei:
(i) die Krebsbiomarker einen oder mehrere Lungen-, Pankreas-, Magen-, Prostata- oder Leberkrebsbiomarker oder Lymphom- oder Leukämiebiomarker umfassen; oder
(ii) die Krebsbiomarker einen oder mehrere Kolorektalkrebsbiomarker umfassen.

## Revendications

1. Procédé de détection de la présence ou de l'absence d'un réarrangement génomique dans un échantillon, le procédé comprenant :
la division d'un échantillon de molécules d'ADN étiquetées préparé à partir d'un échantillon provenant d'un sujet en au moins une première et une deuxième aliquote ;
l'isolement d'une pluralité de molécules d'ADN étiquetées à partir de la première aliquote, la pluralité de molécules ayant une séquence adaptatrice commune ;
l'amplification linéaire d'au moins une partie des molécules d'ADN étiquetées isolées à partir de la première aliquote avec un ensemble d'amorces qui ciblent une première pluralité de locus d'intérêt et comprennent un code-barres de détection de réarrangement, un adaptateur de séquençage et un partenaire de liaison non nucléotidique, ce qui permet de produire une première population d'ADN traité ;
la capture de la première population d'ADN traité sur un support solide en utilisant la liaison au partenaire de liaison non nucléotidique ;
l'amplification et/ou l'élution de la première population d'ADN traité ;
l'enrichissement de la deuxième aliquote en une deuxième pluralité de locus d'intérêt, produisant ainsi une deuxième population d'ADN traité ;
le séquençage d'au moins une partie de la première population d'ADN traité amplifiée et/ou éluée et au moins une partie de la deuxième population d'ADN traité ; et
la détection de la présence ou de l'absence d'une pluralité de réarrangements génomiques dans la première population d'ADN traité.

2. Procédé selon la revendication 1, comprenant en outre la détection de la présence ou de l'absence d'une ou plusieurs mutations par rapport à une séquence de référence au moyen des données de séquence provenant de la deuxième population d'ADN traité.

3. Procédé selon la revendication 1 ou la revendication 2, dans lequel l'échantillon d'ADN :
(i) comprend de l'ADN acellulaire étiqueté ;
(ii) comprend des molécules ayant une taille dans la plage de 100 à 200 nucléotides, 200 à 300 nucléotides, 300 à 400 nucléotides, 400 à 500 nucléotides, 500 à 600 nucléotides ou 600 à 700 nucléotides ;
(iii) est constitué de molécules ayant une taille moyenne dans la plage de 100 à 200 nucléotides, 200 à 300 nucléotides, 300 à 400 nucléotides, 400 à 500 nucléotides, 500 à 600 nucléotides ou 600 à 700 nucléotides ;
(iv) est une partie de l'échantillon provenant du sujet ;
(v) comprend une partie hypométhylée de l'échantillon du sujet ; et/ou
(vi) comprend au moins deux parties de l'échantillon provenant du sujet qui ont été recombinées, les au moins deux parties de l'échantillon provenant éventuellement du sujet qui ont été recombinées comprenant une partie hypométhylée et une partie hyperméthylée.

4. Procédé selon l'une quelconque des revendications précédentes, dans lequel :
(i) l'isolement d'une pluralité de molécules d'ADN étiquetées à partir de la première aliquote comprend le blocage de molécules comprenant le complément de la séquence adaptatrice commune ;
(ii) les amorces qui ciblent la première pluralité de locus d'intérêt comprennent un lieur clivable entre l'adaptateur de séquençage et le partenaire de liaison non nucléotidique, le lieur clivable comprenant éventuellement au moins un ribonucléotide, une désoxyuridine ou un site abasique ; et/ou
(iii) le procédé comprend en outre le lavage du support solide après la capture de la première population d'ADN traité sur le support solide et/ou avant l'amplification et/ou l'élution de la première population d'ADN traité.

5. Procédé selon l'une quelconque des revendications précédentes, dans lequel :
(i) la pluralité de réarrangements génomiques comprend une pluralité de fusions de gènes ;
(ii) la pluralité de réarrangements génomiques comprend une pluralité de réarrangements génomiques ou fusions de gènes oncogènes ;
(iii) la pluralité de réarrangements génomiques comprend une ou plusieurs fusions de gènes interchromosomiques ;
(iv) la pluralité de réarrangements génomiques comprend une ou plusieurs fusions de gènes intrachromosomiques ;
(v) la pluralité de réarrangements génomiques comprend des réarrangements affectant l'un ou plusieurs parmi ALK, EML4, BCR, ABL1, MYC, un gène d'immunoglobuline, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ ou TFE3 ; et/ou
(vi) la première pluralité de locus d'intérêt comprend l'un ou plusieurs parmi ALK, EML4, BCR, ABL1, MYC, un gène d'immunoglobuline, EWSR1, FLI1, SS18, SSX1, PML1, RARA, ATF1, ETV6, NTRK3, PAX8, PPARG, MECT1, MAML2, TMPRSS2, ERG, ETV1, BRAF, KIAA1549, MYB, NFIB, ESRRA, C11orf20, FGFR3, TACC3, PTPRK, RSPO3, EIF3E, RSPO2, SFPQ ou TFE3.

6. Procédé selon l'une quelconque des revendications précédentes, dans lequel l'ensemble d'amorces qui ciblent la première pluralité de locus d'intérêt comprend :
(i) des amorces qui couvrent les locus d'intérêt ou au moins une partie de ceux-ci ;
(ii) des amorces qui couvrent les locus d'intérêt ou au moins une partie de ceux-ci à une densité d'au moins 1, 2, 3, 4, 5, 6, 7, 8, 9 ou 10 amorces pour 1000 nt ; et/ou
(iii) des amorces qui couvrent les locus d'intérêt ou au moins une partie de ceux-ci à une densité d'au moins 1, 2, 3, 4 ou 5 amorces pour 100 nt ;
facultativement, dans lequel l'ensemble d'amorces qui ciblent la première pluralité de locus d'intérêt comprend :
(a) des amorces qui couvrent au moins 100 nucléotides des locus d'intérêt ;
(b) des amorces qui couvrent au moins 5 kb des locus d'intérêt ;
(c) des amorces qui couvrent au moins 10 kb des locus d'intérêt ;
(d) des amorces qui couvrent au moins 20 kb des locus d'intérêt ; ou
(e) des amorces qui couvrent au moins 50 kb des locus d'intérêt.

7. Procédé selon l'une quelconque des revendications précédentes, dans lequel :
(i) la séquence adaptatrice commune comprend une séquence d'amorce de séquençage ou un complément de celle-ci ;
(ii) l'amplification linéaire comprend au moins 2, 3, 4, 5, 6, 7, 8, 9 ou 10 cycles d'amplification linéaire ;
(iii) l'échantillon de molécules d'ADN étiquetées est préparé par ligature d'étiquettes à des molécules d'ADN ;
(iv) les molécules d'ADN étiquetées ne sont étiquetées de manière non unique ;
(v) les molécules d'ADN étiquetées sont des molécules d'ADN étiquetées amplifiées ;
(vi) au moins une partie des étiquettes des molécules d'ADN étiquetées comprennent un indicateur d'état épigénétique, l'état épigénétique étant éventuellement un taux de méthylation ;
(vii) le code-barres de détection de réarrangement comprend au moins 8, 9, 10, 11 ou 12 nucléotides ; et/ou
(viii) le support solide est une pluralité de billes.

8. Procédé selon l'une quelconque des revendications précédentes, dans lequel :
(i) le partenaire de liaison non nucléotidique comprend un fragment de biotine et le support solide comprend un agent de liaison à la biotine, l'agent de liaison à la biotine comprenant éventuellement une avidine, une streptavidine ou un anticorps anti-biotine ; ou
(ii) le partenaire de liaison non nucléotidique comprend respectivement une digoxygénine, un anticorps anti-dig, du maltose ou une protéine se liant au maltose et le support solide comprend un agent de liaison à la digoxygénine, à un anticorps anti-dig, au maltose ou à une protéine se liant au maltose, respectivement.

9. Procédé selon l'une quelconque des revendications précédentes, dans lequel :
(i) les molécules d'ADN étiquetées sont préparées par un processus comprenant la ligature d'étiquettes à l'ADN provenant du sujet, le procédé comprenant éventuellement en outre l'amplification des molécules d'ADN étiquetées avant la division de l'échantillon en première et deuxième aliquotes ; et/ou
(ii) les molécules d'ADN étiquetées sont préparées par un processus comprenant l'amplification de l'ADN du sujet avec des amorces comprenant des étiquettes.

10. Procédé selon l'une quelconque des revendications précédentes, le sujet étant un humain.

11. Procédé selon l'une quelconque des revendications précédentes, dans lequel :
I. le sujet est suspecté d'avoir un cancer ;
II. le procédé comprend en outre la détection de la présence ou de l'absence d'un cancer chez le sujet ;
III. le procédé comprend en outre la détermination d'une probabilité du fait que le sujet est ou non atteint d'un cancer ; et/ou
IV. le sujet est atteint d'un cancer, éventuellement dans lequel :
(a) le sujet a besoin d'un traitement nouveau ou différent pour le cancer ;
(b) le sujet a reçu un traitement pour le cancer, par exemple dans lequel :
(i) le traitement n'a pas entraîné de réponse complète ;
(ii) le traitement n'a pas entraîné de rémission ;
(iii) le cancer est une tumeur solide et le traitement n'a pas entraîné d'arrêt de la croissance de la tumeur ;
(iv) le cancer est un cancer hématologique et le traitement n'a pas entraîné de réduction des cellules cancéreuses circulantes, ou n'a pas entraîné de réduction des cellules cancéreuses dans la moelle osseuse ; ou
(v) le sujet a connu une récidive suite au traitement ; et/ou
(c) au moins un réarrangement génomique du cancer est détecté, éventuellement dans lequel :
(i) le réarrangement génomique est une fusion de gènes, telle qu'une fusion de gènes oncogène ; et/ou
(ii) le réarrangement génomique ou la fusion de gènes indique une probabilité accrue d'efficacité pour un traitement nouveau ou différent, par exemple lorsque le sujet reçoit le traitement nouveau ou différent après la détection du réarrangement génomique ou de la fusion de gènes ;
éventuellement dans lequel le cancer est ou comprend un cancer colorectal ou l'un ou plusieurs parmi un cancer du poumon, du pancréas, de l'estomac, de la prostate ou du foie, ou un lymphome ou une leucémie.

12. Procédé selon l'une quelconque des revendications précédentes, dans lequel le séquençage comprend un séquençage à haut débit, un pyroséquençage, un séquençage par synthèse, un séquençage de molécule unique, un séquençage à base de nanopores, un séquençage par semiconducteur, un séquençage par ligature, un séquençage par hybridation, RNA-Seq (Illumina), Digital Gene Expression (Helicos), un séquençage de nouvelle génération (NGS), un séquençage de molécule unique par synthèse (SMSS) (Helicos), un séquençage parallèle massif, Clonal Single Molécule Array (Solexa), un séquençage shotgun, Ion Torrent, Oxford Nanopore, Roche Genia, un séquençage de Maxim-Gilbert, un arpentage chromosomique, un séquençage utilisant PacBio, SOLiD, Ion Torrent ou une plateforme Nanopore.

13. Procédé selon l'une quelconque des revendications précédentes, dans lequel la première population d'ADN traité et la deuxième population d'ADN traité sont :
(a) regroupées avant le séquençage, éventuellement dans lequel :
(i) le regroupement se produit après la capture de la première population d'ADN traité sur un support solide ;
ou
(ii) le regroupement se produit pendant ou après l'enrichissement de la deuxième aliquote en une deuxième pluralité de locus d'intérêt ;
et/ou
(b) séquencées dans la même cellule de séquençage ;
ou
(c) séquencées sans être mutualisées.

14. Procédé selon l'une quelconque des revendications précédentes, dans lequel :
(a) la deuxième pluralité de locus d'intérêt comprend un ensemble de régions cibles à séquence variable, comprenant éventuellement en outre la détermination du fait que les molécules d'ADN correspondant à l'ensemble de régions cibles à séquence variable comprennent ou non des mutations associées au cancer ; et/ou
(b) la deuxième pluralité de locus d'intérêt comprend un ensemble de régions cibles épigénétiques, éventuellement dans lequel :
(i) la deuxième pluralité de locus d'intérêt comprend un ensemble de régions cibles épigénétiques et un ensemble de régions cibles à séquence variable, et les régions cibles à séquence variable sont séquencées à une plus grande profondeur de séquençage que les régions cibles épigénétiques ;
(ii) l'ensemble de régions cibles épigénétiques comprend un ensemble de régions cibles variables d'hyperméthylation ; et/ou
(iii) l'ensemble de régions cibles épigénétiques comprend un ensemble de régions cibles variables de fragmentation, par exemple dans lequel l'ensemble de régions cibles variables de fragmentation comprend au moins l'une parmi des régions de site d'initiation de transcription ou des régions de liaison à CTCF.

15. Procédé selon l'une quelconque des revendications précédentes, dans lequel :
(a) l'enrichissement de la deuxième aliquote comprend la mise en contact de l'ADN de la deuxième aliquote avec une pluralité de sondes de liaison de cible spécifiques pour la deuxième pluralité de locus d'intérêt ; et/ou
(b) le procédé comprend en outre la détection de la présence ou de l'absence d'un ou plusieurs biomarqueurs de cancer dans un échantillon provenant du sujet, éventuellement dans lequel l'échantillon est un échantillon de sang, dans lequel, facultativement en outre, la détection de la présence ou de l'absence d'un ou plusieurs biomarqueurs de cancer dans l'échantillon comprend la mise en contact de l'échantillon avec un ou plusieurs agents d'affinité, tels que des anticorps, spécifiques pour les un ou plusieurs biomarqueurs de cancer, par exemple dans lequel :
(i) les biomarqueurs de cancer comprennent un ou plusieurs biomarqueurs de cancer du poumon, du pancréas, de l'estomac, de la prostate ou du foie, ou des biomarqueurs de lymphome ou de leucémie ; ou
(ii) les biomarqueurs de cancer comprennent un ou plusieurs biomarqueurs de cancer colorectal.
